(19) Europäisches Patentamt
European Patent Office
Office européen des brevets

(11) EP 1 136 503 A1

(12) **EUROPEAN PATENT APPLICATION**
published in accordance with Art. 158(3) EPC

(43) Date of publication:
**26.09.2001 Bulletin 2001/39**

(21) Application number: **99973037.7**

(22) Date of filing: **29.11.1999**

(51) Int Cl.[7]: **C07K 14/46**, C12N 15/12, C12N 1/21, C12N 5/10, C12P 21/02, C07K 16/18, C12P 21/08, A61P 25/00, A61P 9/00, A61P 13/00, A61P 27/00, G01N 33/53

(86) International application number:
**PCT/JP99/06649**

(87) International publication number:
**WO 00/32627 (08.06.2000 Gazette 2000/23)**

(84) Designated Contracting States:
**AT BE CH CY DE DK ES FI FR GB GR IE IT LI LU MC NL PT SE**
Designated Extension States:
**AL LT LV MK RO SI**

(30) Priority: **30.11.1998 JP 33898498**
**04.02.1999 JP 2684899**
**26.08.1999 JP 23936799**

(71) Applicant: **Takeda Chemical Industries, Ltd.**
**Osaka-shi, Osaka 541-0045 (JP)**

(72) Inventors:
- **MORI, Masaaki**
**Tsukuba-shi, Ibaraki 305-0821 (JP)**
- **ABE, Michiko**
**Inashiki-gun, Ibaraki 300-1252 (JP)**
- **SHIMOMURA, Yukio, Takeda Yakuhin**
**Tsukuba-shi, Ibaraki 305-0035 (JP)**
- **SUGO, Tsukasa**
**Tsukuba-shi, Ibaraki 300-3261 (JP)**
- **KITADA, Chieko**
**Sakai-shi, Osaka 590-0073 (JP)**

(74) Representative: **Best, Michael, Dr. et al**
**Lederer, Keller & Riederer**
**Patentanwälte**
**Prinzregentenstrasse 16**
**80538 München (DE)**

(54) **NOVEL PHYSIOLOGICALLY ACTIVE SUBSTANCE, PROCESS FOR PRODUCING THE SAME AND UTILIZATION THEREOF**

(57) A novel peptide recognized as a ligand by a G protein-coupled receptor protein. The above peptide is usable in: (1) developing a receptor-bonded assay system and screening a candidate compound for a drug with the use of a recombinant receptor protein expression system; and (2) developing drugs such as a central function controlling agent, a circulatory function controlling agent, a heart function controlling agent, an immunological function controlling agent, a digestive function controlling agent, a metabolic function controlling agent or a genital function controlling agent.

EP 1 136 503 A1

**Description**

FIELD OF THE INVENTION

The present invention relates to a novel polypeptide having a ligand activity to an SENR (sensory epithelium neuropeptide-like receptor), which is a G protein-coupled receptor protein, a DNA encoding the same, etc.

BACKGROUND ART

A variety of hormones, neurotransmitters and the like control or regulate the functions of a body in vivo via specific receptors present on cell membranes. Many of these receptors are coupled to guanine nucleotide-binding proteins (hereinafter, sometimes referred to as G proteins) to mediate the transduction of intracellular signals via activation of the G proteins. These receptors possess the common structure, i.e., seven transmembrane domains and are thus referred to generally as G protein-coupled receptor proteins or seven transmembrane receptors.

These hormones, neurotransmitters and the like interact with G protein-coupled receptor protein and, via the interactive reaction, regulate important functions for a living body such as maintenance of homeostasis, reproduction, development of individuals, metabolism, growth, control of the nervous, circulatory, immune, digestive or metabolic system, sensory adaptation, or the like. As such it is known that there are receptor proteins to various hormones or neurotransmitters for regulating the functions *in vivo* and these receptor proteins play pivotal roles for regulating these functions. However, it often remains unclear if there are any other unknown substances (hormones, neurotransmitters, etc.) and receptors to these substances.

In recent years, polymerase chain reaction (hereinafter abbreviated as PCR) has been actively performed to explore a DNA encoding a novel receptor protein, utilizing the nature of G protein-coupled receptor protein to show similarity in part of the amino acid sequence in its structure, and many ligand-unknown, so-called orphan G protein-coupled receptor proteins have been cloned (Libert, F., et al., Scince, 244, 569-572, 1989, Welch, S.K., et al., Biochem. Biophys. Res. Commun., 209, 606-613, 1995; Marchese, A., et al., Genomics, 23, 609-618, 1994; Marches, A., Genomics, 29, 335-344, 1995). Also, novel G protein-coupled receptor proteins have been found one after another by random sequencing of genomic DNA or cDNA (Nomura, N., et al., DNA Research, 1, 27-35, 1994). Only general method for determining ligands to these G protein-coupled receptor proteins was to deduce ligands from similarity in primary structure of G protein-coupled receptor proteins. However, many orphan G protein-coupled receptor proteins have low homology to known receptors. Practically, it was difficult to deduce ligands only from similarity in primary structure, except that they are receptor subtypes of known ligands. On the other hand, since many orphan G protein-coupled receptor proteins were found by gene analysis, it is anticipated that there would be many other unknown ligands corresponding to those proteins. However, only a few ligands have actually been identified to G protein-coupled receptor proteins.

Recently, it was reported to survey a novel opioid peptide by introducing cDNA encoding an orphan G protein-coupled receptor protein into an animal cell (Reinsheid, R. K., et al., Science, 270, 792-794, 1995; Menular, J. C., et al., Nature, 377, 532-535, 1995). In this case, however, it was easily expected that the ligand would belong to the opioid peptide family in view of its similarity to known G protein-coupled receptor protein or its tissue distribution. During a long history in research and development of substances that act *in vivo* via opioid receptors, various antagonists and agonists were developed. Therefore, the structure of a ligand is determined by selecting an agonist to the receptor from compounds artificially synthesized, verifying that the receptor is expressed in the receptor cDNA-transduced cells using the agonist as a probe, then searching an activated substance of intracellular signal transduction system similar to the agonist and purifying the substance.

It was further reported that a novel physiologically active peptide was identified by introducing cDNA encoding a snail orphan G protein-coupled receptor (GRL104) into CHO cells, using as an index an increase of a specific intracellular free calcium concentration in the receptor-expressed cells (Cox, K. J. A., et al., J. Neurosci., 17(4), 1197-1205, 1997). This novel physiologically active peptide had a high homology to known leucokinin and GRL104 also had a reactivity with known leucokinin. As such, few ligands could be roughly assumed in orphan G protein-coupled receptor proteins. In particular, when similarity to known G protein-coupled receptor protein family is low, there is little information on ligands. It was thus difficult to deduce its ligands.

An SENR is reported to be one of the orphan G protein-coupled receptors (Tal, M. et al., Biochem. Biophys. Res. Commun., 209, 752-759, 1995). The SENR has a low homology to somatostatin receptor (SSTR4) but it was unknown what its ligand was. GPR14 reported by Marchese, A., et al. (Marchese, A., Genomics, 29, 335-344, 1995) is the same receptor as the SENR. Ligands to the SENR, which is a G protein-coupled receptor expressed in the organ such as central nervous system, circulatory system, genital system, immune system, digestive apparatus, urinary system, sensory organs and the like, are considered to be useful as drugs but the structure and functions are unknown yet.

DISCLOSURE OF THE INVENTION

The present inventors have succeeded in screening a polypeptide recognized by the receptor protein as a ligand under the index of a specific cell stimulating (signal transduction) activity measured, etc., using a cell in which an SENR-encoding cDNA has been expressed by an appropriate means.

The inventors have further found that a compound that can alter the binding property between the activator ligand and an SENR described above can be screened.

Thus, the present invention relates to the following features.

(1) A polypeptide comprising the same or substantially the same amino acid sequence as that represented by SEQ ID NO: 7, its amide or ester, or a salt thereof.

(2) A polypeptide or its amide or ester, or a salt thereof according to (1), wherein substantially the same amino acid sequence is represented by SEQ ID NO:8 or SEQ ID NO:21.

(3) A precursor protein of the polypeptide according to (1), or a salt thereof.

(4) A precursor protein or a salt thereof according to (3), comprising the same or substantially the same amino acid sequence represented by SEQ ID NO:18 or SEQ ID NO:19.

(5) A DNA containing a DNA having the base sequence encoding the polypeptide according to (1).

(6) A DNA according to (5), which has the base sequence represented by SEQ ID NO: 27 or SEQ ID NO: 28.

(7) A DNA containing a DNA having a base sequence encoding the precursor protein according to (3).

(8) A DNA according to (7), which contains the base sequence represented by SEQ ID NO: 15, SEQ ID NO: 16 or SEQ ID NO: 17.

(9) A recombinant vector containing the DNA according to (5) or (7).

(10) A transformant transformed with the recombinant vector according to (9).

(11) A method for manufacturing the polypeptide, its amide or ester, or a salt thereof according to (1) or the precursor protein or its salt according to (3), which comprises culturing the transformant according to (10), producing and accumulating the polypeptide according to (1) or the precursor protein according to (3) and collecting the same.

(12) An antibody to the polypeptide, its amide or ester, or a salt thereof according to (1) or to the precursor protein or its salt according to (3).

(13) A pharmaceutical composition comprising the polypeptide, its amide or ester, or a salt thereof according to (1) or the precursor protein or its salt according to (3).

(14) A pharmaceutical composition comprising the DNA according to (5) or (7).

(15) A pharmaceutical composition according to (13) or (14), which is an agent for regulating the central functions, an agent for regulating the circulatory functions, an agent for regulating the cardiac functions, an agent for regulating the renal functions, an agent for regulating the urinary functions or an agent for regulating the sensory functions.

(16) A method for screening a compound or its salt that alters the binding property between an SENR and the polypeptide, its amide or ester, or a salt thereof according to (1), or the precursor protein or its salt according to (3), which comprises using the polypeptide, its amide or ester, or a salt thereof according to (1) or the precursor protein or its salt according to (3).

(17) A kit for screening a compound or its salt that alters the binding property between an SENR and the polypeptide, its amide or ester, or a salt thereof according to (1), or the precursor protein or its salt according to (3), comprising the polypeptide, its amide or ester, or a salt thereof according to (1) or the precursor protein or its salt according to (3).

(18) A method for screening a compound or its salt that alters the binding property between an SENR and a polypeptide containing the amino acid sequence shown by SEQ ID NO:22, or its amide or ester, or a salt thereof, which comprises using a polypeptide containing the amino acid sequence shown by SEQ ID NO:22, or its amide or ester, or a salt thereof.

(19) A kit for screening a compound or its salt that alters the binding property between an SENR and a polypeptide containing the amino acid sequence shown by SEQ ID NO:22, its amide or ester, or a salt thereof, comprising a polypeptide containing the amino acid sequence shown by SEQ ID NO:22, or its amide or ester, or a salt thereof.

(20) A compound that alters the binding property between 1) an SENR and the polypeptide, its amide or ester, or a salt thereof according to (1) or the precursor protein or its salt according to (3), or 2) an SENR and a polypeptide containing the amino acid sequence shown by SEQ ID NO:22, its amide or ester, or a salt thereof, which is obtainable by using the screening method according to (16) or (18) or by using the screening kit according to (17) or (19).

(21) A composition for the prevention and treatment of hypertension, comprising a compound or its salt according to (20).

(22) A method for quantifying the polypeptide, its amide or ester, or a salt thereof according to (1) or the protein or its salt according to (3), which comprises using the antibody of (12).

(23) A diagnostic composition for disease associated with the functions of the polypeptide, its amide or ester, or a salt thereof according to (1) or the precursor protein or its salt according to (3), comprising the antibody according to (12).

The present invention further relates to the following features:

(24) A polypeptide, its amide or ester, or a salt thereof according to (1) or a precursor protein or its salt according to (3), which is derived from an animal cell.

(25) A pharmaceutical composition according to (13) or (14), which is an agent for the treatment and prevention of diseases such as hypertension (hypotension), renal disease, cardiac disease, pollakiuria, urinary continence, impaired hearing, cacosmia, visual abnormality, or the like.

Turning specifically to an SENR to the polypeptide in the present invention, the present invention not only includes the aforesaid known SENRs or salts thereof but also includes the following:

(26) An SENR comprising the same or substantially the same amino acid sequence as the amino acid sequence shown by SEQ ID NO:9 or SEQ ID NO:26, or a salt thereof.

(27) An SENR or a salt thereof according to (26), wherein said SENR is a protein containing the amino acid sequence shown by SEQ ID NO:9 or SEQ ID NO:26 wherein at least 1 to 30, preferably at least 1 to 10, inclusive, amino acids are deleted; a protein containing the amino acid sequence shown by SEQ ID NO:9 or SEQ ID NO: 26, to (or into) which at least 1 to 30, preferably at least 1 to 10, inclusive, amino acids are added (or inserted); or a protein containing the amino acid sequence shown by SEQ ID NO:9 or SEQ ID NO:26 wherein at least 1 to 30, preferably at least 1 to 10, inclusive, amino acids are substituted with other amino acids; or a salt thereof.

BRIEF DESCRIPTION OF THE DRAWINGS

FIG. 1 shows a cDNA sequence of rat SENR isolated by the PCR technique using total rat brain-derived cDNA (SEQ ID NO: 3).

FIG. 2 shows the results obtained by assaying the activity of promoting the release of arachidonic acid metabolites from the CHO/SENR cell line, in the HPLC fraction prepared from porcine spinal cord extract.

FIG. 3 shows a behavior of the HPLC fraction #33 in EXAMPLE 6 on the arachidonic acid metabolite-releasing activity when treated with pronase.

FIG. 4 shows the results obtained by measuring the arachidonic acid metabolite release-promoting activity specific to CHO/SENR, with respect to the fraction purified on CN column (Develosil CN-UG-5) in EXAMPLE 7.

FIG. 5 shows the results obtained by measuring the arachidonic acid metabolite release-promoting activity specific to CHO/SENR, with respect to the fraction purified on CN column (Develosil CN-UG-5) in EXAMPLE 7.

FIG. 6 shows the results obtained by measuring the arachidonic acid metabolite release-promoting activity specific to CHO/SENR, with respect to the fraction purified on ODS column (Wakosil-II 3C18HG) in EXAMPLE 7.

FIG. 7 shows the results of measuring the activity for accelerating the release obtained by measuring the arachidonic acid metabolite release-promoting activity specific to CHO/SENR, with respect to the fraction purified on ODS column (Wakosil-II 3C18HG) in EXAMPLE 7.

FIG. 8 shows the entire base sequence of swine SENR ligand precursor protein cDNA isolated from porcine spinal cord-derived cDNA and the entire amino acid sequence of swine SENR ligand precursor protein deduced therefrom, wherein the portion within the box indicates a sequence of the SENR ligand polypeptide.

FIG. 9 shows the arachidonic acid metabolite-releasing activity of synthetic swine SENR ligand to CHO/SENR cell line.

FIG. 10 shows the vasoconstrictive action of synthetic swine SENR ligand on rat thoracic aorta ring preparations.

FIG. 11 shows the arachidonic acid metabolite-releasing activity of synthetic human SENR ligand (human urotensin II) to CHO/hSENR cell line.

FIG. 12 shows the binding activity of synthetic bovine SENR ligand to CHO/SENR cell membrane fraction.

FIG. 13 shows the binding activity of synthetic human SENR ligand to CHO/hSENR cell membrane fraction.

FIG. 14 shows the entire base sequence of bovine SENR ligand precursor protein cDNA isolated from total bovine brain-derived cDNA and the entire amino acid sequence of bovine SENR ligand precursor protein deduced therefrom, wherein the portion within the box indicates a sequence of the bovine SENR ligand polypeptide.

BEST MODE FOR CARRYING OUT THE INVENTION

Throughout the specification, the term "substantially the same" is used to mean that the activities of a polypeptide or protein, e.g., the binding activity between a ligand and a receptor (SENR), physiological properties, etc. are substantially the same. Substitution, deletion, addition or insertion of an amino acid does not often cause any significant change in physiological properties or chemical properties of a polypeptide or protein; in this case, such a polypeptide that undergoes substitution, deletion, addition or insertion is considered to be substantially the same as the polypeptide that does not undergo substitution, deletion, addition or insertion. Substantially the same substituent of an amino acid in the amino acid sequence can be selected from, e.g., other amino acids of the class to which the amino acid belongs. Examples of non-polar (hydrophobic) amino acids include alanine, leucine, isoleucine, valine, proline, phenylalanine, tryptophan, methionine and the like. Examples of polar (neutral) amino acids are glycine, serine, threonine, cysteine, tyrosine, asparagine, glutamine, and the like. Examples of positively charged (basic) amino acids are arginine, lysine, histidine, and the like. Examples of negatively charged (acidic) amino acids include aspartic acid, glutamic acid, and the like.

The polypeptide of the present invention, its amide or ester, or a salt thereof is a ligand to SENR and refers specifically to the polypeptide containing the same or substantially the same amino acid sequence represented by SEQ ID NO:7, its amide or ester, or a salt thereof, etc.

The polypeptide of the present invention, its amide or ester, or a salt thereof (hereinafter sometimes referred to as the polypeptide of the present invention), a method for manufacturing the same and utility thereof are described below in more detail.

The polypeptide of the present invention may be any polypeptide so long as it is derived from any tissues (e.g., pituitary gland, pancreas, brain, kidney, liver, genital gland, thyroid gland, gall bladder, spinal cord, adrenal, skin, muscle, lung, digestive tract, blood vessel, heart, etc.), cells or the like of human and other warm-blooded animals (e.g., guinea pig, rat, mouse, swine, sheep, bovine, monkey, etc.) and has the same or substantially the same amino acid sequence as the amino acid sequence shown by SEQ ID NO:7. Examples of the polypeptide of the present invention include, in addition to the polypeptide containing the amino acid sequence shown by SEQ ID NO:7, a polypeptide which has substantially equivalent activity to the polypeptide containing the amino acid sequence shown by SEQ ID NO:7 (e.g., a polypeptide containing the amino acid sequence shown by SEQ ID NO:8 or SEQ ID NO:21, etc.) and the like. Examples of substantially equivalent activity include a receptor-binding activity, a signal transduction activity, and the like. The term substantially equivalent is used to mean that the nature of the receptor binding activity, etc. is equivalent. Therefore, differences in degree such as a level of the receptor binding activity and quantitative factors such as a molecular weight of the polypeptide may be present and allowable.

Specific examples of the polypeptides which contain substantially the same amino acid sequence as the amino acid sequence shown by SEQ ID NO: 7 are polypeptides containing the amino acid sequence shown by SEQ ID NO: 7 in which the third amino acid (Thr) from the N-terminus is substituted with other amino acid (e.g., Ala, Leu, Ile, Val, Pro, Phe, Trp, Met, Gly, Ser, Cys, Tyr, Asn, Gln, Arg, Lys, His, Asp, Glu). Of these polypeptides, preferred are a polypeptide containing the amino acid sequence shown by SEQ ID NO: 7 in which the third amino acid (Thr) from the N-terminus is substituted with Pro (SEQ ID NO:8) and a polypeptide containing the amino acid sequence shown by SEQ ID NO: 7 in which the third amino acid (Thr) from the N-terminus is substituted with Ser (SEQ ID NO: 21) and the like.

Throughout the present specification, the polypeptides are represented in accordance with the conventional way of describing peptides, that is, the N-terminus (amino terminus) at the left hand and the C-terminus (carboxyl terminus) at the right hand. In the polypeptides containing the amino acid sequences such as 1) the amino acid sequence shown by SEQ ID NO: 7, 2) the amino acid sequence shown by SEQ ID NO: 8 and 3) the amino acid sequence shown by SEQ ID NO: 21, the C-terminus is usually in the form of a carboxyl group (-COOH) or a carboxylate ($-COO^-$) but may be in the form of an amide ($-CONH_2$) or an ester (-COOR). Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a phenyl-$C_{1-2}$ alkyl group, e.g., benzyl, phenethyl, benzhydryl, etc.; a $C_{7-14}$ aralkyl such as an $\alpha$-naphthyl-$C_{1-2}$ alkyl group, e.g., $\alpha$-naphthylmethyl, etc.; and the like. In addition, pivaloyloxymethyl or the like which is used widely as an ester for oral administration may also be used.

The polypeptide of the present invention may be used in the form of salts with physiologically acceptable bases (e.g., alkali metal salts) or acids (e.g., inorganic acids or organic acids), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide of the present invention or salts thereof may be manufactured by a method used to purify a polypeptide from human or other warm-blooded animal cells or tissues described above. Alternatively, the polypeptide of the present invention or salts thereof may also be manufactured by the methods for synthesizing polypeptides, which will be described hereinafter, or by modified methods. Furthermore, the polypeptide of the present invention or its salts may also be manufactured by culturing a transformant containing a DNA encoding the polypeptide, as will also be later described.

Where the polypeptide or salts thereof are manufactured from human or warm-blooded animal tissues or cells, human or warm-blooded animal tissues or cells are homogenized, then extracted with an acid, an organic solvent or the like, and the extract is isolated and purified by means of salting-out, dialysis, gel filtration, or a combination of chromatography techniques such as reverse phase chromatography, ion exchange chromatography, affinity chromatography, and the like.

The polypeptide of the present invention can be manufactured by publicly known methods for polypeptide synthesis or by cleaving a polypeptide containing the polypeptide of the present invention with an appropriate peptidase. For the peptide synthesis methods, for example, either solid phase synthesis method or liquid phase synthesis method may be used. That is, partial peptides or amino acids that may compose the polypeptide of the present invention are condensed with the residual portion. When the product has protecting groups, the desired peptide can be obtained by removing the protecting groups. The publicly known condensation and protecting group removal methods include the methods described in 1)-5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso* to *Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the polypeptide of the present invention may be purified and isolated by a combination of conventional purification methods such as solvent extraction, distillation, column chromatography, liquid chromatography and recrystallization. When the polypeptide obtained by the above methods is in a free form, the polypeptide can be converted into an appropriate salt by a publicly known method; when the polypeptide is obtained in a salt form, it can be converted into a free form by a publicly known method.

To synthesize the amides of the polypeptide, commercially available resins for peptide synthesis that are suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetoamidemethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin, in accordance with the order of the sequence of the objective peptide, by various condensation methods publicly known in the art. At the end of the reaction, the peptide is excised from the resin and at the same time, the protecting groups are removed. Then, intramolecular disulfide bond-forming reaction is performed in a highly diluted solution to obtain the objective polypeptide.

For condensation of the protected amino acids described above, a variety of activating reagents for peptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be appropriately chosen from solvents that are known to be usable for peptide condensation reactions. Examples of such solvents are acid amides such as N,N-dimethylformamide, N,N-dimethylacetoamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; tertiary amines such as pyridine, etc.; ethers such as dioxane, tetrahydrofuran, etc.; nitriles such as acetonitrile, propionitrile, etc.; esters such as methyl acetate, ethyl acetate, etc.; and suitable mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is tested using the ninhydrin reaction; when the test reveals that the condensation is insufficient, the condensation can be completed by repeating the condensation reaction without removal of the protecting groups. When the condensation is still insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting group R for a carboxyl group include, in addition to those examples for the $C_{1-6}$ alkyl group, the $C_{3-8}$ cycloalkyl and the $C_{7-14}$ aralkyl described hereinabove, 2-adamantyl, 4-nitrobenzyl, 4-methoxybenzyl, 4-chlorobenzyl, phenacyl and benzyloxycarbonylhydrazide, t-butoxycarbonylhydrazide, tritylhydrazide, and the like.

The hydroxyl group of serine and threonine can be protected through, for example, their esterification or etherification. Examples of groups appropriately used for the esterification include a lower alkanoyl group such as acetyl, etc., an aroyl group such as benzoyl group, and a group derived from carbon such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bz1, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting materials include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated amino groups in the starting material, the corresponding phosphoric amides are employed.

To eliminate (split off) the protecting groups, there are used catalytic hydrogenation under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a solution mixture of these acids; a treatment with a base such as diisopropylethylamine, triethylamine, piperidine or piperazine; and reduction with sodium in liquid ammonia. Elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethyl sulfide, 1,4-butanedithiol or 1,2-ethanedithiol. Furthermore, 2,4-dinitrophenyl group known as a protecting group for the imidazole of histidine is removed by a treatment with thiophenol. Formyl group used as a protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the polypeptide, for example, the α-carboxyl group of the carboxyl terminal amino acid is first amidated; the peptide chain is then extended from the amino group side to a desired length. Thereafter, a peptide in which only the protecting group of the N-terminal α-amino group has been eliminated from the peptide and a peptide in which only the protecting group of the C-terminal carboxyl group has been eliminated are manufactured. The two peptides are condensed in a mixture of the solvents described above. The details of the condensation reaction are the same as described above. After the protected peptide obtained by the condensation is purified, all the protecting groups are eliminated by the method described above to give the desired crude polypeptide. This crude polypeptide is purified by various known purification means. Lyophilization of the major fraction gives the amide of the desired polypeptide.

To prepare the esterified polypeptide, for example, the α-carboxyl group of the carboxy terminal amino acid is condensed with a desired alcohol to prepare the amino acid ester, which is followed by procedure similar to the preparation of the amidated polypeptide above to give the ester of the desired polypeptide.

Any polypeptide is usable as the polypeptide of the present invention so long as it contains the same or substantially the same amino acid sequence as that represented by SEQ ID NO:7 described above and possesses the same activities as those of the polypeptide, e.g., activities for regulating central nervous functions, circulatory functions, heart functions, renal functions, urinary functions, or sensory functions, etc. Such a polypeptide is, for example, a peptide having the amino acid sequence shown by SEQ ID NO:8 or SEQ ID NO:21.

In addition, the polypeptide of the present invention can be used as an antigen for preparing an antibody to the polypeptide. As such a polypeptide that can be used as the antigen, partial peptides such as N-terminal peptide, C-terminal peptide, a peptide at the central part, etc. of the polypeptide in the present invention may be employed as well, in addition to the polypeptides of the present invention described above.

The partial peptide that can be employed may be a peptide containing each of the individual domains or a peptide containing a plurality of domains concurrently.

In the partial peptide of the present specification, the C-terminus may also be in the form of an amide ($-CONH_2$) or an ester (-COOR). The same examples as given for the polypeptide described above apply to the amides of the partial peptide. Where the partial peptide contains a carboxyl group or a carboxylate at a site other than the C terminus, those wherein these groups are amidated or esterified are also included in the partial peptide of the present invention. Examples of such esters are those of the C-terminal esters described above, and the like.

The polypeptide of the present invention or its partial peptide may be a fused protein with a protein, which functions or properties are well known.

Examples of salts of the partial peptide which can be used for the polypeptide of the present invention are the same as those of the aforesaid polypeptide salts.

The partial peptide of the polypeptide of the present invention, its amide or ester, or a salt thereof may be manufactured by the same synthesis method as in the polypeptide of the present invention described above, or by cleaving the polypeptide of the present invention with an appropriate peptidase.

The DNA encoding the polypeptide of the present invention may be any DNA so long as it contains a DNA encoding a polypeptide containing the same or substantially the same amino acid sequence represented by SEQ ID NO:7. Also, the DNA may be any one of genomic DNA, genomic DNA library, cDNA derived from the cells or tissues described above, cDNA library derived from the cells or tissues described above and synthetic DNA. The vector used for the library may be any of bacteriophage, plasmid, cosmid, phagemid and the like. In addition, the DNA can be directly amplified by reverse transcriptase polymerase chain reaction (hereinafter abbreviated as RT-PCR technique) using an RNA fraction prepared from the cells or tissues described above.

Herein, the polypeptide containing the same or substantially the same amino acid sequence as that shown by SEQ ID NO:7 includes the amino acid sequence shown by SEQ ID NO:8 or SEQ ID NO:21, as described above. The DNA containing the polypeptide-encoding DNA containing the amino acid sequence shown by SEQ ID NO:8 is, for example, a DNA containing a DNA having the base sequence shown by SEQ ID NO:27, and the like, and the DNA containing the polypeptide-encoding DNA containing the amino acid sequence shown by SEQ ID NO:21 is, e.g., a DNA containing a DNA having the base sequence shown by SEQ ID NO:28.

Examples of the DNA containing a DNA encoding the polypeptide having the same or substantially the same amino acid sequence as the amino acid sequence shown by SEQ ID NO:7 are a DNA containing a DNA having a base sequence with homology of at least about 80%, preferably at least about 90%, more preferably at least about 95%, and most preferably at least about 98%, to the base sequence shown by SEQ ID NO:27 or SEQ ID NO:28.

Examples of the DNA containing a DNA encoding the polypeptide having the same or substantially the same amino acid sequence as the amino acid sequence shown by SEQ ID NO:7 include DNAs containing (i) the base sequence represented by SEQ ID NO:27 or SEQ ID NO:28, of which at least 1 or 2 (preferably 1 to 30, more preferably 1 to 10 and most preferably (1 or 2)) bases are deleted; (ii) the base sequence represented by SEQ ID NO:27 or SEQ ID NO:28, to which at least 1 or 2 (preferably 1 to 30, more preferably 1 to 10 and most preferably (1 or 2)) bases are added; (iii) the base sequence represented by SEQ ID NO:27 or SEQ ID NO:28, in which at least 1 or 2 (preferably 1 to 30, more preferably 1 to 10 and most preferably (1 or 2)) bases are inserted; (iv) in the amino acid sequence represented by SEQ ID NO:27 or SEQ ID NO:28, the base sequence in which at least 1 or 2 (preferably 1 to 30, more preferably 1 to 10 and most preferably (1 or 2)) bases are substituted by other bases; or (v) a combination of the above base sequences. More specifically, there are employed (1) a mammal-derived DNA that is hybridizable under stringent conditions to the sequence possessed by a DNA containing a DNA capable of binding to a receptor protein containing the same or substantially the same amino acid sequence as the amino acid sequence represented by SEQ ID NO:7; (2) a DNA that is not hybridizable due to degeneracy of genetic code to the sequence possessed by the DNA containing a DNA capable of binding to a receptor protein containing the same or substantially the same amino acid sequence as the amino acid sequence represented by SEQ ID NO:7 and the sequence defined in (1) but encoding a polypeptide having the same amino acid sequence, and the like. The hybridization can be carried out by publicly known methods or by a modified known methods. The stringent conditions described above are, for example, conditions of 42°C, 50% formamide, 4 x SSPE (1 x SSPE = 150 mM NaCl, 10 mM $NaH_2PO_4 \cdot H_2O$, 1 mM EDTA, pH 7.4), 5 x Denhardt's solution and 0.1% SDS.

As the DNA hybridizable to the sequence possessed by a DNA containing a DNA encoding a polypeptide containing the same or substantially the same amino acid sequence as the amino acid sequence represented by SEQ ID NO:7, there are employed, e.g., a DNA containing a base sequence having homology of at least about 70%, preferably at least about 80%, more preferably at least about 90%, and most preferably at least about 95%, to the base sequence shown by SEQ ID NO:27 or SEQ ID NO:28, and the like.

DNA fragments containing a partial base sequence of the DNA encoding the polypeptide containing 1) the amino acid sequence shown by SEQ ID NO:7, 2) the amino acid sequence shown by SEQ ID NO:8, 3) the amino acid sequence shown by SEQ ID NO:21, etc. are also preferably used as probes for DNA detection.

The DNA encoding the polypeptide of the present invention may also be manufactured by the following genetic engineering techniques.

For cloning of the DNA that fully encodes the polypeptide of the present invention, the DNA may be either amplified from the DNA library, etc. *supra* by publicly known PCR using synthetic DNA primers containing a partial base sequence of the polypeptide of the present invention or, the DNA inserted into an appropriate vector can be selected by hybridization with a labeled DNA fragment or synthetic DNA that encodes a part or entire region of the polypeptide of the present invention. The hybridization can be carried out, for example, according to the method described in Molecular Cloning (2nd ed.; J. Sambrook et al., Cold Spring Harbor Lab. Press, 1989). When a commercially available library is used, the hybridization may also be performed in accordance with the protocol described in the attached instructions.

The cloned DNA encoding the polypeptide of the present invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the polypeptide of the present invention can be manufactured, for example, by (a) excising the desired DNA fragment from the DNA encoding the polypeptide of the present invention, (b) and then ligating the DNA fragment to an appropriate expression vector downstream of a promoter in the vector.

Examples of the vector include plasmids derived form *E. coli* (e.g., pBR322, pBR325, pUC12, pUC13), plasmids derived from *Bacillus subtilis* (e.g., pUB110, pTP5, pC194), plasmids derived from yeast (e.g., pSH19, pSH15), bacteriophages such as λ phage, etc., animal viruses such as retrovirus, vaccinia virus, baculovirus, etc.

The promoter used in the present invention may be any promoter if it matches well with a host to be used for gene expression.

When an animal cell is used as a host for transformation, there may be utilized SV40-derived promoter, retrovirus promoter, metallothionein promoter, heat shock promoter, cytomegalovirus promoter, SRα promoter, etc. Where the host is bacteria of the genus Escherichia, preferred examples of the promoter include trp promoter, T7 promoter, lac promoter, recA promoter, λPL promoter, lpp promoter, etc. When bacteria of the genus Bacillus are used as the host, preferred example of the promoter are SPO1 promoter, SPO2 promoter and penP promoter. When yeast is used as the host, preferred examples of the promoter are PHO5 promoter, PGK promoter, GAP promoter, ADH1 promoter, GAL promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as Neo, G418 resistance), etc. In particular, when DHFR gene is used as the selection marker using CHO (dhfr), selection may also be made on thymidine free media.

If necessary and desired, a signal sequence that matches with a host is added to the N-terminal side of the polypeptide or its partial peptide. Examples of the signal sequence that can be utilized are phoA signal sequence, OmpA signal sequence, etc. when bacteria of the genus Escherichia are used as the host; α-amylase signal sequence, subtilisin signal sequence, etc. when bacteria of the genus Bacillus are used as the host; mating factor α(MF α) signal sequence, invertase signal sequence, etc. when yeast is used as the host; and insulin signal sequence, α-interferon signal sequence, antibody molecule signal sequence, etc. when animal cells are used as the host, respectively.

Using the vector comprising the DNA encoding the polypeptide thus constructed, transformants can be prepared.

Examples of the host, which may be employed, are bacteria belonging to the genus Escherichia, bacteria belonging to the genus Bacillus, yeast, insects or insect cells and animal cells, etc.

Specific examples of the bacteria belonging to the genus Escherichia include *Escherichia coli* K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include *Bacillus subtilis* MI114 (Gene, 24, 255 (1983)), 207-21 (Journal of Biochemistry, 95, 87 (1984)), etc.

Examples of yeast include Saccharomyces cereviseae AH22, $AH22R^-$, NA87-11A, DKD-5D, 20B-12, etc.

As the insect, for example, a larva of Bombyx mori can be used (Maeda et al., Nature, 315, 592 (1985)).

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711) and Sf21 cell (both cells are described in Vaughn, J. L. et al., In vitro, 13, 213-217 (1977)), etc.

Examples of animal cells include monkey cell COS-7, Vero cell, Chinese hamster cell CHO, DHFR gene deficient Chinese hamster cell CHO ($dhfr^-$ CHO cell), mouse L cell, mouse 3T3 cell, mouse myeloma cell, human HEK293 cell, human FL cell, 293 cell, C127 cell, BALB3T3 cell, Sp-2/O cell, etc.

Bacteria belonging to the genus Escherichia can be transformed, for example, by the method described in Proc. Natl. Acad. Sci. U.S.A., 69, 2110 (1972) or Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Proc. Natl. Acad. Sci. U.S.A., 75, 1929 (1978).

Insect cells or insects can be transformed, for example, according to the method described in Bio/Technology, 6, 47-55(1988), etc.

Animal cells can be transformed, for example, according to the method described in Virology, 52, 456 (1973).

Methods for introducing the expression vectors into the cells include, for example, the lipofection method [Felgner, P. L., et al., Proceedings of the National Academy of Sciences of the United States of America, 84, 7413 (1987)], the calcium phosphate method [Graham, F. L. and van der Eb, A., J. Virology, 52, 456-467 (1973)], the electroporation method [Nuemann, E. et al., EMBO J., 1, 841-845 (1982)], etc.

As described above, transformants transformed with the expression vector containing the DNA encoding the polypeptide of the present invention are obtained.

For stably expressing the polypeptide of the present invention using animal cells, there is applicable a method of selecting the cells by clone selection in which the aforesaid expression vectors transfected to animal cells are introduced into chromosomes. Specifically, transformants are selected using as an index the selection marker described above. Further by repeated clone selections on the transformants thus obtained using the selection marker, stable animal cell line capable of highly expressing the polypeptide of the present invention can be obtained. Furthermore, when the dhfr gene is used as the selection marker, cultivation can be performed by gradually increasing a level of MTX, resistant cells are selected thereby to amplify the DNA encoding the polypeptide of the present invention or its partial peptide in the cells together with the dhfr gene. Thus, the animal cell line of higher expression can be obtained.

The transformant described above is cultivated under conditions that the DNA encoding the polypeptide of the present invention can express, to produce and accumulate the polypeptide of the present invention. Thus, the polypeptide of the present invention can be manufactured.

Where the host is bacteria belonging to the genus Escherichia or the genus Bacillus, the transformant can be appropriately cultivated in a liquid medium which contains materials required for growth of the transformant, such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrates, corn steep liquor, peptone, casein, meat extract, soybean cake, potato extract, etc. Examples of the inorganics are calcium chloride, sodium dihydrogenphosphate, magnesium chloride, etc. In addition, yeast, vitamins, growth promoting factors, etc. may also be added to the medium. Preferably, pH of the medium is adjusted to about 5 to about 8.

A preferred example of the medium for cultivation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15 to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium [Bitter, G. A. et al., Proc. Natl. Acad. Sci. U.S.A., 81, 5330 (1984)] supplemented with 0.5% Casamino acids. Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

Especially when the CHO(dhfr') cell and the dhfr gene are employed as the selection markers, it is preferred to use substantially thymidine-free DMEM supplemented with dialyzed bovine fetal serum.

The polypeptide of the present invention can be separated and purified from the culture described above by the following procedures.

When the polypeptide of the present invention is extracted from the culture or cells, after cultivation the transformant or cell is collected by a publicly known method and suspended in an appropriate buffer. The transformant or cell is then disrupted by publicly known methods such as ultrasonication, a treatment with lysozyme and/or freeze-thaw cycling, followed by centrifugation, filtration, etc. Thus, the crude extract of the polypeptide can be obtained. The buffer used for the procedures may contain a protein modifier such as urea or guanidine hydrochloride, or a surfactant such as Triton X-100 (trademark, hereinafter sometimes abbreviated as TM), etc.

When the polypeptide is secreted in the culture broth, after completion of the cultivation the supernatant can be separated from the transformant or cell to collect the supernatant by a publicly known method.

The polypeptide of the present invention contained in the thus-obtained culture supernatant or the extract can be purified by appropriately combining the publicly known methods for separation and purification. Such known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charges such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectric focusing, chromatofocusing, etc.; and the like.

When the polypeptide of the present invention thus obtained is in a free form, it can be converted into the salt by publicly known methods or modifications thereof. On the other hand, when the polypeptide is obtained in a salt form, it can be converted into the free form or a different salt form by publicly known methods or modifications thereof.

The polypeptide of the present invention produced by the recombinant can be treated, prior to or after the purification, with an appropriate protein modifying enzyme so that the polypeptide can be suitably modified or partially removed. Examples of the protein modifying enzyme include trypsin, chymotrypsin, arginyl endopeptidase, protein kinase, glycosidase and the like.

The activity of the thus formed polypeptide of the present invention can be determined by an enzyme immunoassay using a specific antibody, or the like.

The DNA encoding the polypeptide of the present invention or the polypeptide of the present invention can be used: (1) for the survey of physiological activities that the polypeptide of the present invention possesses, (2) for preparing synthetic oligonucleotide probes or primers for PCR, (3) for acquiring DNAs encoding ligands to SENR or precursor proteins, (4) for the development of the receptor-binding assay system using the expression system of recombinant receptor protein and screening of a candidate drug, (5) for acquiring antibodies and antisera, (6) for the development of diagnostic agents using DNAs, RNAs, antibodies or antisera, (7) for the development of drugs such as agents for regulating central nervous functions, circulatory functions, heart functions, renal functions, urinary functions, sensory functions, etc., (8) for gene therapy, and the like.

In particular, the receptor-binding assay system using the expression system of recombinant SENR described hereinafter can be used for screening SENR agonists or antagonists specific to a warm-blooded animal like human. These agonists or antagonists may be employed as agents for the prevention/treatment of various diseases.

Furthermore in (7) described above, when an SENR is expressed in the central nervous system, the circulatory system or the cardiac, renal, urinary organ or sensory organ system, the SENR recognizes the polypeptide of the present invention or the DNA encoding the same as a ligand and is thus useful as safe and low toxic drug. Since the polypeptide of the present invention or the DNA encoding the same participates in the actions for regulating the central nervous functions, circulatory functions, heart functions, renal functions, urinary functions, sensory functions, etc., the polypeptide or DNA can be used as a therapeutic and prophylactic drug for, e.g., senile dementia, cerebrovascular dementia, dementia associated with genealogical retroplastic diseases (e.g., Alzheimer's disease, Parkinson's disease, Pick's disease, Huntington's disease, etc.), hypertension (hypotension), renal diseases (e.g., chronic renal failure, nephritis, etc.), cardiac diseases (e.g., heart failure, acute myocardial infarction, etc.), pollakiuria, urinary incontinence, deafness, dysosmia, optical abnormality, or the like.

When the polypeptide of the present invention or the DNA encoding the same is used as the drugs described above, its pharmaceutical preparations can be prepared in a conventional manner. The polypeptide of the present invention or the DNA encoding the same can be used orally, for example, in the form of tablets with sugar coating or enteric coating, if necessary and desired, capsules, elixirs, microcapsules etc., or parenterally in the form of injectable preparations such as a sterile solution, a suspension, etc. in water or with other pharmaceutically acceptable liquid. These preparations can be manufactured by mixing the compound described above with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

Where the DNA of the present invention is used, the DNA is inserted into an appropriate vector such as retrovirus vector, adenovirus vector, adenovirus-associated virus vector, etc. and then administered in a conventional manner.

Additives miscible with tablets, capsules etc. include a binder such as gelatin, corn starch, tragacanth, gum arabic, etc.; an excipient such as crystalline cellulose, etc.; a swelling agent such as corn starch, gelatin, alginic acid, etc.; a lubricant such as magnesium stearate; a sweetening agent such as sucrose, lactose, or saccharin; and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80 (™) and HCO-50), etc. Examples of the oily medium include sesame oil, soybean oil, etc., which may also be used in combination with a dissolution aid such as benzyl benzoate, benzyl alcohol, etc.

The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, or the like. The thus-prepared liquid for injection is normally filled in an appropriate ampoule.

Since the thus obtained pharmaceutical preparation is safe and low toxic, the preparation can be administered to, e.g., human or other mammals (e.g., mouse, rat, guinea pig, rabbit, sheep, swine, bovine, cat, dog, monkey, etc.).

The dose of the polypeptide of the present invention or the DNA encoding the same varies depending on condition, etc. In oral administration, the dose is normally about 0.1 mg to about 100 mg, preferably about 1.0 to about 50 mg, and more preferably about 1.0 to about 20 mg per day for the adult patient with heart failure (as 60 kg body weight). In parenteral administration, the single dose varies depending on subject to be administered, target organ, condition, method for administration, etc. but it is advantageous to administer the active ingredient intravenously at a daily dose of about 0.01 to about 30 mg, preferably about 0.1 to about 20 mg, and more preferably about 0.1 to about 10 mg for the adult patient with heart failure (as 60 kg body weight). For other animal species, the corresponding dose as converted per 60 kg body weight can be administered.

The precursor protein of the polypeptide in accordance with the present invention and salts thereof, and a method for manufacturing the same as well as utility thereof are described below in detail.

As the precursor protein of the polypeptide of the present invention and salts thereof (hereinafter sometimes referred to as the precursor protein of the present invention), there are, for example, the aforesaid proteins of the present invention wherein at least 1 or 2, preferably 1 to about 200, more preferably 1 to about 120, and most preferably about 50 to about 120 amino acids are bound at the N terminus and/or the C terminus, or salts thereof.

Specific examples of the precursor protein of the present invention used are proteins having the same or substantially the same amino acid sequence as that of SEQ ID NO: 18, SEQ ID NO: 19 or SEQ ID NO: 29.

The precursor protein of the present invention may be any protein so long as it is a protein derived from any tissue (e.g., pituitary gland, pancreas, brain, kidney, liver, genital gland, thyroid gland, gall bladder, spinal cord, adrenal, skin, muscle, lung, digestive tract, blood vessel, heart, etc.), any cell or the like of human or other warm-blooded animals (e.g., guinea pig, rat, mouse, swine, sheep, bovine, monkey, etc.) and has the same or substantially the same amino acid sequence as the amino acid sequence shown by SEQ ID NO:18, SEQ ID NO:19 or SEQ ID NO:29. Examples of the substantially equivalent activity include the receptor binding activity, the signal transduction activity, etc. The substantially equivalent activity is used to mean that the nature of the receptor binding activity, etc. is equivalent. Therefore, differences in degree such as a level of the receptor binding activity, quantitative factors such as a molecular weight of the protein may be present and allowable.

Specific examples of the amino acid sequence having the same or substantially the same amino acid sequence shown by SEQ ID NO:18, SEQ ID NO:19 or SEQ ID NO:29 are amino acid sequences showing homology of at least about 50%, preferably at least about 60%, more preferably at least about 70%, much more preferably at least about 80%, further much more preferably at least about 90% and most preferably at least about 95%, to the amino acid sequence shown by SEQ ID NO:18, SEQ ID NO:19 or SEQ ID NO:29.

Examples of the precursor protein of the present invention include proteins containing (i) the amino acid sequence represented by SEQ ID NO:18, SEQ ID NO:19 or SEQ ID NO:29, in which at least 1 or 2 (preferably about 1 to about 30, more preferably about 1 to about 10 and most preferably (1 or 2)) amino acids are deleted; (ii) the amino acid sequence represented by SEQ ID NO:18, SEQ ID NO:19 or SEQ ID NO:29, to which at least 1 or 2 (preferably about 1 to about 30, more preferably about 1 to about 10 and most preferably (1 or 2)) amino acids are added; (iii) the amino acid sequence represented by SEQ ID NO:18, SEQ ID NO:19 or SEQ ID NO:29, in which at least 1 or 2 (preferably about 1 to about 30, more preferably about 1 to about 10 and most preferably (1 or 2)) amino acids are inserted; (iv) in the amino acid sequence represented by SEQ ID NO:18, SEQ ID NO:19 or SEQ ID NO:29, the amino acid sequence in which at least 1 or 2 (preferably about 1 to about 30, more preferably about 1 to about 10 and most preferably (1 or 2)) amino acids are substituted by other amino acids; and (v) a combination of the above amino acid sequences.

Specific examples of the precursor protein of the polypeptide of the present invention containing the amino acid sequence shown by SEQ ID NO:8 are proteins containing the amino acid sequence shown by SEQ ID NO:18 or SEQ ID NO:19; and,

Specific examples of the precursor protein of the polypeptide of the present invention containing the amino acid sequence shown by SEQ ID NO: 21 are proteins containing the amino acid sequence shown by SEQ ID NO: 29.

In the precursor proteins of the present specification, the N-terminus (amino terminus) is designated at the left hand and the C-terminus (carboxyl terminus) at the right hand, according to a conventional way of describing peptides. In the precursor proteins containing amino acid sequences such as the amino acid sequence shown by SEQ ID NO:18, SEQ ID NO:19 or SEQ ID NO:29, the C-terminus is usually in the form of a carboxyl group (-COOH) or a carboxylate ($-COO^-$) but may be in the form of an amide ($-CONH_2$) or an ester (-COOR). Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a phenyl-$C_{1-2}$ alkyl group, e. g., benzyl, phenethyl, benzhydryl, etc.; a $C_{7-14}$ aralkyl such as an α-naphthyl-$C_{1-2}$ alkyl group, e.g., α-naphthylmethyl, etc.; and the like. In addition, pivaloyloxymethyl or the like which is used widely as an ester for oral administration may also be used.

Examples of salts of the precursor protein of the present invention are the same as the salts given for the polypeptide of the present invention described above.

The precursor protein of the present invention may be manufactured either by methods used to purify protein from human or other warm-blooded animal cells or tissues described above through modifications of the methods for manufacturing the polypeptide of the present invention or by modifications of methods for protein synthesis. Alternatively, the precursor protein may also be manufactured by culturing a transformant containing a DNA encoding the precursor protein of the present invention, by modifications of the methods for manufacturing the polypeptide of the present invention described above.

Where the precursor protein of the present invention are manufactured from human or warm-blooded animal tissues or cells, the human or warm-blooded animal tissues or cells are homogenized, then extracted with an acid, an organic solvent or the like, and the extract is purified and separated by means of salting-out, dialysis, gel filtration, or a combination of chromatography techniques such as reverse phase chromatography, ion exchange chromatography, affinity chromatography, and the like.

To synthesize the amides of the precursor protein of the present invention, commercially available resins for peptide synthesis that are suitable for amide formation may be used. Using such resins, amino acids in which α-amino groups and functional groups on the side chains are appropriately protected are condensed on the resin, in accordance with the order of the sequence of the objective peptide, by various condensation methods publicly known in the art. At the end of the reaction, the peptide is excised from the resin and at the same time, the protecting groups are removed. Then, intramolecular disulfide bond-forming reaction is performed in a highly diluted solution to obtain the objective precursor protein of the present invention.

The precursor protein of the present invention may per se have the same activities as those of the polypeptide of the present invention, e.g., activities for regulating central nervous functions, circulatory functions, heart functions, renal functions, urinary functions, or sensory functions, etc., as far as the precursor protein contains the same or substantially the same amino acid sequence as the amino acid sequence shown by SEQ ID NO: 18, SEQ ID NO: 19 or SEQ ID NO: 29 described above.

Furthermore, the precursor protein of the present invention can be used as an antigen for preparing an antibody to the precursor protein. As such a protein that can be used as the antigen, partial peptides such as N-terminal peptide, C-terminal peptide, a peptide at the central part, etc. of the precursor protein of the present invention may be employed as well, in addition to the precursor proteins of the present invention described above.

The partial peptide may be either a peptide containing each of the individual domains or a peptide containing a plurality of domains at the same time.

The salt of partial peptide of the precursor protein of the present invention used includes the same as the salt of the partial peptide of the precursor protein described above.

The partial peptides of the precursor protein of the present invention, its amides or esters, or salts thereof can be manufactured by publicly known synthesis methods as in the precursor protein described above or by cleaving the precursor protein of the present invention with an appropriate peptidase.

The DNA encoding the precursor protein of the present invention may be any DNA so long as it contains a DNA encoding a protein containing the same or substantially the same amino acid sequence represented by SEQ ID NO:18, SEQ ID NO:19 or SEQ ID NO:29. Also, the DNA may be any one of genomic DNA, genomic DNA library, cDNA derived from the cells or tissues described above, cDNA library derived from the cells or tissues described above and synthetic DNA. The vector used for the library may be any of bacteriophage, plasmid, cosmid, phagemid and the like. In addition, the DNA can be directly amplified by reverse transcriptase polymerase chain reaction (hereinafter abbreviated as RT-PCR technique) using an RNA fraction prepared from the cells or tissues described above.

Herein, the DNA containing a DNA encoding a protein containing the same or substantially the same amino acid sequence as that shown by SEQ ID NO: 18, SEQ ID NO: 19 or SEQ ID NO: 29 includes a DNA containing a DNA having the base sequence shown by SEQ ID NO: 15, SEQ ID NO: 16, SEQ ID NO: 17 or SEQ ID NO: 30. Further examples are a DNA containing a DNA having a base sequence with homology of at least about 50%, preferably at least about 60%, more preferably at least about 70%, further more preferably at least about 80%, further much more preferably at least about 90% and most preferably at least about 95%, to the base sequence shown by SEQ ID NO: 15, SEQ ID NO:16, SEQ ID NO:17 or SEQ ID NO:30.

Examples of the DNA containing a DNA encoding a protein containing the same or substantially the same amino acid sequence as the amino acid sequence shown by SEQ ID NO: 18, SEQ ID NO: 19 or SEQ ID NO: 29 include DNAs carrying DNAs containing (i) the base sequence represented by SEQ ID NO: 15, SEQ ID NO: 16, SEQ ID NO: 17 or SEQ ID NO: 30, in which at least 1 or 2 (preferably about 1 to about 30, more preferably about 1 to about 10 and most preferably several (1 or 2)) bases are deleted; (ii) the base sequence represented by SEQ ID NO:15, SEQ ID NO:16, SEQ ID NO:17 or SEQ ID NO:30, to which at least 1 or 2 (preferably about 1 to about 30, more preferably about 1 to about 10 and most preferably several (1 or 2)) bases are added; (iii) the base sequence represented by SEQ ID NO:15, SEQ ID NO:16, SEQ ID NO:17 or SEQ ID NO:30, in which at least 1 or 2 (preferably about 1 to about 30, more preferably about 1 to about 10 and most preferably several (1 or 2)) bases are inserted; (iv) in the amino acid sequence represented by SEQ ID NO:15, SEQ ID NO:16, SEQ ID NO:17 or SEQ ID NO:30, the base sequence in which at least 1 or 2 (preferably about 1 to about 30, more preferably about 1 to about 10 and most preferably several (1 or 2)) bases are substituted by other bases; and (v) a combination of the above base sequences.

More specifically, there are employed (1) a mammal-derived DNA that is hybridizable under stringent conditions to the sequence possessed by a DNA containing a DNA encoding a protein containing the same or substantially the same amino acid sequence as the amino acid sequence represented by SEQ ID NO: 18, SEQ ID NO: 19 or SEQ ID NO: 29; and (2) a DNA that is not hybridizable due to degeneracy of genetic code to the sequence possessed by the DNA containing a DNA encoding a protein containing the same or substantially the same amino acid sequence as the amino acid sequence represented by SEQ ID NO:18, SEQ ID NO:19 or SEQ ID NO:29 and the sequence defined in (1) but encoding a protein having the same amino acid sequence, and the like. The hybridization can be carried out by publicly known methods or by modifications of the known methods. The stringent conditions described above are, for example, conditions of 42°C, 50% formamide, 4 x SSPE (1 x SSPE = 150 mM NaCl, 10 mM $NaH_2PO_4 \cdot H_2O$, 1 mM EDTA, pH 7.4), 5 x Denhardt's solution and 0.1% SDS.

As the DNA hybridizable to the sequence possessed by a DNA containing a DNA encoding a protein containing the same or substantially the same amino acid sequence as the amino acid sequence represented by SEQ ID NO:18, SEQ ID NO:19 or SEQ ID NO:29, there are employed, e.g., a DNA containing a base sequence having homology of at least about 70%, preferably at least about 80%, more preferably at least about 90%, and most preferably at least about 95%, to the base sequence shown by SEQ ID NO:15, SEQ ID NO:16, SEQ ID NO:17 or SEQ ID NO:30, and the like.

DNA fragments containing a partial base sequence of the DNA encoding the protein containing the same or substantially the same amino acid sequence as the amino acid sequence shown by SEQ ID NO:18, SEQ ID NO:19 or SEQ ID NO:29 are also preferably used as probes for detecting DNAs.

The DNA encoding the precursor protein of the present invention may also be manufactured by the following genetic engineering techniques as in the polypeptide of the present invention described above.

The DNA encoding the precursor protein of the present invention or the precursor protein of the present invention can be used: (1) for the survey of physiological activities that the precursor protein of the present invention (or the polypeptide of the present invention) possesses, (2) for preparing synthetic oligonucleotide probes or primers for PCR, (3) for acquiring DNAs encoding the polypeptide of the present invention, (4) for the development of the receptor-binding assay system using the expression system of a recombinant receptor protein and for the screening of compounds as candidate drugs, (5) for acquiring antibodies and antisera, (6) for the development of diagnostic agents using DNAs, RNAs, antibodies or antisera, (7) for the development of drugs such as agents for regulating central nervous functions, circulatory functions, heart functions, renal functions, urinary functions, sensory functions, etc., (8) for gene therapy, and the like.

In particular, the receptor-binding assay system using the expression system of recombinant SENR described hereinafter can be used for screening SENR agonists or antagonists specific to a warm-blooded animal such as human. These agonists or antagonists may be employed as agents for the prevention/treatment of various diseases.

Further with regard to (7) described above, when an SENR is expressed in the central nervous system, the circulatory system or the cardiac, renal, urinary organ or sensory organ system, the SENR recognizes the precursor protein of the present invention or the DNA encoding the same as a ligand and is thus useful as safe and low toxic drug. Since the precursor protein of the present invention or the DNA encoding the same participates in the actions for regulating the central nervous functions, circulatory functions, heart functions, renal functions, urinary functions, sensory functions, etc., the precursor protein or DNA can be used as a therapeutic and prophylactic drug for, e.g., senile dementia, cerebrovascular dementia, dementia associated with genealogical retroplastic diseases (e.g., Alzheimer's disease, Parkinson's disease, Pick's disease, Huntington's disease, etc.), hypertension (hypotension), renal diseases (e.g., chronic renal failure, nephritis, etc.), cardiac diseases (e.g., heart failure, acute myocardial infarction, etc.), pollakiuria, urinary incontinence, deafness, dysosmia, optical abnormality, or the like.

When the precursor protein of the present invention or the DNA encoding the same is used as the drugs described above, its pharmaceutical preparations can be prepared in a conventional manner. The precursor protein of the present invention or the DNA encoding the same can be used orally, for example, in the form of tablets with sugar coating or enteric coating, if necessary and desired, capsules, elixirs, microcapsules etc., or parenterally in the form of injectable preparations such as a sterile solution, a suspension, etc. in water or with other pharmaceutically acceptable liquid. These preparations can be manufactured by mixing the compound described above with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

As additives miscible with tablets, capsules etc., the additives given hereinabove may be employed.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80(™) and HCO-50), etc. Examples of the oily medium include sesame oil, soybean oil, etc., which may also be used in combination with a dissolution aid such as benzyl benzoate, benzyl alcohol, etc.

The aqueous medium for injection may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, or the like. The thus-prepared liquid for injection is normally filled in an appropriate ampoule.

Since the thus obtained pharmaceutical preparation is safe and low toxic, the preparation can be administered to, e.g., human or other mammals (e.g., mouse, rat, guinea pig, rabbit, sheep, swine, bovine, cat, dog, monkey, etc.).

The dose of the precursor protein of the present invention or the DNA encoding the same varies depending on condition, etc. In oral administration, the dose is normally about 0.1 mg to about 100 mg, preferably about 1.0 to about 50 mg, and more preferably about 1.0 to about 20 mg per day for the adult patient with heart failure (as 60 kg body weight). In parenteral administration, the single dose varies depending on subject to be administered, target organ, conditions, method for administration, etc. but in the form of injection, it is advantageous to administer the active ingredient intravenously at a daily dose of about 0.01 to about 30 mg, preferably about 0.1 to about 20 mg, and more preferably about 0.1 to about 10 mg for the adult patient with heart failure (as 60 kg body weight). For other animal species, the corresponding dose as converted per 60 kg body weight can be administered.

As the SENR in the present invention, there are SENR described in Tal, M. et al., Biochem. Biophys. Res. Commun., 209, 752-759, 1995, SENR described in Marchese, A., Genomics, 29, 335-344, 1995 and SENR described in EP 859052, as well as an SENR characterized by containing the same or substantially the same amino acid sequence shown by SEQ ID NO:9 or SEQ ID NO:26, or salts thereof, and an SENR which is a protein containing the amino acid sequence shown by SEQ ID NO:9 or SEQ ID NO:26 wherein at least 1 to 30, preferably at least 1 to 10, inclusive, amino acids are deleted; a protein containing the amino acid sequence shown by SEQ ID NO:9 or SEQ ID NO:26, to (or into) which at least 1 to 30, preferably at least 1 to 10, inclusive, amino acids are added (or inserted); or a protein containing the amino acid sequence shown by SEQ ID NO:9 or SEQ ID NO:26 wherein at least 1 to 30, preferably at least 1 to 10, inclusive, amino acids are substituted with other amino acids; or salts thereof.

As the partial peptide of the SENR of the present invention used in the present invention, any partial peptide can be used so long as it is a partial peptide of the SENR of the present invention. For example, a part of the SENR molecule of the present invention which is exposed to the outside of a cell membrane can be used so long as it has an activity for binding to the polypeptide of the present invention.

These SENRs or partial peptides thereof used in the present invention can be manufactured by the same methods as described in Tal, M. et al., Biochem. Biophys. Res. Commun., 209, 752-759, 1995, Marchese, A., Genomics, 29, 335-344, 1995 or EP 859052, or by modifications of these methods. Alternatively, the SENR or its partial peptides may also be manufactured by the same methods as applied to the polypeptide of the present invention *supra*.

Examples of the salts of SENR or their partial peptides used in the present invention include those as given for the salts of the polypeptide of the present invention described above.

The DNA encoding the SENR or its partial peptide used in the present invention may be any DNA so long as it contains a DNA encoding the SENR or its partial peptide described above. The DNA may be any one of genomic DNA, genomic DNA library, cDNA derived from the cells or tissues described above, cDNA library derived from the cells or tissues described above and synthetic DNA. The vector used for library may also be any of bacteriophage, plasmid, cosmid, phagemid and the like. In addition, the DNA can be directly amplified by RT-PCR technique using an RNA fraction prepared from the cells or tissues described above. The DNA encoding the SENR or its partial peptide employed in the present invention may also be obtained by the same methods as described in Tal, M. et al., Biochem. Biophys. Res. Commun., 209, 752-759, 1995, Marchese, A., Genomics, 29, 335-344, 1995 or EP 859052, or by modifications of these methods.

Applications of the polypeptide of the present invention, its precursor proteins, the DNA encoding the polypeptide or the precursor protein and antibodies are specifically described below.

(1) Prophylactic/therapeutic agent for polypeptide deficiency

The DNA encoding the polypeptide of the present invention may also be employed as a prophylactic/therapeutic agent for deficiency of the polypeptide or SENR, depending upon the activities of the polypeptide of the present invention and its precursor protein on the SENR.

When a patient has a reduced level of the polypeptide of the present invention, its precursor protein or SENR in his or her body so that the patient cannot expect the ligand physiological activities (activities for regulating central nervous functions, circulatory functions, heart functions, renal functions, urinary functions, or sensory functions, etc.), the activity of the polypeptide or its precursor protein of the present invention can be provided sufficiently or properly for the patient, (a) by administering the DNA encoding the polypeptide of the present invention or its precursor protein to the patient to express the same, or (b) by inserting the DNA encoding the polypeptide or its precursor protein of the present invention into brain cells, etc. to express the polypeptide or its precursor protein and transplanting the brain cells to the patient thereby to increase the level of the polypeptide or its precursor protein in brain cells of the patient. Therefore, the DNA encoding the polypeptide of the present invention or its precursor protein can be employed as a safe and low toxic prophylactic/therapeutic agent for deficiency of the polypeptide or its precursor protein.

Where the DNA described above is used as the prophylactic/therapeutic agent *supra,* the DNA alone is administered, or the DNA is inserted into an appropriate vector such as retrovirus vector, adenovirus vector, adenovirus-associated virus vector, etc. and then administered, by the same means as in the case of using the DNA encoding the polypeptide of the present invention, its precursor protein, or partial peptides thereof as drugs described above.

(2) Method for quantification of SENR to polypeptide

Since the polypeptide of the present invention or its precursor protein is capable of binding to the SENR or its salts or its partial peptides or salts thereof, the *in vivo* level of the SENR or its salts or the partial peptides of SENR or salts thereof can be quantified with a good sensitivity.

This method for quantification can be used in combination with, e.g., the competitive method. That is, the level of SENR or its salts, or the partial peptides of SENR or its salts in a sample fluid can be measured by bringing the sample fluid in contact with the polypeptide of the present invention or its precursor protein.

Specifically, the quantification can be performed by the following method (1) or (2) publicly known or its modifications:

(1) Hiroshi Irie (ed.): "Radioimmunoassay" (1974, published by Kodansha, Japan); and
(2) Hiroshi Irie (ed.): "Radioimmunoassay, Second Series" (1979, published by Kodansha, Japan).
(3) Method for screening a compound that alters the binding property between an SENR and the polypeptide of the present invention or its precursor protein (hereinafter sometimes referred to as the ligand or polypeptide)

By using the SENR or its salts or its partial peptides or salts thereof, or by constructing the expression system of recombinant SENR and using the receptor-binding assay system using the expression system, compounds (e.g., peptides, proteins, non-peptide compounds, synthetic compounds, fermentation products, etc.) or salts thereof that alter the binding property of the polypeptide or its precursor protein to the SENR can be screened. Examples of such compounds include compounds showing SENR-mediated cell-stimulating activities (e.g., the activities that accelerate or suppress release of arachidonic acid, release of acetylcholine, release of intracellular $Ca^{2+}$, intracellular cAMP production, intracellular cGMP production, inositol phosphate production, changes in cell membrane potential, phosphorylation of intracellular proteins, activation of c-fos, pH reduction, etc.)(namely, SENR agonists) and compounds having no such cell-stimulating activities (namely, SENR antagonists). The term "alter the binding property to the ligand" includes both cases that suppresses the binding to the ligand and that accelerates the binding to the ligand.

In the screening method described above, in addition to those described above, the polypeptide containing the amino acid sequence shown by SEQ ID NO:22, its amides or esters, or salts thereof as well as its precursor protein or salts thereof are employed as the polypeptide of the present invention or its precursor protein.

The polypeptide containing the amino acid sequence shown by SEQ ID NO:22, its amides or esters, or salts thereof as well as its precursor protein or salts thereof can be manufactured by the same method as in the polypeptide of the present invention or its salts or amides, or esters thereof, and its precursor protein or salts thereof described above.

The DNA encoding the polypeptide containing the amino acid sequence shown by SEQ ID NO:22 or its precursor protein may be any DNA so long as it contains a DNA encoding the polypeptide containing the amino acid sequence shown by SEQ ID NO:22 or its precursor protein. The DNA may also be any one of genomic DNA, genomic DNA library, cDNA derived from the cells or tissues described above, cDNA library derived from the cells or tissues described above and synthetic DNA. The vector used for the library may be any of bacteriophage, plasmid, cosmid, phagemid and the like. In addition, the DNA can be directly amplified by the RT-PCR technique using an RNA fraction prepared from the cells or tissues described above. The DNA encoding the polypeptide containing the amino acid sequence shown by SEQ ID NO:22 or its precursor protein, which is employed in the present invention, can be obtained by the same method as in the DNA encoding the polypeptide of the present invention or its precursor protein.

In the following section describing the method for screening the compounds that alter the binding property between an SENR and the polypeptide of the present invention or its precursor protein, the term "polypeptide of the present invention" is used to mean the "polypeptide of the present invention" and "polypeptide containing the amino acid sequence shown by SEQ ID NO:22" described above, and the term "precursor of the polypeptide of the present invention" is used to mean the "precursor of the polypeptide of the present invention" and "precursor of the polypeptide containing the amino acid sequence shown by SEQ ID NO:22" described above.

Further in the following section describing the method for screening the compounds that alter the binding property between an SENR and the polypeptide of the present invention or its precursor protein, the term "DNA encoding the polypeptide of the present invention" is used to mean the "DNA encoding the polypeptide of the present invention" and "DNA encoding polypeptide containing the amino acid sequence shown by SEQ ID NO:22" described above, and the term "DNA encoding the precursor of the polypeptide of the present invention" is used to mean the "DNA encoding the precursor of the polypeptide of the present invention" and "DNA encoding the precursor of the polypeptide containing the amino acid sequence shown by SEQ ID NO:22" described above.

That is, the present invention provides a method for screening a compound or its salts that alter the binding property between the polypeptide of the present invention or its precursor protein and the SENR described above, which comprises comparing (i) the case that the polypeptide of the present invention or its precursor protein is brought into contact with the SENR or its salts or the partial peptide of the SENR or its salts, and (ii) the case that the polypeptide of the present invention or its precursor protein and a test compound are brought into contact with the aforesaid SENR or its salts or the partial peptide of the SENR or its salts.

In the screening method of the present invention, the comparison is made by measuring, e.g., the amount of the ligand bound to the SENR or its partial peptide, the cell-stimulating activity, etc., (i) when the polypeptide of the present invention or its precursor protein is brought into contact with the aforesaid SENR or its salts or the partial peptide of the SENR or its salts, and (ii) when the polypeptide of the present invention or its precursor protein and a test compound are brought into contact with the aforesaid SENR or its salts or the partial peptide of the SENR or its salts; and comparing (i) and (ii).

More specifically, the screening method of the present invention provides the following features.

(1) A method for screening a compound or salts thereof that alter the binding property between the polypeptide of the present invention or its precursor protein and an SENR, which comprises measuring the binding amounts of the labeled polypeptide of the present invention or its labeled precursor protein bound to the aforesaid SENR or its salts or the partial peptide of the SENR or its salts, when the labeled polypeptide of the present invention or its labeled precursor protein is brought in contact with the SENR or its salts or the partial peptide of the SENR or its salts and when the labeled polypeptide of the present invention or its labeled precursor protein and a test compound are brought in contact with the SENR or its salts or the partial peptide of the SENR or its salts and, comparing the binding amounts.

(2) A method for screening a compound or salts thereof that alter the binding property between the polypeptide of the present invention or its precursor protein and an SENR, which comprises measuring the binding amounts of the labeled polypeptide of the present invention or its labeled precursor protein bound to an SENR-containing cell or membrane fraction of the cell, when the labeled polypeptide of the present invention or its labeled precursor protein is brought in contact with the SENR-containing cell or membrane fraction of the cell and when the labeled polypeptide of the present invention or its labeled precursor protein and a test compound are brought in contact with the SENR-containing cell or membrane fraction of the cell and, comparing the binding amounts.

(3) A method for screening a compound or salts thereof that alter the binding property between the polypeptide of the present invention or its precursor protein and an SENR, which comprises measuring the binding amounts of the labeled polypeptide of the present invention or its labeled precursor protein bound to the aforesaid SENR, when the labeled polypeptide of the present invention or its labeled precursor protein is brought in contact with the SENR expressed on a cell membrane by culturing a transformant containing the DNA encoding the SENR, and when the labeled polypeptide of the present invention or its labeled precursor protein and a test compound are brought in contact with the SENR expressed on a cell membrane by culturing a transformant containing the DNA encoding the SENR and, comparing the binding amounts.

(4) A method for screening a compound or salts thereof that alter the binding property between the polypeptide of the present invention or its precursor protein and an SENR, which comprises measuring SENR-mediated cell stimulating activities (e.g., activities that accelerate or suppress release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.), when a compound for activating the SENR (e.g., the polypeptide of the present invention or its precursor protein) is brought in contact with a cell containing the SENR and when a compound for activating the SENR and a test compound are brought in contact with the cell containing the SENR and, comparing the SENR-mediated cell stimulating activities.

(5) A method for screening a compound or salts thereof that alter the binding property between the polypeptide of the present invention or its precursor protein and an SENR, which comprises measuring SENR-mediated cell stimulating activities (e.g., activities that accelerate or suppress release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.), when a compound for activating the SENR (e.g., the polypeptide of the present invention or its precursor protein) is brought in contact with the SENR expressed on a cell membrane by culturing a transformant containing the DNA encoding the SENR and when a compound for activating the SENR and a test compound are brought in contact with the SENR expressed on a cell membrane by culturing a transformant containing the DNA encoding the SENR and, comparing the SENR-mediated cell stimulating activities.

The screening methods of the present invention are specifically described below.

First, the SENR used for the screening methods of the present invention may be any SENR so long as it contains the aforesaid SENR or the partial peptide of the SENR. Preferred SENR includes membrane fractions from the organs of human or warm-blooded animals. Since it is very difficult to obtain human-derived organs, the SENR expressed in large quantities by use of recombinants is suitable as an SENR for the screening.

For manufacturing the SENR, the methods *supra,* etc. are used.

When the SENR-containing cells or cell membrane fractions are employed in the screening methods of the present invention, the cells or cell membrane fractions may be prepared by the method which will be described hereinafter.

Where the SENR-containing cells are used, the cells may be fixed using glutaraldehyde, formalin, etc. The fixation can be made by a publicly known method.

The cells containing the SENR refer to host cells that have expressed the SENR. Examples of the host cells include *Escherichia coli*, *Bacillus subtilis,* yeast, insect cells, animal cells, etc., described above.

The cell membrane fraction is a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 minute to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the SENR expressed and membrane components such as cell-derived phospholipids and membrane proteins.

The amount of the SENR in the cells containing the SENR-containing cells or in the membrane fractions is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) for screening the compound that alters the binding property between the polypeptide of the present invention or its precursor protein and an SENR, an appropriate SENR fraction and the labeled polypeptide of the present invention or its precursor protein are employed. The SENR fraction is preferably a naturally occurring SENR fraction or a recombinant SENR fraction having an activity equivalent to that of the natural SENR fraction. Herein, the term "equivalent activity" is intended to mean a ligand binding activity, or the like, equivalent to that of the natural SENR fraction. As the labeled ligand, there are used a labeled ligand, a labeled ligand analog compound, etc. For example, a ligand which is labeled with [$^3$H], [$^{125}$I], [$^{14}$C], [$^{35}$S], etc. can be utilized.

Specifically, the compound that alters the binding property between the polypeptide of the present invention or its precursor protein and the SENR is screened by the following procedures. First, a standard receptor preparation is prepared by suspending SENR-containing cells or membrane fractions thereof in a buffer appropriate for the screening. Any buffer can be used so long as it does not interfere with ligand-receptor binding, such buffers including a phosphate buffer or a Tris-HCl buffer having pH of 4 to 10 (desirably pH of 6 to 8). For the purpose of minimizing non-specific binding, a surfactant such as CHAPS, Tween-80™ (Kao-Atlas Inc.), digitonin, deoxycholate, etc. may optionally be added to the buffer. Further for the purpose of suppressing the degradation of the receptor or the polypeptide of the present invention by a protease, a protease inhibitor such as PMSF, leupeptin, E-64 (manufactured by Peptide Institute, Inc.) and pepstatin may also be added. A given amount (5,000 to 500,000 cpm) of the labeled polypeptide of the present invention is added to 0.01 ml to 10 ml of the receptor solution and at the same time, a test compound of $10^{-10}$ to $10^{-7}$ M is allowed to be co-present. To determine the amount of non-specific binding (NSB), a reaction tube added with unlabeled polypeptide of the present invention in a large excess is also provided. The reaction is carried out at 0 to 50°C, preferably 4 to 37°C for 20 minutes to 24 hours, preferably 30 minutes to 3 hours. After completion of the reaction, the reaction mixture is filtrated through a glass fiber filter paper, etc. and washed with a suitable volume of the same buffer. The residual radioactivity in the glass fiber filter paper is then measured by means of a liquid scintillation counter or a γ-counter. When the count (B-NSB$_0$) obtained by subtracting non-specific binding (NSB) from the count (B$_0$) in the absence of an antagonistic substance is taken as 100%, the test compound giving a non-specific binding (B-NSB) of, e.g., 50% or less can be selected as a candidate substance having a competitive inhibitory activity.

The method (4) or (5) described above for screening the compound that alters the binding property between the polypeptide of the present invention or its precursor protein and an SENR can be performed as follows. The SENR-mediated cell-stimulating activities (e.g., the activities that promote or suppress release of arachidonic acid, release of acetylcholine, release of intracellular $Ca^{2+}$, intracellular cAMP production, intracellular cGMP production, inositol phosphate production, change in cell membrane potential, phosphorylation of intracellular proteins, activation of c-fos, pH reduction, etc.) may be assayed by a publicly known method, or using an assay kit commercially available. Specifically, the SENR-containing cells are first cultured on a multiwell plate, etc. Prior to the screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by the respective procedures. Where it is difficult to detect the production of the index substance (e.g., arachidonic acid) for the cell-stimulating activity due to degrading enzymes contained in the cells, an inhibitor against such a degradation enzyme may be added prior to the assay. For detecting activities such as the cAMP production suppression activity, the baseline production in the cells is increased by forskolin or the like. Then, the suppressing effect on the increased baseline production can be detected.

For screening by measurement of the cell-stimulating activity, a suitable cell in which an SENR has been expressed is required. Such a cell that the SENR of the present invention has been expressed is desirably the aforesaid recombinant SENR-expressed cell line, and the like.

Examples of the test compound include a peptide, a protein, a non-peptide compound, a synthetic compound, a fermentation product, a cell extract, a plant extract, an animal tissue extract and the like.

The kit for screening the compound or its salt of the present invention that alter the binding property between the polypeptide of the present invention or its precursor protein and an SENR comprises an SENR or its salts, partial peptides of the SENR or their salts, SENR-containing cells or membrane fractions of the SENR-containing cells, and the polypeptide of the present invention or its precursor protein.

The screening kit according to the present invention comprises, for example, the following:

1. Reagent for screening

(1) Buffers for assay and washing

Hanks' Balanced Salt Solution (manufactured by Gibco) supplemented with 0.05% of bovine serum albumin (manufactured by Sigma).

The buffers may be sterilized by filtration through a membrane filter with a 0.45 μm pore size and stored at 4°C, or may be prepared at use.

(2) SENR preparation

SENR-expressed CHO cells are subcultured at 5 x $10^5$ cells/well on a 12-well plate followed by culturing at 37°C under a 5% $CO_2$ and 95% air for 2 days.

(3) Labeled ligand

Ligand labeled with [$^3$H], [$^{125}$I], [$^{14}$C], [$^{35}$S], etc. The product dissolved in a suitable solvent or buffer is stored at 4°C or at -20°C, which will be diluted at use to 1 µM with a buffer for the assay.

(4) Standard ligand solution

The polypeptide of the present invention or its precursor protein are dissolved in PBS containing 0.1% of bovine serum albumin (manufactured by Sigma) to make the volume 1 mM and then stored at -20°C.

2. Method for assay

(1) Cells are cultured in a 12-well tissue culture plate to express the SENR. After washing the CHO cells twice with 1 ml of buffer for the assay, 490 µl of the buffer for assay is added to each well.
(2) After 5 µl of a test compound solution of $10^{-3}$ to $10^{-10}$ M is added, 5 µl of the labeled peptide of the present invention or its precursor protein is added to the system followed by incubation at room temperature for an hour. To determine the amount of the non-specific binding, 5 µl of the ligand of $10^{-3}$ M is added to the system, instead of the test compound.
(3) The reaction mixture is removed and the cells are washed three times with 1 ml each of the buffer for washing. The labeled ligand bound to the cells is dissolved in 0.2N NaOH-1% SDS and mixed with 4 ml of a liquid scintillator A (manufactured by Wako Pure Chemical).
(4) Radioactivity is measured using a liquid scintillation counter (manufactured by Beckman) and percent of the maximum binding (PMB) is calculated in accordance with the following equation:

$$PMB = [(B-NSB)/(B_0 - NSB)] \times 100$$

wherein:

PMB: percent of the maximum binding
B: value when a specimen is added
NSB: non-specific binding
$B_0$: maximum binding

The compound or salts thereof obtainable by the screening methods or by the screening kit of the present invention is the compound that alters the binding property between the polypeptide of the present invention or its precursor protein and an SENR (inhibits or accelerates the binding), specifically a compound or salts thereof having an SENR-mediated cell-stimulating activity (so-called SENR agonists) or a compound having no cell-stimulating activity (so-called SENR antagonists). Examples of the compound include a peptide, a protein, a non-peptide compound, a synthetic compound, a fermentation product, etc. and the compound may be either novel or known compound.
To determine the SENR agonists or antagonists, the following method (i) or (ii) is available.
(i) The binding assay recited in the screening methods (1) through (3) supra is performed to obtain the compound that alters the binding property between the polypeptide of the present invention or its precursor protein and an SENR (especially inhibits the binding) followed by assay for the compound to determine if the compound has the SENR-mediated cell-stimulating activity described above. The compound having the cell-stimulating activity or its salts are SENR agonists, whereas the compound having no such activity or its salts are SENR antagonists.
(ii)(a) A test compound is brought into contact with SENR-containing cells to assay the SENR-mediated cell-stimulating activity. The compound having the cell-stimulating activity or its salts are SENR agonists.
(b) The SENR-mediated cell-stimulating activity is measured both when a compound (e.g., the polypeptide of the present invention or its precursor protein, an SENR agonist, etc.) that activates an SENR is brought into contact with an SENR-containing cell and when the compound that activates the SENR and a test compound are brought into contact with the SENR-containing cell and comparison is made on the cell-stimulating activity between the two cases. The compound that can reduce the cell-stimulating activity by the SENR-activating compound or its salts are SENR antagonists.

The SENR agonists exhibit activities similar to the physiological activities of the polypeptide of the present invention or its precursor protein and are thus useful as safe and low toxic drugs likewise the polypeptide of the present invention or its precursor protein.

To the contrary, since the SENR antagonists can suppress the physiological activities that the polypeptide of the present invention possesses, they are useful as safe and low toxic drugs for suppressing the receptor activity.

The polypeptide of the present invention or its precursor protein participates in the actions for regulating the central nervous functions, circulatory functions, heart functions, renal functions, urinary functions, sensory functions, etc. Thus, the SENR agonists may be used as a therapeutic and prophylactic agent for, e.g., senile dementia, cerebrovascular dementia, dementia associated with genealogical retroplastic diseases (e.g., Alzheimer's disease, Parkinson's disease, Pick's disease, Huntington's disease, etc.), hypertension (hypotension), renal diseases (e.g., chronic renal failure, nephritis, etc.), cardiac diseases (e.g., heart failure, acute myocardial infarction, etc.), pollakiuria, urinary incontinence, deafness, dysosmia, optical abnormality, or the like.

The salt of compound obtainable by using the screening methods or kits described above is preferably a pharmaceutically acceptable salt, exemplified by salts with inorganic bases, salts with organic bases, salts with inorganic acids, salts with organic acids and salts with basic or acidic amino acids, and the like.

Preferred examples of the salts with inorganic bases include alkali metal salts such as sodium salts, potassium salts, etc.; alkaline earth metal salts such as calcium salts, magnesium salts, etc.; and aluminum salts, ammonium salts, and the like.

Preferred examples of the salts with organic bases include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.

Preferred examples of the salts with inorganic acids include salts with hydrochloric acid, hydrobromic acid, sulfuric acid, phosphoric acid, etc.

Preferred examples of the salts with organic acids include salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid, etc.

Preferred examples of the salts with basic amino acids include salts with arginine, lysine, ornithine, etc. Preferred examples of the salts with acidic amino acids include salts with aspartic acid, glutamic acid, etc.

Where the compound or salts thereof obtainable by the screening methods or kits of the present invention are used as the drugs described above, a conventional means may be applied to making pharmaceutical preparations and administering such preparations.

(4) Manufacture of antibodies to the polypeptide of the present invention or its precursor protein or antisera

Antibodies (e.g., polyclonal antibodies, monoclonal antibodies) to the polypeptide of the present invention or its precursor protein, or antisera can be manufactured by publicly known methods for manufacturing antibodies or antisera, using as antigen the polypeptide of the present invention or its precursor protein.

For example, polyclonal antibodies can be manufactured by the method described below.

[Preparation of polyclonal antibody]

A polyclonal antibody to the polypeptide of the present invention or its precursor protein can be manufactured by publicly known methods or modifications thereof. The polyclonal antibody can be manufactured, for example, by producing the complex of an immunogen (a polypeptide antigen, etc.) and a carrier protein, immunizing a warm-blooded animal (e.g., mammals (e.g., rabbit, sheep, goat, rat, mouse, guinea pig, bovine, horse, swine), birds (e.g., chicken, dove, duck, goose, quail), etc.) as well as the method for manufacturing a monoclonal antibody described below, collecting the product containing the antibody to the polypeptide of the present invention from the immunized animal and then separating and purifying the antibody.

In the complex of an immunogen and a carrier protein used to immunize a warm-blooded animal, a type of the carrier protein and a mixing ratio of the carrier to the hapten (the polypeptide of the present invention or its partial peptide) may be any type and in any ratio, as long as the antibody is efficiently produced to the hapten immunized by crosslinking to the carrier. For example, bovine serum albumin, bovine thyroglobulin, Keyhole limpet hemocyanin, etc. is coupled to the hapten in a carrier-to-hapten weight ratio of approximately 0.1 to 20, preferably about 1 to about 5.

A variety of condensation agents can be used for coupling of the carrier to the hapten. Glutaraldehyde, carbodiimide, maleimide activated ester, activated ester reagents containing thiol group or dithiopyridyl group and the like are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to about 6 weeks and about 3 to about 10 times in total.

The polyclonal antibody can be collected from the blood, ascites, etc., preferably from the blood of the warm-blooded animal immunized by the method described above.

The polyclonal antibody titer to the polypeptide of the present invention or its precursor protein in antisera can be assayed by the same procedure as that for determining the antibody titer in hybridoma culture supernatant described hereinafter. The separation and purification of the antibody can be made, following the method for the separation and purification of immunoglobulins performed as in the separation and purification of monoclonal antibodies described below.

A monoclonal antibody can also be manufactured according to the method described below.

[Preparation of monoclonal antibody]

(a) Preparation of monoclonal antibody-producing cells

The polypeptide of the present invention or its precursor protein is administered to warm-blooded animals (for example, mammals (e.g., rabbit, sheep, goat, rat, mouse, guinea pig, bovine, horse, swine), birds (e.g., chicken, dove, duck, goose, quail, etc.) and the like) either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every 2 to 6 weeks and 2 to 10 times in total.

In preparing monoclonal antibody-producing cells, the aforesaid warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells to give monoclonal antibody-producing hybridomas. The antibody titer in antisera may be assayed, for example, by reacting the labeled polypeptide of the present invention or its precursor protein, or partial peptides thereof, which will be described later, with the antiserum followed by assaying the activity of the labeling agent bound to the antibody. The fusion may be carried out, for example, by the known method by Koehler and Milstein [(Nature, 256, 495, (1975)]. Examples of the fusion accelerator used include polyethylene glycol (PEG), Sendai virus, etc., of which PEG is preferably employed.

Examples of the myeloma cells are NS-1, P3U1, SP2/0, AP-1, etc. In particular, P3U1 is preferably employed. A preferred ratio of the count of the antibody-producing cells (spleen cells) used to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, efficient cell fusion can be carried out.

Various methods can be used for the screening of a monoclonal antibody-producing hybridoma to the polypeptide of the present invention or its precursor protein. Examples of such methods include a method which comprises adding the supernatant of a hybridoma to a solid phase (e.g., microplate) adsorbed with the polypeptide of the present invention or its precursor protein as an antigen directly or together with a carrier, adding an anti-immunoglobulin antibody (where mouse cells are used for the cell fusion, an anti-mouse immunoglobulin antibody is used) labeled with a radioactive substance or an enzyme, or Protein A and detecting the monoclonal antibody to the polypeptide of the present invention or its precursor protein bound to the solid phase; a method which comprises adding the supernatant of hybridoma to a solid phase adsorbed with an anti-immunoglobulin antibody or Protein A, adding the polypeptide of the present invention labeled with a radioactive substance or an enzyme and detecting the monoclonal antibody against the polypeptide of the present invention or its precursor protein bound to the solid phase, and so on.

The monoclonal antibody to the polypeptide of the present invention or its precursor protein can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like may be used for the selection and growth medium. The cultivation is carried out generally at 20°C to 40°C, preferably at 37°C, for about 5 days to about 3 weeks, preferably 1 to 2 weeks, normally in 5% $CO_2$. The antibody titer of the culture supernatant of the hybridoma can be determined as in the assay for the antibody titer in the antisera described above.

(b) Purification of monoclonal antibody

The monoclonal antibody to the polypeptide of the present invention or its precursor protein can be separated and purified, as applied to conventional separation and purification of polyclonal antibodies, according to the methods for separation and purification of immunogloblins [e.g., salting-out, alcohol precipitation, isoelectric point precipitation, electrophoresis, adsorption/desorption with ion exchangers (e.g., DEAE), ultracentrifugation, gel filtration, or a specific purification method which comprises collecting only an antibody by means of an antigen-binding solid phase or, an activated adsorbent such as Protein A or Protein G and dissociating the binding to obtain the antibody].

The antibodies to the polypeptide of the present invention or its precursor protein manufactured by the methods (a) and (b) described above are capable of specifically recognizing the polypeptide of the present invention or its precursor protein, respectively. Thus, these antibodies can be used for quantification of the polypeptide of the present invention or its precursor protein in a test sample fluid, in particular, for quantification by sandwich immunoassay.

That is, the present invention provides:

(i) a method for quantification of the polypeptide of the present invention or its precursor protein in a test sample fluid, which comprises competitively reacting antibodies that are reactive with the polypeptide of the present invention or its precursor protein, a test sample fluid and the labeled polypeptide of the present invention or the labeled precursor protein of the present invention, and measuring the ratio of the labeled polypeptide of the present invention or its labeled precursor protein bound to the antibodies; and,

(ii) a method for quantification of the polypeptide of the present invention or its precursor protein in a test sample fluid, which comprises reacting the test sample fluid simultaneously or continuously with an antibody immobilized on a carrier and a labeled antibody, and then measuring the activity of the labeling agent on the insolubilized carrier, wherein one antibody is capable of recognizing the N-terminal region of the polypeptide of the present invention or its precursor protein and another antibody is capable of recognizing the C-terminal region of the polypeptide of the present invention or its precursor protein.

The polypeptide of the present invention or its precursor protein may be assayed using the monoclonal antibody capable of recognizing the polypeptide of the present invention or its precursor protein. Moreover, the monoclonal antibody may also be used for detection by means of tissue staining, etc. For these purposes, the antibody molecule per se may be used or $F(ab')_2$, Fab' or Fab fractions of the antibody molecule may be used as well. There is no particular limitation for the assay method using the antibody of the present invention. Any method may be used so far as it relates to a method in which the amount of antibody, antigen or antibody-antigen complex can be detected by a chemical or physical means, depending on or corresponding to the amount of antigen (e.g., the amount of the polypeptide) in a test sample fluid to be assayed, and then calculated using a standard curve prepared by a standard solution containing a known amount of the antigen. Advantageously used are, for example, nephrometry, competitive method, immunometric method and sandwich method. In terms of sensitivity and specificity, the sandwich method, which will be described later, is particularly preferred.

Examples of the labeling agent used in the assay method using a labeling substance are radioisotopes, enzymes, fluorescent substances and luminescent substances, etc. Examples of the radioisotope are [$^{125}I$], [$^{131}I$], [$^{3}H$], [$^{14}C$], etc. Preferred examples of the enzyme are those that are stable and have a high specific activity, including β-galactosidase, β-glucosidase, an alkaline phosphatase, a peroxidase and malate dehydrogenase. Examples of the fluorescent substance are fluorescamine, fluorescein isothiocyanate, etc. Examples of the luminescent substance are luminol, a luminol derivative, luciferin, lucigenin, etc. Furthermore, the biotin-avidin system may be used as well for binding between an antibody or antigen and a labeling agent.

Upon immobilization of antigens or antibodies, physical adsorption may be used. Alternatively, chemical binding that is conventionally used to immobilize proteins or enzymes may be used as well. Examples of the carrier include insoluble polysaccharides such as agarose, dextran and cellulose; synthetic resins such as polystyrene, polyacrylamide, silicone, etc.; glass; and the like.

In the sandwich method, a test sample fluid is reacted with an immobilized anti-polypeptide antibody (first reaction), then reacted with another labeled anti-polypeptide antibody (second reaction) and the activity of the labeling agent on the insoluble carrier is measured, whereby the amount of the polypeptide in the test sample fluid can be quantified. The first and second reactions may be carried out in a reversed order or simultaneously or they may be conducted with an interval.

The type of the labeling agent and the method for immobilization may be similar to those described hereinabove. In the immunoassay by the sandwich method, it is not always necessary that the antibody used for the labeled antibody and for the solid phase should be one type or one species but a mixture of two or more antibodies may also be used for the purpose of improving the measurement sensitivity, etc.

In the method for assaying the polypeptide of the present invention or its precursor protein by the sandwich method according to the present invention, antibodies wherein the binding sites to the polypeptide of the present invention or its precursor protein are different from one another are preferably used as the anti-polypeptide antibody or its anti-precursor protein antibody used for the first and the second reactions. Thus, the antibodies used in the first and the second reactions are those wherein, when the antibody used in the second reaction recognizes the C-terminal region of the polypeptide of the present invention or its precursor protein, the antibody recognizing the site other than the C-terminal regions, e.g., recognizing the N-terminal region, is preferably used in the first reaction.

The antibody to the polypeptide of the present invention or its precursor protein may be used in an assay system other than the sandwich method, such as a competitive method, an immunometric method, a nephrometry, etc. In a competitive method, an antigen in the test sample fluid and a labeled antigen are made to react with an antibody in a competitive manner, then an unreacted labeled antigen (F) and a labeled antigen binding with an antibody (B) are separated (B/F separation) and the labeled amount of any of B and F is measured, whereby the amount of the antigen in the test sample fluid is quantified. In the methods for such reactions, there are a liquid phase method in which a soluble antibody is used as the antibody and the B/F separation is conducted by polyethylene glycol, a second antibody to the said antibody, etc. is used; and a solid phase method in which an immobilized antibody is used as the first antibody or, a soluble antibody is used as the first antibody while an immobilized antibody is used as the second antibody.

In the immunometric method, an antigen in the test sample fluid and an immobilized antigen are subjected to a competitive reaction with a given amount of a labeled antibody followed by separating into solid and liquid phases; or the antigen in the test sample fluid is reacted with an excess amount of labeled antibody, then an immobilized antigen is added to bind an unreacted labeled antibody to the solid phase and the solid phase is separated from the liquid phase. After that, the labeled amount of any of the phases is measured to determine the amount of the antigen in the test sample fluid.

In a nephrometry, the amount of insoluble sediment, which is produced as a result of the antigen-antibody reaction in a gel or in a solution, is measured. Even when the amount of an antigen in a test sample fluid is small and only a small amount of the sediment is obtained, a laser nephrometry utilizing laser scattering can be suitably used.

In applying each of those immunoassays to the assay method for the present invention, it is not necessary to set up any special condition, operation, etc. therefor. The assay system for the polypeptide, its precursor protein or their partial peptides of the present invention may be constructed in addition to conditions or operations conventionally used for each of the methods, taking into account the technical consideration of one skilled in the art. For the details of such conventional technical means, a variety of reviews, reference books, etc. may be referred to [for example, Hiroshi Irie (ed.): "Radioimmunoassay" (published by Kodansha, 1974)]; Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.].

As described above, the polypeptide of the present invention or its precursor protein can be quantified with high sensitivity, using the antibody to the polypeptide of the present invention or its precursor protein.

By quantifying the polypeptide of the present invention or its precursor protein in a test sample fluid, diagnosis of diseases in which the polypeptide of the present invention or its precursor protein participates can be conducted. Examples of such diseases that the polypeptide of the present invention participates in are senile dementia, cerebrovascular dementia, dementia associated with genealogical retroplastic diseases (e.g., Alzheimer's disease, Parkinson's disease, Pick's disease, Huntington's disease, etc.), hypertension (hypotension), renal diseases (e.g., chronic renal failure, nephritis, etc.), cardiac diseases (e.g., heart failure, acute myocardial infarction, etc.), pollakiuria, urinary incontinence, deafness, dysosmia, optical abnormality, and the like. A test sample fluid can be prepared from mammals to be tested (e.g., human, rabbit, sheep, goat, rat, mouse, guinea pig, bovine, horse, swine) by publicly known methods. Examples of the test sample fluid include blood, lymph, urine, etc.

In the specification and drawings, when bases, amino acids, etc. are shown by abbreviations, the codes of bases and amino acids are denoted in accordance with the IUPAC-IUB Commission on Biochemical Nomenclature or by the codes conventionally used in the art, examples of which are shown below. With respect to amino acids that may have their optical isomers, L form is presented unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |

(continued)

| | |
|---|---|
| Y | thymine or cytosine |
| N | thymine, cytosine, adenine or guanine |
| R | adenine or guanine |
| M | cytosine or adenine |
| W | thymine or adenine |
| S | cytosine or guanine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediaminetetraacetic acid |
| SDS | sodium dodecyl sulfate |
| TFA | trifluoroacetic acid |
| EIA | enzyme immunoassay |
| Gly or G | glycine |
| Ala or A | alanine |
| Val or V | valine |
| Leu or L | leucine |
| Ile or I | isoleucine |
| Ser or S | serine |
| Thr or T | threonine |
| Cys or C | cysteine |
| Met or M | methionine |
| Glu or E | glutamic acid |
| Asp or D | aspartic acid |
| Lys or K | lysine |
| Arg or R | arginine |
| His or H | histidine |
| Phe or F | phenylalanine |
| Tyr or Y | tyrosine |
| Trp or W | tryptophan |
| Pro or P | proline |
| Asn or N | asparagine |
| Gln of Q | glutamine |
| pGlu | pyroglutamic acid |
| Me | methyl group |
| Et | ethyl group |
| Bu | butyl group |
| Ph | phenyl group |
| Tc | thiazoline-4(R)-carboxamide group |
| Bom | benzyloxymethyl |
| NMP | N-methylpyrrolidone |
| PAM | phenylacetamidomethyl |

The substituents, protecting groups and reagents which are frequently used in the present specification are denoted by the following symbols.

| | |
|---|---|
| Tos | p-toluenesulfonyl |

(continued)

| | |
|---|---|
| HONB | N-hydroxy-5-norbornene-2,3-dicarboximide |
| Bzl | benzyl |
| Z | benzyloxycarbonyl |
| Br-Z | 2-bromobenzyloxycarbonyl |
| Cl-Z | 2-chlorobenzyloxycarbonyl |
| Boc | t-butoxycarbonyl |
| HOBt | 1-hydroxybenztriazole |
| DCC | N,N'-dichlorohexylcarbodiimide |
| TFA | trifluoroacetic acid |
| Fmoc | N-9-fluorenylmethoxycarbonyl |
| DNP | dinitrophenol |
| Bum | t-butoxymethyl |
| Trt | trityl |
| MeBzl | 4-methylbenzyl |
| CHO | formyl |
| NMP | N-methylpyrrolidone |
| OcHex | cyclohexyl ester |

The sequence identification numbers in the sequence listing of the specification indicate the following sequences, respectively.

[SEQ ID NO:1]

This represents a synthetic DNA used in screening for cDNA encoding rat SENR protein.

[SEQ ID NO:2]

This represents a synthetic DNA used in screening for cDNA encoding rat SENR protein.

[SEQ ID NO:3]

This represents an entire base sequence of rat SENR protein cDNA wherein the base sequence recognized by restriction enzyme Sal I is added at the 5' end and the base sequence recognized by restriction enzyme Spe I is added at the 3' end.

[SEQ ID NO:4]

This represents a riboprobe used to measure the expression amount of SENR receptor protein mRNA in each clone of the SENR-expression CHO cell line.

[SEQ ID NO:5]

This represents an amino acid sequence obtained from N-terminal amino acid sequence analysis of a ligand peptide to the SENR purified from swine spinal cord.

[SEQ ID NO:6]

This represents an amino acid sequence obtained from N-terminal amino acid sequence analysis of a ligand peptide to the SENR purified from swine spinal cord.

[SEQ ID NO:7]

This represents an amino acid sequence determined from N-terminal amino acid sequence analysis of a ligand peptide to the SENR purified from swine spinal cord.

[SEQ ID NO:8]

This represents an amino acid sequence determined from N-terminal amino acid sequence analysis of a ligand peptide to the SENR purified from swine spinal cord.

[SEQ ID NO:9]

This represents an amino acid sequence of rat SENR protein identified in EXAMPLE 2.

[SEQ ID NO:10]

This represents a synthetic DNA used to obtain a partial sequence of cDNA encoding swine SENR ligand precursor protein.

[SEQ ID NO:11]

This represents a synthetic DNA used to obtain a partial sequence of cDNA encoding swine SENR ligand precursor protein.

[SEQ ID NO:12]

This represents a base sequence of cDNA encoding a part of swine SENR ligand precursor protein.

[SEQ ID NO:13]

This represents a synthetic DNA probe used for screening of cDNA encoding swine SENR ligand precursor protein.

[SEQ ID NO:14]

This represents a synthetic DNA probe used for screening of cDNA encoding swine SENR ligand precursor protein.

[SEQ ID NO:15]

This represents an entire base sequence of swine SENR ligand precursor protein cDNA.

[SEQ ID NO:16]

This represents an entire base sequence of swine SENR ligand precursor protein cDNA.

[SEQ ID NO:17]

This represents an entire base sequence of swine SENR ligand precursor protein cDNA.

[SEQ ID NO:18]

This represents an entire amino acid sequence of swine SENR ligand precursor protein.

[SEQ ID NO:19]

This represents an entire amino acid sequence of swine SENR ligand precursor protein.

[SEQ ID NO:20]

This represents a base sequence of cDNA encoding a part of bovine SENR ligand precursor protein.

[SEQ ID NO:21]

This represents an amino acid sequence of bovine SENR ligand peptide.

[SEQ ID NO:22]

This represents an amino acid sequence of human SENR ligand polypeptide (human urotensin II).

[SEQ ID NO:23]

This represents a synthetic DNA used for screening cDNA encoding human SENR protein.

[SEQ ID NO:24]

This represents a synthetic DNA used for screening cDNA encoding human SENR protein.

[SEQ ID NO:25]

This represents an entire base sequence of human SENR protein cDNA wherein the base sequence recognized by restriction enzyme Sal I is added at the 5' end and the base sequence recognized by restriction enzyme Spe I is added at the 3' end.

[SEQ ID NO:26]

This represents an entire amino acid sequence of human SENR protein identified in EXAMPLE 20.

[SEQ ID NO:27]

This represents a DNA sequence of SEQ ID NO:8 (swine ligand 2).

[SEQ ID NO:28]

This represents a DNA sequence of SEQ ID NO:21 (bovine ligand).

[SEQ ID NO:29]

This represents an entire amino acid sequence of bovine SENR ligand precursor protein.

[SEQ ID NO:30]

This represents an entire amino acid sequence of bovine SENR ligand precursor protein cDNA.

[SEQ ID NO:31]

This represents a synthetic DNA used for RACE-PCR to acquire a partial sequence of the 5' end of cDNA encoding bovine SENR ligand precursor protein.

[SEQ ID NO:32]

This represents a synthetic DNA used for RACE-PCR to acquire a partial sequence of the 5' end of cDNA encoding bovine SENR ligand precursor protein.

[SEQ ID NO:33]

This represents a base sequence of the 5' end partial sequence of cDNA encoding bovine SENR ligand precursor protein.

[SEQ ID NO:34]

This represents a synthetic DNA used for RACE-PCR to acquire a partial sequence of the 3' end of cDNA encoding bovine SENR ligand precursor protein.

[SEQ ID NO:35]

This represents a synthetic DNA used for RACE-PCR to acquire a partial sequence of the 3' end of cDNA encoding bovine SENR ligand precursor protein.

[SEQ ID NO:36]

This represents a base sequence of the 3' end partial sequence of cDNA encoding bovine SENR ligand precursor protein.

[SEQ ID NO:37]

This represents a synthetic DNA used to acquire an full-length sequence of cDNA encoding bovine SENR ligand precursor protein.

[SEQ ID NO:38]

This represents a synthetic DNA used to acquire an full-length sequence of cDNA encoding bovine SENR ligand precursor protein.

[SEQ ID NO:39]

This represents an amino acid sequence of "Haze" or long-jawed mudsucker, urotensin II peptide used as an antigen to produce an antibody capable of recognizing the C terminus of SENR ligand polypeptide.

Transformant *Escherichia coli* XL1 Blue/pZ1-puro2 containing the base sequence shown by SEQ ID NO:15, which was obtained in EXAMPLE 10 later described was on deposit with the Ministry of International Trade and Industry, Agency of Industrial Science and Technology, National Institute of Bioscience and Human Technology (NIBH) as the Accession Number FERM BP-6858 on August 23, 1999 and with Institute for Fermentation (IFO) as the Accession Number IFO 16271 on March 18, 1999.

Transformant *Escherichia coli* XL1 Blue/pZ1-puro5 containing the base sequence shown by SEQ ID NO:17, which was obtained in EXAMPLE 10 later described was on deposit with the Ministry of International Trade and Industry, Agency of Industrial Science and Technology, National Institute of Bioscience and Human Technology (NIBH) as the Accession Number FERM BP-6859 on August 23, 1999 and with Institute for Fermentation (IFO) as the Accession Number IFO 16272 on March 18, 1999.

Transformant *Escherichia coli* XL1 Blue/pZ1-puro9 containing the base sequence shown by SEQ ID NO:16, which was obtained in EXAMPLE 10 later described was on deposit with the Ministry of International Trade and Industry, Agency of Industrial Science and Technology, National Institute of Bioscience and Human Technology (NIBH) as the Accession Number FERM BP-6860 on August 23, 1999 and with Institute for Fermentation (IFO) as the Accession Number IFO 16273 on March 18, 1999.

Transformant *Escherichia coli* TOP10/pCR-buro containing the base sequence shown by SEQ ID NO:36, which was obtained in EXAMPLE 36 later described was on deposit with the Ministry of International Trade and Industry, Agency of Industrial Science and Technology, National Institute of Bioscience and Human Technology (NIBH) as the Accession Number FERM BP-6932 on November 8, 1999 and with Institute for Fermentation (IFO) as the Accession Number IFO 16332 on October 27, 1999.

EXAMPLES

The present invention is described below in more detail with reference to EXAMPLES, but not intended to limit the scope of the present invention thereto.

EXAMPLE 1 Amplification of rat SENR (=GPR14) receptor cDNA by PCR using rat brain-derived cDNA

By using rat brain-derived poly(A)$^+$RNA (Clonetech Laboratories, Inc.) as a template, reverse transcription was carried out using a random primer. For the reverse transcription, reagents available from Takara RNA PCR ver. 2 kit were used. Then using the reverse transcription product as a template, amplification by PCR was performed, using the synthetic DNA primers shown by SEQ ID NO:1 and SEQ ID NO:2. The synthetic DNA primers were constructed to amplify genes at the region to be translated into receptor proteins. In this case, recognition sites of the respective restriction enzymes were added at the 5' and 3' sides so that the base sequence recognized by restriction enzyme Sal I will be added at the 5' side of the gene and the base sequence recognized by restriction enzyme Spe I at the 3' side. The reaction solution was composed of 5 ml of the cDNA template, 1 μM each of the synthetic DNA primers, 0.2 mM dNTPs, 1 mM $MgCl_2$, 1 μl of KOD (King of DNA) DNA polymerase and a buffer attached to the enzyme, which were mixed together to make the total volume 50 μl. Using Thermal Cycler (Perkin-Elmer Co.) for amplification, after heating at 94°C for 60 seconds, a cycle was set to include 94°C for 30 seconds, 59°C for 30 seconds and 74°C for 60 seconds. Totally this cycle was repeated 35 times. Amplification of DNAs was confirmed by 0.8% agarose gel electrophoresis followed by ethidium bromide staining.

EXAMPLE 2 Subcloning of the PCR products into plasmid vectors and confirmation of the inserted cDNA sequence by decoding a base sequence of the inserted cDNA region

The PCR products obtained by PCR in EXAMPLE 1 were separated by using a 0.8% low-melting temperature agarose gel. The band parts were excised from the gel with a razor blade and then homogenized. The homogenate was extracted with phenol and then with phenol/chloroform and precipitated in ethanol to recover DNAs. According to the protocol attached to PCR-Script™ Amp SK(+) Cloning Kit (Stratagene Co.), the recovered DNAs were subcloned into the plasmid vector, pCR-Script Amp SK(+). The recombinant vectors were introduced into *Escherichia coli* JM109 competent cells (Takara Shuzo Co.) to produce transformants. Then, clones having a cDNA-inserted fragment were selected in an LB agar medium supplemented with ampicillin and X-gal. Only the clones exhibiting white color were picked up with a sterilized toothpick to obtain transformant *E. coli* JM109/SENR. The individual clones were cultured overnight in an LB medium containing ampicillin. Plasmid DNAs were prepared using QIA prep8 mini prep (Qiagen Co.). An aliquot of the DNAs thus prepared was processed for cleavage by restriction enzymes Sal I and Spe I to confirm the size of the receptor cDNA fragment inserted. The reaction for base sequencing was carried out by using a DyeDeoxy Terminator Cycle Sequence Kit (Perkin-Elmer Co.), followed by decoding with a fluorescent automatic sequencer. It was confirmed by sequence analysis that the sequences of 3 clones obtained entirely coincided with the gene sequence in the DNA sequence (Tal, M. et al., Biochem. Biophys. Res. Commun., Vol. 209, pp. 752-759 (1995)) of the full sequence-reported SENR (sensory epithelial neuropeptide-like receptor), wherein the Sal I recognition site was added at the 5' side and the Spe I recognition site was added at the 3' side (FIG. 1 and SEQ ID NO:3). According to the reported sequence of GPR14 gene (Marchese, A. et al., Genomics, vol. 29, pp. 335-344 (1995)), when the first base is A from the initiation codon ATG, the 945th should be G but was C in the SENR sequence and in the sequence determined above.

EXAMPLE 3 Preparation of SENR-expression CHO cells

Using Plasmid Midi Kit (Qiagen Co.), plasmid was prepared from clones of *E. coli* transformed by the plasmid encoding the rat brain-derived SENR full-length amino acid sequence, which sequence was confirmed in EXAMPLE 2, with the Sal I recognition sequence added at the 5' side and with the Spe I recognition sequence added at the 3' side. The plasmid was cleaved with restriction enzymes Sal I and Spe I to excise the insert part out. After electrophoresis, the insert DNA was excised from the agarose gel with a razor blade and then homogenized. The homogenate was extracted with phenol and then with phenol/chloroform, followed by precipitation in ethanol. Thus, the insert DNA was recovered. The insert DNA was added to Sal I- and Spe I-cleaved vector plasmid pAKK0-111H for animal cell expression (the same as the vector plasmid pAKK01.11G described in Hinuma, S. et al., Biochim. Biophys. Acta, Vol. 1219, pp. 251-259 (1994)) followed by ligation using T4 ligase (Takara Shuzo Co.). Thus, plasmid pAKK0-SENR for protein expression was constructed.

After *E. coli* DH5 (Toyobo Co.) transformed by pAKK0-SENR was cultured, plasmid DNA of pAKK0-SENR was prepared using Plasmid Midi Kit (Qiagen Co.). According to the protocol attached to CellPhect Transfection Kit (Amersham Pharmacia Biotech Co.), the plasmid DNA was introduced into CHO $dhfr^-$ cells. DNA, 10 μg, was co-precipitated with calcium phosphate in suspension. The resulting suspension was added to a 10 cm Petri dish in which $5 \times 10^5$ or $1 \times 10^6$ CHO $dhfr^-$ cells had been seeded before 24 hours. The cells were cultured in MEMα containing 10% fetal calf serum for one day. After passage, the cells were cultured in nucleic acid-free selection medium MEMα containing 10% dialyzed fetal calf serum and 68 clones of the transformant SENR-expression CHO cells, growing in the selection medium, were selected.

EXAMPLE 4 Selection of the CHO/SENR cell line with high expression of the full-length SENR receptor protein mRNA

The amounts of the expressed full-length SENR receptor protein mRNAs of 68 clones from the CHO/SENR strain established in EXAMPLE 3 were measured as follows using Cytostar T Plate (Amersham Pharmacia Biotech Co.), in accordance with the protocol attached thereto. Each clone of the CHO/SENR strain was inoculated on Cytostar T Plate in 2.5 x $10^4$ cells/ well. After culturing for 24 hours, the cells were fixed with 10% formalin. After 0.25% Triton X-100 was added to each well to increase cell permeability, $^{35}$S-labeled riboprobe of SEQ ID NO:4 was added to the cells for hybridization. By adding 20 mg/ml RNaseA to each well, free riboprobe was digested. After the plate was thoroughly washed, radioactivity of the riboprobe hybridized was measured with Topcounter. The strain with a high radioactivity provides a high expression amount of mRNA. Two clones (#36 and #61) which showed a high expression amount of mRNA were used for the following experiment, especially clone #61 as a main clone.

EXAMPLE 5 Detection of the activity contained in porcine spinal cord extract that promotes the release of arachidonic acid metabolites specifically from the CHO/SENR cell line

Fractions of the porcine spinal cord extract by high performance liquid chromatography (HPLC) were prepared by the method described below. Swine spinal cord, 350 g (corresponding to 10 pigs), which had been purchased from Tokyo Shibaura Zoki Co. and kept under ice cooling after the spinal cord was withdrawn from swine on the day of their sacrifice, was homogenized, immediately put into 1.4 liter of boiling distilled water and boiled for 10 minutes. Immediately after the boiling, the homogenate was ice-cooled and 84 ml of acetic acid was added to the homogenate to make the final concentration 1.0 M. Using a polytron (20,000 rpm, 6 mins.), the mixture was homogenized. The homogenate was centrifuged (8,000 rpm, 30 mins.) and the supernatant was taken out. After 1.4 liter of 1.0 M acetic acid was added to the precipitate, the mixture was again homogenized by a polytron. The homogenate was stirred overnight and then centrifuged (8,000 rpm, 30 mins.) to obtain the supernatant. After 2-fold volume of chilled acetone was slowly added dropwise to the supernatant at 4°C, the supernatant obtained by the first centrifugation was stirred overnight and, the supernatant obtained by the second centrifugation was stirred for 4 hours. The acetone-added extract was centrifuged (8,000 rpm, 30 mins.) to remove the precipitate and acetone was evaporated off in vacuum from the supernatant, using an evaporator. An equal volume of diethyl ether was added to the acetone-removed extract, the ethereal layer containing lipids was separated using a separating funnel to recover the aqueous layer. After the lipids were removed with ether, the extract was concentrated in vacuum using an evaporator to completely remove the ether. The concentrate was filtrated through a glass fiber filter paper (Advantech, DP70 (90 mmϕ)) and the filtrate was charged in a glass-made column (20ϕ x 240 mm) packed with C18 column (YMC, YMCgel ODS-AM 120-S50). After washing with 300 ml of 1.0 M acetic acid, the column was eluted with 300 ml of 60% acetonitrile containing 0.1% trifluoroacetic acid. The eluate was concentrated in vacuum, the solvent was distilled off and then the concentrate was lyophilized. About 0.2 g of the lyophilized product was dissolved in 14 ml of 10% acetonitrile containing 0.1% trifluoroacetic acid. An aliquot of 7 ml each was subjected to HPLC on 10% to 60% acetonitrile containing 0.1% trifluoroacetic acid by density gradient elution using C18 column (Toso, TSKgel ODS-80™ (21.5ϕ x 300 mm)). HPLC was performed twice. The eluate was fractionated into 60 fractions and the eluates in two runs were collected. Each fraction was concentrated and evaporated to dryness in vacuum. The residue was dissolved in 0.35 ml of dimethylsulfoxide (DMSO).

CHO/SENR cells and mock CHO cells were inoculated on a 24-well plate in 5 x $10^4$ cells/well. After incubation for 24 hours, [$^3$H] arachidonic acid was added to the system in 0.25 μCi/well. Sixteen hours after the addition of [$^3$H] arachidonic acid, the cells were washed with Hanks' balanced salt solution (HBSS) supplemented with 0.05% bovine serum albumin (BSA) and 500 μl of 0.05% BSA-containing HBSS, to which 2 μl (corresponding to 2 g of the spinal cord) of the aforesaid DMSO solution of the HPLC fraction was added, was added to each well. After incubation at 37°C for 30 minutes, 350 μl out of 500 μl of the reaction solution was charged in a scintillator to measure the amount of [3H] arachidonic acid metabolites released during the reaction, using a scintillation counter. The results indicate that the arachidonic acid metabolite release activity specific to the CHO/SENR cells was noted in fraction #33 (FIG. 2). In FIG. 2, the arachidonic acid metabolite release-promoting activity was expressed by % in terms of the amount of the [$^3$H] arachidonic acid metabolites released upon addition of the HPLC fraction (2 μl), when the amount of the [$^3$H] arachidonic acid metabolites released when 2 μl of DMSO alone was added was made 100%. The arachidonic acid metabolite release-promoting activity specific to the CHO/SENR cell line was noted with the fraction #33. Since the arachidonic acid metabolite release-promoting activity noted from fractions #26 through #29 was observed in the mock CHO cells as well, the arachidonic acid metabolite release-promoting activity was not specific to the CHO/SENR cell line. The activity was expressed in terms of percentage to the amount of arachidonic acid metabolites released in the control group where DMSO alone was added.

EXAMPLE 6 Inactivation of the active substances showing the arachidonic acid metabolite release activity specific to the SENR-expression CHO cells in the porcine spinal cord extract

The HPLC fraction #33 which showed the arachidonic acid metabolite release activity to the CHO/SENR cells in EXAMPLE 5 was treated with proteolytic enzyme, pronase (Sigma, protease Type XIV (P5147)) to examine if the active substance is proteinaceous.

The HPLC fraction (#33), 4 μl, from the spinal cord extract described above was added to 200 μl of 0.2 M ammonium acetate and 3 μl of pronase was further added thereto. After incubation at 37°C for 2 hours, the culture was boiled in boiling water for 10 minutes to inactivate the pronase. To the reaction solution was added 2 ml of distilled water containing 0.05 mg of BSA and 0.05 mg of CHAPS, followed by lyophilization. In order to examine if pronase itself, or heating and lyophilization have an affect, pronase alone, the HPLC fraction alone, and a mixture of the HPLC fraction with pronase alone after its heating were treated in a similar manner and then lyophilized. Each sample fluid lyophilized was dissolved in 500 μl of 0.05% BSA-containing HBSS. The solution was added to the CHO/SENR cells by the procedures described in EXAMPLE 5 followed by assay for the arachidonic acid metabolite release activity. The results are shown in FIG. 3. The activity was expressed in terms of percentage to the amount of arachidonic acid metabolites released in a well charged with 500 μl of 0.05% BSA-containing HBSS. Since the active substances showing the arachidonic acid metabolite release activity on the CHO/SENR cells in the porcine spinal cord extract were completely inactivated, the substances were suspected to be proteins or peptides.

EXAMPLE 7 Purification of the active substances showing the arachidonic acid metabolite release activity specific to the SENR-expression CHO cells from the swine spinal cord

A representative example of purifying from swine spinal cord the active substances showing the arachidonic acid metabolite release activity specific to the CHO/SENR cells is specifically described below. Swine spinal cord, 1.0 kg (corresponding to 50 pigs), which had been purchased from Tokyo Shibaura Zoki Co. and kept under ice cooling after the spinal cord was withdrawn from swine on the day of their sacrifice, was homogenized in 10 1 of 70% acetone containing 40 mM hydrochloric acid and 1.0 M acetic acid using Polytron (20,000 rpm, 10 min). The homogenate was centrifuged (8,000 rpm, 30 mins.) and the supernatant was taken out. Again 10 1 of 70% acetone containing 40 mM hydrochloric acid and 1.0 M acetic acid was added to the precipitate and homogenized by a polytron. After stirring overnight, the homogenate was centrifuged (8,000 rpm, 30 mins.) to obtain the supernatants. The supernatants were collected and acetone was distilled off in vacuum using an evaporator. An equal volume of diethyl ether was added to the acetone-removed extract and the ethereal layer containing lipids was separated using a separating funnel to recover the aqueous layer. After the lipids were removed by ether, the extract was concentrated in vacuum using an evaporator to completely evaporate the ether off. The concentrate was filtrated through a glass fiber-made filter paper (Advantech, DP70 (90 mmϕ)) and a half of the filtrate was charged in a glass-made column (30ϕ x 240 mm) packed with C18 (YMC, YMCgel ODS-AM 120-S50). After washing with 400 ml of 1.0 M acetic acid, the column was eluted with 500 ml of 60% acetonitrile containing 0.1% trifluoroacetic acid. The eluate was concentrated in vacuum, the solvent was distilled off. The concentrate was then lyophilized. The remaining half of the filtrate was similarly treated and lyophilized. About 1.9 g in total of the lyophilized product was dissolved in 60 ml of 10% acetonitrile containing 0.1% trifluoroacetic acid. An aliquot of 10 ml each was subjected to HPLC on 10% to 60% acetonitrile containing 0.1% trifluoroacetic acid by density gradient elution using C18 column (Toso, TSKgel ODS-80$T_s$ (21.5ϕ x 300 mm)). HPLC was performed six times. The eluate was fractionated into 60 fractions and the eluates in 6 runs were collected. Each fraction was added to the SENR-expression CHO cells and assayed for the arachidonic acid metabolite release activity by the procedures described in EXAMPLE 5. The activity was observed in the fractions #31 and #32.

These fractions #31 (①) and #32 (②) were purified separately by the same procedures shown below. After each of the active fractions was concentrated in vacuum to remove the solvent, the concentrate was lyophilized. The lyophilized product was dissolved in 10 ml of 10 mM ammonium formate containing 10% acetonitrile. The solution was passed through a cationic exchange column (Toso, TSKgel SP-5PW (20 mmφ x 150 mm)). Then the column was eluted with 10 mM to 300 mM ammonium formate containing 10% acetonitrile by means of density gradient. In both ①and ②, their activity was recovered around 140 mM ammonium formate. The active fractions were lyophilized followed by dissolving in 1.0 ml of 10% acetonitrile containing 0.1% trifluoroacetic acid. After 0.5 ml each of the solution was passed through a diphenyl column (Separation Group, Vydac 219-TP54), elution was performed by density gradient of 26% to 31% acetonitrile containing 0.1% trifluoroacetic acid. HPLC was carried out twice and the eluates in 2 runs were collected and fractionated. The activity appeared around 27.1% acetonitrile for ①and around 27.6% acetonitrile for ②. Each of the active fractions was lyophilized and dissolved in 0.1 ml of DMSO. Further 0.4 ml of 10% acetonitrile containing 0.1% trifluoroacetic acid was added to the solution. After passing the resulting solution through a CN column (Nomura Kagaku, Develosil CN-UG-5), elution was performed by density gradient with 28.5% to 33.5% acetonitrile containing 0.1% trifluoroacetic acid. The eluate was manually fractionated for every peak. The activity appeared around 29.7% acetonitrile for ①and around 29.9% for ②(FIGS. 4 and 5). The eluates containing the active peaks were diluted to about 2-fold with distilled water. After passing the dilution through an ODS column (Wako Junyaku, Wakosil-II 3C18HG), elution was performed by density gradient with 30% to 35% acetonitrile containing 0.1% heptafluorobutyric acid. The activity appeared around 32.2% acetonitrile for ①and around 32.5% for ②(FIGS. 6 and 7).

EXAMPLE 8 Determination of amino acid sequences for the active substances showing the arachidonic acid metabolite release activity specific to the SENR-expression CHO cells purified from the swine spinal cord

Amino acid sequencing of the active substances showing the arachidonic acid metabolite release activity specific to the CHO/SENR cells purified in EXAMPLE 7 was performed. Since it was speculated that the active substances would be proteins or peptides as shown in EXAMPLE 6, amino-terminal amino acid sequencing was conducted by use of Procise 494 Protein Sequencer available from Perkin-Elmer, using the eluates containing the active peaks. As a result, the sequences shown by SEQ ID NO:5 and SEQ ID NO:6 were obtained. No amino acid was detected on the 6th and 11th residues. Thus, the active substance ②was reduced and pyridylethylated using tributylphosphine and 4-vinylpyridine, which was then subjected to sequencing. No amino acid was yet detected on the 11th residue but pyridylethyl cysteine was detected on the 6th residue. From the foregoing it was surmised that the 6th and 11th residues of the active substance ②would be cysteine and these two Cys residues would form an intramolecular disulfide bond. The active substance ①having a similar structure has cysteine on the 6th and 11th residues, speculating that these two Cys residues would likewise form an intramolecular disulfide bond. Based on the foregoing, SEQ ID NO:7 and SEQ ID NO:8 were deduced to represent the amino acid sequences of the two active substances.

EXAMPLE 9 Acquisition of the partial sequence of swine SENR ligand precursor protein by PCR

Using swine genome DNA (Clonetech Laboratories, Inc.) as a template, PCR was carried out using primers shown by SEQ ID NO:10 and SEQ ID NO:11, which are partial sequences of the base sequence (accession No. AA535545) encoding a part of the precursor protein of human urotensin II (Coulouarn, Y. et al., Proc. Natl. Acad. Sci. USA, vol. 95, pp. 15803-15808 (1998)) registered in the GenBank database. The reaction solution was composed of 1 μM each of the synthetic primers, 500 ng of the template DNA, 0.2 mM dNTPs, 1.25 unit of ExTaq DNA polymerase (Takara Shuzo Co.) and a buffer attached to the enzyme, which were mixed together to make the whole volume of the reaction solution 20 μl. Using Thermal Cycler (Perkin-Elmer Co.) for amplification, after heating at 94°C for 4 minutes, a cycle was set to include 94°C for 30 seconds, 60°C for 30 seconds and 72°C for 15 seconds, with 2 repetitions; a cycle of 94°C for 30 seconds, 55°C for 30 seconds and 72°C for 15 seconds, with 4 repetitions; a cycle of 94°C for 30 seconds, 52.5°C for 30 seconds and 72°C for 15 seconds, with 6 repetitions; and a cycle of 94°C for 30 seconds, 50°C for 30 seconds and 72°C for 15 seconds, with 30 repetitions. The amplified product was confirmed by 1.5% agarose electrophoresis and ethidium bromide staining. Using TOPO TA cloning kit (Invitrogen Inc.), 2 μl of the reaction solution thus obtained was subcloned to plasmid vector pcr II and then introduced into *Escherichia coli* DH5α. From the resulting transformant, the plasmid DNA was purified using QIA prep8 mini prep (Qiagen Co.). The sequencing reaction for base sequence determination was carried out using DyeDeoxy Terminator Cycle Sequence Kit (Perkin-Elmer Co.). The DNAs were decoded by an automated fluorescent sequencer. As a result, the base sequence (SEQ ID NO:12), which are speculated to be a part of swine SENR ligand precursor cDNA containing the partial sequence of the SENR ligand polypeptide purified from the swine spinal cord, was obtained. Using this sequence as a probe, a cDNA library of the swine spinal cord described in EXAMPLE 10 was screened.

EXAMPLE 10 Determination of cDNA sequence containing the entire coding region of the SENR ligand precursor protein obtained from swine spinal cord cDNA library

By use of Isogen (Nippon Gene Co.), total RNAs were prepared from swine spinal cord. Then, poly(A) $^+$RNA fraction was prepared using Oligotex$(dT)_{30}$ (Takara Shuzo Co.). From 2 μg of this poly(A) $^+$RNA, cDNA was introduced into λZiplox Not I/Sal I Arm, using Superscript Lamda System for cDNA Synthesis and λ cloning kit (Gibco BRL), according to the manual attached. By packaging using Gigapack III Gold (Stratagene Co.), swine spinal cord cDNA library was prepared. From the library 1.6 x $10^6$ pfu (plaque forming unit) was taken and mixed with *Escherichia coli* Y1090ZL. After incubation at 37°C for 15 minutes, 0.7% agarose (FMC Co.) LB was added to the culture, which was inoculated on 121 plates of 1.5% agar LB. A nitrocellulose filter was put on the plates to transfer plaques. The filter was treated with an alkali for denaturation, then neutralized, dried, exposed to UV rays at 254 nm and heated at 80°C for 30 minutes thereby to fix the DNA. The filter was incubated at 45°C for 4 hours in 0.5 M phosphate containing 1 mM EDTA, 7% SDS and 1% BSA. The filter was then incubated for 16 hours with the probes described below for hybridization. The probes were selected from the sequence obtained in EXAMPLE 9, in which the forward strand was SEQ ID NO:13 and the reverse strand complementary in part to the former sequence was SEQ ID NO:14. The synthesis of these probes were consigned (to Nippon Bio Service Co.). After denaturation of the probes at 70°C, the probes were slowly cooled to hybridize to one another, which was then radioactively labeled using Klenow enzyme in the presence of [$^{32}$P]dCTP (du Pont). The labeled probe was further purified through a Nick column (Amersham Pharmacia Biotech Co.) and used for hybridization in a concentration of 1,000,000 cpm/ml. The labeled probe was washed at room temperature 4 times with a 0.2 x SSC (diluted from 20 x SSC manufactured by Nippon Gene Co.) solution containing 0.1% SDS, and then twice at 65°C. Subsequently autoradiography was performed at -80°C to detect plaques hybridized. By this screening, hybridization signal was noted in 9 independent plaques. The desired plasmid containing the swine SENR ligand precursor cDNA was excised from these positive plaques by the *in vivo* excision technique, according to the manual attached to Superscript Lamda System for cDNA Synthesis and λ cloning kit (Gibco BRL) and transformed *Escherichia coli* XLIBlue. Using QIA prep8 mini prep (Qiagen Co.), the plasmid DNA was purified from the *Escherichia coli*. The sequencing reaction for base sequence determination was carried out using DyeDeoxy Terminator Cycle Sequence Kit (Perkin-Elmer Co.). The DNAs were decoded by an automated fluorescent sequencer. As a result, three base sequences (SEQ ID NO:15, SEQ ID NO:16 and SEQ ID NO:17) encoding the entire sequence of the swine SENR ligand precursor protein were obtained. When A from the initiation codon ATG is made the first base, the 129th base is T in SEQ ID NO:15 and C in SEQ ID NO:16, but they were both translated into the same amino acid Asp (GAT, GAC). SEQ ID NO:17 was found to be a splicing variant, in which C at the 101st to G at the 208th of SEQ ID NO:15 are deleted when the first base is A from the initiation codon ATG. The corresponding amino acid sequences are: SEQ ID NO:18 for SEQ ID NO:15 and SEQ ID NO:16 and, SEQ ID NO:19 for SEQ ID NO:17. All of these precursor proteins were the precursors of swine SENR ligand ②(SEQ ID NO:8). FIG. 8 shows the DNA sequence (SEQ ID NO:15) of swine SENR ligand precursor and the corresponding amino acid sequence (SEQ ID NO:18).

EXAMPLE 11 Determination of partial sequence of swine SENR ligand precursor protein by PCR

Using bovine genomic DNA (Clonetech Laboratories, Inc.) as a template, PCR was carried out using the primers shown by SEQ ID NO:10 and SEQ ID NO:11 employed in EXAMPLE 9. The reaction solution was composed of 0.5 μM each of the synthetic primers, 500 ng of the template DNA, 0.2 mM dNTPs, 2.5 mM $MgCl_2$, 0.2 μl of AmpliTaq Gold DNA polymerase (Perkin-Elmer Co.) and a buffer attached to the enzyme, which were mixed together to make the whole volume of the reaction solution 20 μl. Using Thermal Cycler (Perkin-Elmer Co.) for amplification, after heating at 95°C for 9 minutes, a cycle was set to include 94°C for 15 seconds, 60°C for 20 seconds and 72°C for 20 seconds, with 2 repetitions; a cycle of 94°C for 15 seconds, 55°C for 20 seconds and 72°C for 20 seconds, with 4 repetitions; a cycle of 94°C for 20 seconds, 52.5°C for 20 seconds and 72°C for 20 seconds, with 6 repetitions; a cycle of 94°C for 20 seconds, 50°C for 20 seconds and 72°C for 20 seconds, with 8 repetitions; and a cycle of 94°C for 30 seconds, 48°C for 20 seconds and 72°C for 20 seconds, with 30 repetitions, followed by maintaining at 72°C for 5 minutes. The amplified product was confirmed by 1.5% agarose electrophoresis and ethidium bromide staining. Using TOPO TA cloning kit (Invitrogen Inc.), 2 μl of the reaction solution thus obtained was subcloned to plasmid vector pcr 2.1 and then introduced into *Escherichia coli* TOP10. From the resulting transformant, the plasmid DNA was purified using QIA prep8 mini prep (Qiagen Co.). The reaction for base sequencing was carried out using DyeDeoxy Terminator Cycle Sequence Kit (Perkin-Elmer Co.). The DNAs were decoded using an automated fluorescent sequencer. As a result, SEQ ID NO:20, which are considered to be a part of the swine SENR ligand precursor because of similarity to the sequence of swine SENR ligand precursor, was obtained as the PCR product. The primer shown by SEQ ID NO:11 is a base sequence encoding a part of the ligand peptide and by comparing the amino acid sequence of swine SENR ligand, it was determined to be SEQ ID NO:21 as a bovine SENR ligand.

EXAMPLE 12 Preparation of swine SENR ligand ①: Gly-Pro-Thr-Ser-Glu-Cys-Phe-Trp-Lys-Tyr-Cys-Val (SEQ ID NO:7)

Commercially available Boc-Val-$OCH_2$-PAM resin (0.77 mmole/g resin) was charged in a reaction tank of peptide synthesizer ABI 430A. Thereafter, Boc-Cys(MeBzl), Boc-Tyr(Br-Z), Boc-Lys(Cl-Z), Boc-Trp(CHO), Boc-Phe, Boc-Cys(MeBzl), Boc-Glu(OcHex), Boc-Ser(Bzl), Boc-Thr(Bzl), Boc-Pro and Boc-Gly were introduced into the resin in this order according to the Boc-strategy (NMP-HOBt) peptide synthesis to give the desired protected peptide resin. The resulting resin, 0.59 g, was stirred at 0°C for 60 minutes in 10 ml of anhydrous hydrogen fluoride containing 2.22 g of p-cresol and 1.2 ml of 1,4-butanediol. Thereafter the hydrogen fluoride was distilled off in vacuum. Diethyl ether was added to the residue and the precipitate was filtrated. Aqueous 50% acetic acid solution was added to the precipitate for extraction and insoluble matters were removed. After the extract was sufficiently concentrated, the concentrate was applied to Sephadex G-25 (trade name) column (2.0 x 80 cm) filled with 50% acetic acid aqueous solution followed by development with the same solvent. The main fractions were collected and applied to reverse phase chromatography column (2.6 x 60 cm) packed with LiChroprep (trade name) RP-18 followed by washing with 200 ml of water containing 0.1% TFA. Then linear density gradient elution was performed with 300 ml of 0.1% TFA and 300 ml of 40% acetonitrile containing 0.1% TFA. The main fractions were collected and concentrated. The concentrate was dissolved in approximately 4 ml of acetic acid. After diluting the solution with distilled water to a volume of 240 ml, pH was adjusted to 7.5 with ammonia water. The dilution was stirred while mildly blowing air therein. The reaction was traced by HPLC. After it was confirmed that the peaks of the SH form peptide were all converted into the SS form, acetic acid was added to the reaction system to adjust pH of the solution to 3. Then the solution was adsorbed onto the LiChroprep (trade name) RP-18 column described above. After washing the column with 200 ml of water containing 0.1% TFA, linear density gradient elution was carried out using 300 ml of water containing 0.1% TFA and 300 ml of 50% acetonitrile water containing 0.1% TFA. The main fractions were collected and lyophilized to give 17 mg of white powders.
Mass spectrum $(M+H)^+$ 1417.4 (calcd. 1417.6)
Elution time on HPLC: 19.0 mins.
Column conditions:

Column: Wakosil 5C18T, 4.6 x 100 mm
Eluant: linear density gradient elution with eluants A/B = 95/5 - 45/55, using aqueous 0.1% TFA as eluant A and acetonitrile containing 0.1% TFA (25 mins.)
Flow rate: 1.0 ml/min.

EXAMPLE 13 Preparation of swine SENR ligand ②: Gly-Pro-Pro-Ser-Glu-Cys-Phe-Trp-Lys-Tyr-Cys-Val (SEQ ID NO: 8)

Except for introducing Pro in lieu of Thr in EXAMPLE 12, excision of the resin, purification of SH peptide, oxidation and purification of SS peptide were carried out in a similar manner to obtain 15 mg of white powders.
Mass spectrum $(M+H)^+$ 1413.4 (calcd. 1413.4)
Elution time on HPLC: 19.3 mins.
Column conditions:

Column: Wakosil 5C18T, 4.6 x 100 mm
Eluant: linear density gradient elution with eluants A/B = 95/5 - 45/55, using aqueous 0.1% TFA as eluant A and acetonitrile containing 0.1% TFA (25 mins.)
Flow rate: 1.0 ml/min.

EXAMPLE 14 Preparation of bovine SENR ligand: Gly-Pro-Pro-Ser-Glu-Cys-Phe-Trp-Lys-Tyr-Cys-Val (SEQ ID NO:21)

Except for introducing Ser in lieu of Thr in EXAMPLE 12, excision of the resin, purification of SH peptide, oxidation and purification of SS peptide were carried out in a similar manner.
Mass spectrum $(M+H)^+$ 1403.5 (calcd. 1403.6)
Elution time on HPLC: 18.8 mins.
Column conditions:

Column: Wakosil 5C18T, 4.6 x 100 mm
Eluant: linear density gradient elution with eluants A/B = 95/5 - 45/55, using aqueous 0.1% TFA as eluant A and acetonitrile containing 0.1% TFA (25 mins.)
Flow rate: 1.0 ml/min.

EXAMPLE 15 Preparation of human SENR ligand: Glu-Thr-Pro-Asp-Cys-Phe-Trp-Lys-Tyr-Cys-Val (human urotensin II, SEQ ID NO:22)

Human SENR ligand (SEQ ID NO:22) is the same peptide as reported to be human urotensin II (Coulouarn, Y. et al., Proc. Natl. Acad. Sci. USA, vol. 95, pp. 15803-15808 (1998)).
Using 0.5 mmole of commercially available Boc-Val-$OCH_2$-PAM resin (0.77 mmole/g resin), Boc-Cys(MeBzl), Boc-Tyr(Br-Z), Boc-Lys(Cl-Z), Boc-Trp(CHO), Boc-Phe, Boc-Cys(MeBzl), Boc-Asp(OcHex), Boc-Pro, Boc-Thr(Bzl) and Boc-Glu(OcHex) were introduced into the resin in this order, as in EXAMPLE 12. This resin was treated, the peptide was excised, oxidized and purified, as in EXAMPLE 12.
Mass spectrum $(M+H)^+$ 1388.4 (calcd. 1388.6)
Elution time on HPLC: 19.0 mins.
Column conditions:

Column: Wakosil 5C18T, 4.6 x 100 mm
Eluant: linear density gradient elution with eluants A/B = 95/5 - 45/55, using aqueous 0.1% TFA as eluant A and acetonitrile containing 0.1% TFA (25 mins.)
Flow rate: 1.0 ml/min.

EXAMPLE 16 Arachidonic acid metabolite release-promoting activity of the synthetic swine SENR ligand polypeptides to the CHO/SENR cell line

The arachidonic acid metabolite release activity of the SENR ligand polypeptides ①and ②(SEQ ID NO:7 and SEQ ID NO:8) in various concentrations, which were synthesized in EXAMPLES 12 and 13, to the SENR-expression CHO cells was measured by the following method. The CHO/SENR cells were inoculated on a 24-well plate in 5 x $10^4$ cells/well. After incubation for 24 hours, [$^3$H] arachidonic acid was added to the system in 0.25 μCi/well. Sixteen hours after the addition of arachidonic acid, the cells were washed with Hanks' balanced salt solution (HBSS) supplemented with 0.05% bovine serum albumin (BSA) and 500 μl of 0.05% BSA-containing HBSS, to which the synthetic SENR ligand polypeptide was added in various concentrations, was added to each well. After incubation at 37°C for 30 minutes, 350 μl out of 500 μl of the reaction solution was charged in a scintillator to measure the amount of [$^3$H] arachidonic acid metabolites released during the reaction with a scintillation counter. As a result it was confirmed that the arachidonic acid metabolites were released dependently on the peptide concentration, in both of the SENR ligand polypeptides ① and ②(SEQ ID NO:7 and SEQ ID NO:8) (FIG. 9). The activity was expressed in terms of percentage based on the amount of the arachidonic acid metabolites released in control group in which the buffer alone was added. A similar activity was confirmed even when using bovine SENR ligand (SEQ ID NO:21) or human SENR ligand (human urotensin II) (SEQ ID NO:22).

EXAMPLE 17 Activity of synthetic swine SENR ligand polypeptides on blood pressure of rats under anesthesia

The activity of the synthetic SENR ligand polypeptides ①and ②(SEQ ID NO:7 and SEQ ID NO:8) in various concentrations, which were synthesized in EXAMPLES 12 and 13, on blood pressure of rats under anesthesia was measured by the following method. Male Wistar rats of 8 to 9 weeks old (purchased from Nippon Crea Co.) were anesthetized with Nembutal injection (Dai-Nippon Pharmaceutical Co., 50 mg/ml sodium pentobarbital, 50 mg/kg i.p.). A catheter (SP-55) for blood pressure measurement connected with a transducer was inserted into the left carotid artery and a catheter (SP-35) for intravenous injection into the left femoral vein. The synthetic ligand was dissolved in physiological saline containing 0.05% BSA, which was cannulated through the left femoral vein in a dose of 1, 10 or 100 nmol/kg. The blood pressure was continuously recorded by means of a polygraph (manufactured by NEC Sanei Co.). The blood pressure in rats decreased dose-dependently and the SENR ligand polypeptides showed a hypotensive action in rats. Table 1 shows the hypotensive action on rat blood pressure (under anesthesia) when swine SENR ligand was administered in a dose of 10 nmols/kg. The hypotensive action was shown by the average blood pressure reduced by administration of SENR ligand to the average blood pressure prior to administration. A similar activity was confirmed also when using bovine SENR ligand (SEQ ID NO:21) or human SENR ligand (human urotensin II) (SEQ ID NO:22). The hypotensive action of these peptides are shown in Table 1.

Table 1

| Hypotensive action of synthetic swine SENR ligand polypeptide, synthetic bovine SENR ligand polypeptide and synthetic human SENR ligand polypeptide (urotensin II) on blood pressure of rats (under anesthesia) | | | |
|---|---|---|---|
| Dose (10 nmols/kg) | | Number of rat | Average blood pressure decreased (mmHg, average + standard error) |
| Swine SENR ligand ① | 10 | 8 | 34.3 ± 4.6 |
| Swine SENR ligand ② | 10 | 8 | 35.3 ± 3.1 |
| Bovine SENR ligand | 10 | 8 | 35.7 ± 7.0 |
| Human SENR ligand | 10 | 8 | 35.1 ± 5.7 |

EXAMPLE 18 Vasoconstrictive action of synthetic swine ligand polypeptide on rat thoracic aorta

The activity of the synthetic SENR ligand polypeptide ②(SEQ ID NO:8) in various concentrations, which were synthesized in EXAMPLE 13, on rat thoracic aorta was measured by the following method. Male Wistar rats of 9 to 12 weeks old (purchased from Nippon Charles River Co.) were anesthetized with Nembutal injection (Dai-Nippon Pharmaceutical Co., 50 mg/ml sodium pentobarbital, 50 mg/kg i.p.). The animal was bled to death by collecting whole blood from the abdominal aorta. The thoracic aorta was removed from the rat to prepare ring preparations of 5 mm width.

The ring preparations were suspended in organ baths filled with 3 ml of Krebs-Henseleit solution (118 mM NaCl, 4.7 mM KCl, 2.5 mM $CaCl_2$, 1.2 mM $KH_2PO_4$, 25 mM $NaHCO_3$, 1.2 mM $MgSO_4$, 10.0 mM glucose) kept at 37°C and gassed with a mixed gas (95%$O_2$ - 5%$CO_2$). The isometric and contractive tension of each preparation was detected by a micro-load transducer (UL-10GR, Minebea) and recorded by a polygraph (NEC Sanei Co.). The resting tension was set to approximately 0.5 g. The presence of endothelium was confirmed by observation that the constriction caused by administration of $10^{-6}$ M norepinephrine was relaxed by administration of $10^{-5}$ M acetylcholine. The swine SENR ligand polypeptide was cumulatively administered to the final concentration of $10^{-10}$ to $10^{-7}$ M. The rat thoracic aorta ring preparation was dose-dependently contracted by addition of the SENR ligand, as shown in FIG. 10. A similar activity was confirmed as well, when using swine SENR ligand ①(SEQ ID NO:7), bovine SENR ligand (SEQ ID NO: 21) or human SENR ligand (human urotensin II) (SEQ ID NO:22).

EXAMPLE 19 Amplification of human SENR (=GPR14) receptor cDNA by PCR using human skeletal muscle-derived cDNA

By using human skeletal muscle-derived cDNA (Clonetech Laboratories, Inc.) as a template, amplification by PCR was carried out using synthetic DNA primers shown by SEQ ID NO:23 and SEQ ID NO:24. The synthetic DNA primers were so constructed as to amplify genes at the region to be translated into receptor proteins, whereupon recognition sites of the respective restriction enzymes were added at the 5' and 3' sides so that the base sequence recognized by restriction enzyme Sal I was added at the 5' side of the gene and the base sequence recognized by restriction enzyme Spe I at the 3' side. The reaction solution was composed of 2.5 μl of the cDNA template, 0.2 μM each of the synthetic DNA primers, 0.2 mM dNTPs, 1 μl of Advantage2 polymerase mix (Clonetech Laboratories, Inc.) and a buffer attached to the enzyme, which were mixed together to make the total volume 50 μml. Using Thermal Cycler (Perkin-Elmer Co.) for amplification, after heating at 95°C for 60 seconds, a cycle was set to include 95°C for 30 seconds and 72°C for 3 minutes, with 5 repetitions; then a cycle of 95°C for 30 seconds and 70°C for 3 minutes, with 5 repetitions; a further cycle of 95°C for 30 seconds and 68°C for 3 minutes, with 20 repetitions; and finally, heating was conducted at 94°C for 3 minutes. The amplified products were confirmed by 0.8% agarose gel electrophoresis followed by ethidium bromide staining.

EXAMPLE 20 Subcloning of the PCR products into plasmid vectors and confirmation of amplified cDNA sequence by decoding base sequences of the inserted cDNA region

The PCR products obtained after PCR in EXAMPLE 19 were separated by using a 0.8% low-melting temperature agarose gel. After the band parts were excised from the gel with a razor blade, DNAs were recovered using GENECLEAN SPIN (Bio 101 Co.). Following the protocol of Eukaryotic TOPO™ TA Cloning Kit (Invitrogen Co.), the recovered DNAs were subcloned into the plasmid vector, pcDNA3.1/V5/His to construct plasmid pcDNA3.1-hSENR for protein expression. The plasmid was introduced into *Escherichia coli* DH5α competent cells (Toyobo Co.) to produce transformants. After that, clones carrying a cDNA-inserted fragment were selected in an LB agar medium containing ampicillin and picked up with a sterilized toothpick to obtain transformant E. *coli* DH5α/pcDNA3.1-hSENR. The individual clones were cultured overnight in an LB medium containing ampicillin. Plasmid DNAs were prepared using Qiawell 8 Ultra Plasmid Kit (Qiagen Co.). An aliquot of the DNAs thus prepared was processed for cleavage by restriction enzyme Sal I to confirm the size and direction of the receptor inserted. The reaction for base sequencing was carried out by using a DyeDeoxy Terminator Cycle Sequence Kit (Perkin-Elmer Co.), followed by decoding with a fluorescent automatic sequencer. It was confirmed by sequence analysis that the sequences of the clones obtained entirely coincided with the gene sequence of the reported sequence of human GPR14 (=SENR) gene (EP 0 859 952 A1) wherein the Sal I recognition site was added at the 5' side and the Spe I recognition site was added at the 3' side (SEQ ID NO: 25 and SEQ ID NO:26). The 1133rd base in the human SENR gene shown by SEQ ID NO:25 is reported to be C in the journal (EP 0 859 952 A1) but C was the base that was determined in this EXAMPLE. However, the two bases are translated into the same amino acid.

EXAMPLE 21 Preparation of human SENR-expression CHO cells

After transformant *E. coli* DH5α/pcDNA3.1-hSENR prepared in EXAMPLE 20 was cultivated, plasmid DNA of pcDNA3.1-hSENR was prepared using Plasmid Midi Kit (Qiagen Co.). Using CellPhect Transfection Kit (Amersham Pharmacia Biotech Co.), the plasmid DNA was introduced into CHO dhfr⁻ cells. Ten microgram of DNA was co-precipitated with calcium phosphate in suspension. The resulting suspension was added to a 10 cm Petri dish in which $5 \times 10^5$ or $1 \times 10^6$ CHO dhfr⁻ cells had been seeded before 24 hours. The cells were cultured for one day in MEMα containing 10% fetal calf serum. After passage, the cells were cultured in MEMα, as selection medium, containing 0.4 mg/ml of G418 (Gibco BRL) and 10% dialyzed fetal calf serum. Colonies of the transformant (CHO/hSENR) as human SENR-expression CHO cell, which grow in the selection medium, were selected.

EXAMPLE 22 Arachidonic acid metabolite release-promoting activity of the synthetic human SENR ligand polypeptides to the CHO/hSENR cell line

The arachidonic acid metabolite release activity of the human SENR ligand polypeptide (human urotensin II) (SEQ ID NO:22) in various concentrations, which was synthesized in EXAMPLES 15, to the human SENR-expression CHO cells was measured by the following method. CHO/hSENR cells were inoculated on a 24-well plate in 5 x $10^4$ cells/well. After incubation for 24 hours, [$^3$H] arachidonic acid was added to the system in 0.25 µCi/well. Sixteen hours after the addition of arachidonic acid, the cells were washed with Hanks' balanced salt solution (HBSS) supplemented with 0.05% bovine serum albumin (BSA) and 500 µl of 0.05% BSA-containing HBSS, to which the synthetic human SENR ligand polypeptide was added in various concentrations, was added to each well. After incubation at 37°C for 30 minutes, 350 µl out of 500 µl of the reaction solution was added to a scintillator and the amount of [$^3$H] arachidonic acid metabolites released during the reaction was measured with a scintillation counter. The result confirmed release of the arachidonic acid metabolites dependently on the peptide concentration in the human SENR ligand polypeptides (human urotensin II, SEQ ID NO:22)(FIG. 11). The activity was expressed by percentage based on the amount of the arachidonic acid metabolites released in control group in which the buffer alone was added. A similar activity was confirmed even when using swine SENR ligands (SEQ ID NO:7 and SEQ ID NO:8) and bovine SENR ligand (SEQ ID NO:21).

EXAMPLE 23 Assay for GTPγS binding activity to SENR-expression CHO cell membrane fraction induced by bovine SENR ligand

The [$^{35}$S] -guanosine 5'-(γ-thio)triphosphate binding promoting activity of bovine SENR ligand (SEQ ID NO: 21) on SENR-expression CHO cell membrane fraction was measured by the following method. First, preparation of membrane fraction is described. To 1 x $10^8$ of CHO/SENR cells was added 10 ml of a homogenate buffer (10 mM $NaHCO_3$, 5 mM EDTA, 0.5 mM PMSF, 1 µg/ml pepstatin, 4 µg/ml E64, 20 µg/ml leupeptin), which was homogenized by using Polytron (12,000 rpm, 1 min.). The cell homogenate was subjected to centrifugation (1,000 g, 15 mins.) to give the supernatant. Next, the supernatant was subjected to ultracentrifugation (Beckman type 30 rotor, 30,000 rpm, 1 hour). The resulting precipitate was used as a rat SENR-expression CHO cell membrane.

The GTPγS binding activity was measured as follows. The rat SENR-expression CHO cell membrane was diluted with a buffer for membrane dilution (50 mM Tris-hydrochloride buffer (pH 7.4), 5 mM $MgCl_2$, 150 mM NaCl, 1 µM GDP) to prepare a cell membrane fraction solution for assay having a protein concentration of 30 µg/ml. To 200 µl of the cell membrane fraction solution for assay were added 2 µl of 51.5 nM [$^{35}$S]-guanosine 5'-(γ-thio)triphosphate (NEN Co.) and 2 µl of bovine SENR ligand (SEQ ID NO:21) in various concentrations. The resulting solution mixture was kept at 25°C for an hour. The mixture was filtrated through a filter. After washing twice with 1.5 ml of a buffer for filter washing (50 mM Tris-hydrochloride buffer (pH 7.4), 5 mM MgCl2, 1 mM EDTA, 0.1% BSA), radioactivity of the filter was measured using a liquid scintillation counter. The bovine SENR ligand dose-dependently increased the amount of [$^{35}$S]-guanosine 5'-(γ-thio)triphosphate bound to the membrane fraction. A similar activity was confirmed as well when using swine SENR ligands (SEQ ID NO: 7 and SEQ ID NO:8) or human SENR ligand (human urotensin II) (SEQ ID NO: 22).

EXAMPLE 24 Assay for GTPγS binding activity to human SENR-expression CHO cell membrane fraction induced by human SENR ligand

The [$^{35}$S] -guanosine 5'-(γ-thio)triphosphate binding promoting activity of human SENR ligand (human urotensin II) (SEQ ID NO:22) on human SENR-expression CHO cell membrane fraction was measured by the following method. First, preparation of membrane fraction is described. To 1 x $10^8$ of CHO/hSENR cells was added 10 ml of a homogenate buffer (10 mM $NaHCO_3$, 5 mM EDTA, 0.5 mM PMSF, 1 µg/ml pepstatin, 4 µg/ml E64, 20 µg/ml leupeptin), which was homogenized by using Polytron (12,000 rpm, 1 min.). The cell homogenate was subjected to centrifugation (1,000 g, 15 mins.) to give the supernatant. Next, the supernatant was subjected to ultracentrifugation (Beckman type 30 rotor, 30,000 rpm, an hour). The resulting precipitate was used as a human SENR-expression CHO cell membrane.

The GTPγS binding activity was measured as follows. The human SENR-expression CHO cell membrane was diluted with a buffer for membrane dilution (50 mM Tris-hydrochloride buffer (pH 7.4), 5 mM MgCl2, 150 mM NaCl, 1 µM GDP) to prepare a cell membrane fraction solution for assay having a protein concentration of 30 µg/ml. To 200 µl of the cell membrane fraction solution for assay were added 2 µl of 51.5 nM [$^{35}$S]-guanosine 5'-(γ-thio)triphosphate (NEN Co.) and 2 µl of human SENR ligand (SEQ ID NO:22) in various concentrations. The resulting solution mixture was kept at 25°C for an hour. The mixture was then filtrated through a filter. After washing twice with 1.5 ml of a buffer for filter washing (50 mM Tris-hydrochloride buffer (pH 7.4), 5 mM $MgCl_2$, 1 mM EDTA, 0.1% BSA), radioactivity of the filter was measured using a liquid scintillation counter. The human SENR ligand dose-dependently increased the amount of [$^{35}$S] -guanosine 5'-(γ-thio)triphosphate bound to the membrane fraction. A similar activity was confirmed as well when using swine SENR ligands (SEQ ID NO:7 and SEQ ID NO:8) or bovine SENR ligand (SEQ ID NO:21).

EXAMPLE 25 Preparation of isotope-labeled bovine SENR ligand

Isotope-labeled bovine SENR ligand to use a binding inhibition test was prepared as follows. After 5 μg of bovine SENR ligand (SEQ ID NO:21) was dissolved in 25 μl of 0.4 M sodium acetate (pH 5.6), 200 ng of lacto-peroxidase (Wako Pure Chemicals Co.) was added to the solution and further 1 mCi [$^{125}$I] -sodium iodide (Amersham Pharmacia Biotech Co.) and 200 ng of hydrogen peroxide (10 μl) were added to the mixture. After allowing to stand at room temperature for 10 minutes, 200 ng of hydrogen peroxide (10 μl) were further added to the mixture followed by allowing to stand for 10 minutes. The mixture was purified by HPLC using TSKgel ODS-80$T_s$ column (4.6 mm x 25 cm, Toso) to give [$^{125}$I]-labeled bovine SENR ligand. Similarly, [$^{125}$I]-labeled swine SENR ligands (SEQ ID NO:7 and SEQ ID NO: 8) or human SENR ligand (SEQ ID NO:22) were prepared.

EXAMPLE 26 Binding inhibition test using the isotope-labeled bovine SENR ligand and CHO/SENR cells

A method for the binding inhibition test using [$^{125}$I]-labeled swine SENR ligands prepared in EXAMPLE 25 and rat SENR-expression CHO cells is shown below. The CHO/SENR cells were inoculated on a 24-well plate in $5 \times 10^4$ cells/well followed by cultivation for 48 hours. The cells were then washed with 0.5 ml of MEMα medium containing 0.05% BSA (hereinafter the 0.05% BSA-containing MEMα medium is referred to as reaction buffer). To the cells were added 0.5 ml each of the reaction buffer containing 200 pM [$^{125}$I]-labeled swine SENR ligand to examine the total binding, the reaction buffer containing 200 pM [$^{125}$I] -labeled swine SENR ligand and 1 μM of isotope-unlabeled bovine SENR ligand to examine non-specific binding and, the reaction buffer containing a test fluid and 200 pM [$^{125}$I]-labeled swine SENR ligand to examine the binding activity to SENR receptor, respectively, followed by reacting at room temperature for 30 minutes. After washing the cells with the reaction buffer, 0.2 ml of 0.5 N NaOH was added thereto to lyze the cells. Radioactivity of the lyzed cells was measured with a gamma counter. Specific binding is obtained by reducing non-specific binding from the total binding. The rat SENR receptor binding activity in the test fluid is shown by a ratio of binding obtained by reducing radioactivity of the test fluid-added lyzed cells from the total binding, to specific binding.

EXAMPLE 27 Binding inhibition test using the isotope-labeled human SENR ligand and CHO/hSENR cells

A method for the binding inhibition test using [$^{125}$I]-labeled human SENR ligand prepared by labeling human SENR ligand (SEQ ID NO:22) with [$^{125}$I] as in EXAMPLE 25 and human SENR-expression CHO cells is shown below. CHO/hSENR cells were inoculated on a 24-well plate in $5 \times 10^4$ cells/well followed by cultivation for 48 hours. The cells were then washed with 0.5 ml of MEMα medium containing 0.05% BSA (hereinafter the 0.05% BSA-containing MEMα medium is referred to as reaction buffer). To the cells were added 0.5 ml each of the reaction buffer containing 150 pM [$^{125}$I]-labeled human SENR ligand to examine the total binding, the reaction buffer containing 150 pM [$^{125}$I]-labeled human SENR ligand and 1 μM of isotope-unlabeled human SENR ligand to examine non-specific binding and, the reaction buffer containing a test fluid and 150 pM [$^{125}$I]-labeled human SENR ligand to examine the binding activity to SENR receptor, respectively, followed by reacting at room temperature for 30 minutes. After washing the cells with the reaction buffer, 0.2 ml of 0.5 N NaOH was added thereto to lyze the cells. Radioactivity of the lyzed cells was measured with a gamma counter. Specific binding is obtained by reducing non-specific binding from the total binding. The human SENR receptor binding activity in the test fluid is shown by a ratio of binding obtained by reducing radioactivity of the test fluid-added lyzed cells from the total binding, to specific binding.

EXAMPLE 28 Binding inhibition test using isotope-labeled bovine SENR ligand and CHO/SENR cell membrane fraction

A method for the binding inhibition test using [$^{125}$I]-labeled bovine SENR ligand prepared in EXAMPLE 25 and rat SENR-expression CHO cell membrane fraction is shown below. The membrane fraction prepared from CHO/SENR cells described in EXAMPLE 23 was diluted with a buffer for membrane dilution (50 mM Tris-hydrochloride buffer (pH 7.4), 5 mM $MgCl_2$, 0.1% BSA, 5 mM EDTA, 0.5 mM PMSF, 1 μg/ml pepstatin, 4 μg/ml E64, 20 μg/ml leupeptin) to prepare the cell membrane fraction solution for assay having a protein concentration of 4 μg/ml. To 100 μl of the membrane fraction solution for assay were added 100 μl each of the buffer for membrane dilution containing 200 pM [$^{125}$I]-labeled bovine SENR ligand to examine the total binding, the buffer for membrane dilution containing 200 pM [$^{125}$I]-labeled bovine SENR ligand and 2 μM isotope-unlabeled bovine SENR ligand to examine non-specific binding and furthermore, the buffer for membrane dilution containing a test fluid and 200 pM [$^{125}$I]-labeled bovine SENR ligand to examine the binding activity on rat SENR receptor, respectively, followed by reacting at room temperature for 60 minutes. The mixture was filtrated through a filter. After washing the filter twice with 1.5 ml of the buffer for membrane dilution, radioactivity of the filter was measured with a gamma counter. Specific binding is obtained by reducing non-specific binding from the total binding. The rat SENR receptor binding activity in the test fluid is shown by a ratio of binding obtained by reducing radioactivity of the test fluid-added cell membrane fraction from the total binding, to specific binding. FIG. 12 shows the binding activity of bovine SENR ligand in various concentrations.

EXAMPLE 29 Binding inhibition test using isotope-labeled human SENR ligand and CHO/hSENR cell membrane fraction

A method for the binding inhibition test using [$^{125}$I] -labeled human SENR ligand prepared in EXAMPLE 25 and human SENR-expression CHO cell membrane fraction is shown below. The membrane fraction prepared from CHO/hSENR cells described in EXAMPLE 24 was diluted with a buffer for membrane dilution (50 mM Tris-hydrochloride buffer (pH 7.4), 5 mM $MgCl_2$, 0.1% BSA, 5 mM EDTA, 0.5 mM PMSF, 1 μg/ml pepstatin, 4 μg/ml E64, 20 μg/ml leupeptin) to prepare the cell membrane fraction solution for assay having a protein concentration of 60 μg/ml. To 100 μl of the membrane fraction solution for assay were added 100 μl each of the buffer for membrane dilution containing 150 pM [$^{125}$I]-labeled human SENR ligand to examine the total binding, the buffer for membrane dilution containing 150 pM [$^{125}$I]-labeled human SENR ligand and 2 μM isotope-unlabeled human SENR ligand to examine non-specific binding and furthermore, the buffer for membrane dilution containing a test fluid and 150 pM [$^{125}$I]-labeled human SENR ligand to examine the binding activity on human SENR receptor, respectively, followed by reacting at room temperature for 60 minutes. The mixture was filtrated through a filter. After washing the filter twice with 1.5 ml of the buffer for membrane dilution, radioactivity of the filter was measured with a gamma counter. Specific binding is obtained by reducing non-specific binding from the total binding. The human SENR receptor binding activity in the test fluid is shown by a ratio of binding obtained by reducing radioactivity of the test fluid-added cell membrane fraction from the total binding, to specific binding. FIG. 13 shows the binding activity of human SENR ligand in various concentrations.

EXAMPLE 30 cAMP synthesis suppressing activity of synthetic bovine SENR ligand on rat SENR-expression CHO cells

A method for the cAMP synthesis suppressing activity of the bovine SENR ligand (SEQ ID NO:21) synthesized in EXAMPLE 14 on rat SENR-expression CHO cells is shown below. The CHO/SENR cells were inoculated on a 24-well plate in 5 × $10^4$ cells/well followed by cultivation for 48 hours. The cells were then washed with Hanks' buffer (pH 7.4) containing 0.2 mM 3-isobutyl-methylxanthine, 0.05% BSA and 20 mM HEPES (hereinafter Hanks' buffer (pH 7.4) containing 0.2 mM 3-isobutyl-methylxanthine, 0.05% BSA and 20 mM HEPES is referred to as a reaction buffer). Thereafter, 0.5 ml of the reaction buffer was added to the system, which was kept in the medium for 30 minutes. The reaction buffer was removed and 0.25 ml of a fresh reaction buffer was added to the cells. Then, 0.25 ml of the reaction buffer containing various amounts of bovine SENR ligand and 2 μM forskolin was added to the cells followed by reacting at 37°C for 24 minutes. By adding 100 μl of 20% perchloric acid, the reaction was terminated. The reaction mixture was then allowed to stand on ice for an hour to extract intracellular CAMP. The amount of cAMP in the extract was measured using cAMP EIA kit (Amersham Pharmacia Biotech). The results reveal that the bovine SENR ligand obviously reduced the amount of intracellular CAMP in the concentration of 0.3 nM. When the peptide concentration increased, the amount of intracellular CAMP decreased dose dependently. A similar activity was confirmed as well when using swine SENR ligands ①and ②(SEQ ID NO:7 and SEQ ID NO:8) or human SENR ligand (human urotensin II)(SEQ ID NO:22).

EXAMPLE 31 cAMP synthesis suppressing activity of synthetic human SENR ligand on human SENR-expression CHO cells

A method for the cAMP synthesis suppressing activity of the human SENR ligand (human urotensin II) (SEQ ID NO:22) synthesized in EXAMPLE 15 on human SENR-expression CHO cells is shown below. The CHO/hSENR cells were inoculated on a 24-well plate in 5 × $10^4$ cells/well followed by cultivation for 48 hours. The cells were then washed with Hanks' buffer (pH 7.4) containing 0.2 mM 3-isobutyl-methylxanthine, 0.05% BSA and 20 mM HEPES (hereinafter Hanks' buffer (pH 7.4) containing 0.2 mM 3-isobutyl-methylxanthine, 0.05% BSA and 20 mM HEPES is referred to as a reaction buffer). Thereafter, 0.5 ml of the reaction buffer was added to the system, which was kept in the medium for 30 minutes. The reaction buffer was removed and 0.25 ml of a fresh reaction buffer was added to the cells. Then, 0.25 ml of the reaction buffer containing various amounts of human SENR ligand and 2 μM forskolin was added to the cells followed by reacting at 37°C for 24 minutes. By adding 100 μl of 20% perchloric acid, the reaction was terminated. The reaction mixture was then allowed to stand on ice for an hour to extract intracellular cAMP. The amount of cAMP in the extract was measured using cAMP EIA kit (Amersham Pharmacia Biotech). The results reveal that the human SENR ligand obviously reduced the amount of intracellular cAMP in the concentration of 0.3 nM. When the peptide concentration increased, the amount of intracellular cAMP decreased dose-dependently. A similar activity was confirmed as well when using swine SENR ligands ①and ②(SEQ ID NO:7 and SEQ ID NO:8) or bovine SENR ligand (SEQ ID NO:21).

EXAMPLE 32 Determination of the partial sequence of swine SENR ligand precursor protein by PCR

Using bovine genomic DNA (Clonetech Laboratories, Inc.) as a template, PCR was carried out using the primers shown by SEQ ID NO:10 and SEQ ID NO:11. The reaction solution was composed of 5 μM of the synthetic primer shown by SEQ ID NO:10, 1 μM of the synthetic primer shown by SEQ ID NO:11, 50 ng of the template DNA, 0.2 mM dNTPs, 2.5 mM $MgCl_2$, 0.4 μl of AmpliTaq Gold DNA polymerase (Perkin-Elmer Co.) and 1/10 volume of 10-fold concentrated AmpliTaq Gold buffer, which were mixed together to make the whole volume of the reaction solution 40 μl. Using Thermal Cycler (Perkin-Elmer Co.) for amplification, after maintaining at 95°C for 9 minutes, a cycle was set to include 94°C for 15 seconds, 60°C for 20 seconds and 72°C for 20 seconds, with 4 repetitions; a cycle of 94°C for 15 seconds, 52.5°C for 20 seconds and 72°C for 20 seconds, with 6 repetitions; a cycle of 94°C for 20 seconds, 52.5°C for 20 seconds and 72°C for 20 seconds, with 6 repetitions; a cycle of 94°C for 20 seconds, 50°C for 20 seconds and 72°C for 20 seconds, with 8 repetitions; and a cycle of 94°C for 15 seconds, 48°C for 20 seconds and 72°C for 20 seconds, with 30 repetitions, followed by maintaining at 72°C for 7 minutes. Using TOPO TA cloning kit (Invitrogen Inc.), 2 μl of the reaction solution thus obtained was subcloned to plasmid vector pcr 2.1 and then introduced into *Escherichia coli* TOP10. From the resulting transformant, the plasmid DNA was purified using QIA prep8 mini prep (Qiagen Co.). The reaction for base sequencing was carried out using DyeDeoxy Terminator Cycle Sequence Kit (Perkin-Elmer Co.). The DNAs were decoded using an automated fluorescent sequencer. As a result, SEQ ID NO:20, which are considered to encode a part of the swine SENR ligand precursor because of its similarity to the sequence of swine SENR ligand precursor, was obtained as the PCR product.

EXAMPLE 33 Preparation of total bovine brain-derived cDNA

Using ThermoScript reverse transcriptase (Gibco BRL Co.) and oligo(dT) primer, reverse transcription was carried out from 1.0 μg of total bovine brain-derived poly(A) $^+$RNA (Clonetech Laboratories, Inc.) at 60°C, following the manual. Thus, cDNAs were prepared. With the cDNAs, a second strand was synthesized and an adapter sequence was added, following the manual of Marathon cDNA amplification kit (Clonetech Laboratories, Inc.).

EXAMPLE 34 Acquisition of the 5' sequence of the gene encoding bovine SENR ligand precursor protein by 5' RACE method

Using as a template the double-stranded cDNA solution obtained in EXAMPLE 33, which corresponds to 4 ng of mRNA, PCR was carried out using adapter primer AP1 attached to Marathon cDNA amplification kit (Clonetech Laboratories, Inc.) and the primer shown by SEQ ID NO:31. The reaction solution was composed of 0.5 μM of the primer shown by SEQ ID NO:31, 0.2 μM of AP1, 0.2 mM dNTPs, 2.5 mM $MgCl_2$, 0.2 μl of AmpliTaq Gold DNA polymerase (Perkin-Elmer Co.) and 1/10 volume of 10-fold concentrated AmpliTaq Gold buffer, which were mixed together to make the whole volume of the reaction solution 20 μl. Using Thermal Cycler (Perkin-Elmer Co.) for amplification, after maintaining at 95°C for 9 minutes, a cycle was set to include 95°C for 10 seconds and 68°C for 1 minute, with 40 repetitions. Using a 0.04 μl aliquot of the reaction solution as a template, PCR was carried out except that the adapter primer AP1 and the primer shown by SEQ ID NO:31 were replaced by the adapter primer AP2 and the primer shown by SEQ ID NO:32, respectively. The reaction solution was composed of 0.5 μM of the primer shown by SEQ ID NO: 32, 0.2 μM of AP2, 0.2 mM dNTPs, 2.5 mM $MgCl_2$, 0.2 μl of AmpliTaq Gold DNA polymerase (Perkin-Elmer Co.) and 1/10 volume of 10-fold concentrated AmpliTaq Gold buffer, which were mixed together to make the whole volume of the reaction solution 20 μl. Using Thermal Cycler (Perkin-Elmer Co.) for amplification, after maintaining at 95°C for 9 minutes, a cycle was set to include 95°C for 10 seconds and 66°C for 1 minute, with 40 repetitions, followed by maintaining at 72°C for 10 minutes. The PCR solution was subjected to electrophoresis using 3.5% Nusieve GTG Agarose (Takara Shuzo Co.) and DNA was extracted from the bands around 420 bp, which were detected by ethidium bromide staining, using GeneClean Spin kit (Bio 101 Co.). The DNA was subcloned to plasmid pcr 2.1 using TOPO TA cloning kit (Invitrogen Co.) and then introduced into *Escherichia coli* TOPO10. From the resulting transformant, the plasmid DNA was purified using QIA prep8 mini prep kit (Qiagen Co.). The reaction for base sequencing was carried out using DyeDeoxy Terminator Cycle Sequence Kit (Perkin-Elmer Co.). Decoding by an automated fluorescent sequencer gave the sequence shown by SEQ ID NO: 33.

EXAMPLE 35 Acquisition of the 3' sequence of the gene encoding bovine SENR ligand precursor protein by 3' RACE method

Using as a template the double-stranded cDNA solution obtained in EXAMPLE 33, which corresponds to 4 ng of mRNA, PCR was carried out using adapter primer AP1 attached to Marathon cDNA amplification kit (Clonetech Laboratories, Inc.) and the primer shown by SEQ ID NO:34. The reaction solution was composed of 0.2 μM of the primer shown by SEQ ID NO:34, 0.2 μM of AP1, 0.2 mM dNTPs, 2.5 mM $MgCl_2$, 0.2 μl of AmpliTaq Gold DNA polymerase (Perkin-Elmer Co.) and 1/10 volume of 10-fold concentrated AmpliTaq Gold buffer, which were mixed together to make the whole volume of the reaction solution 20 μl. Using Thermal Cycler (Perkin-Elmer Co.) for amplification, after maintaining at 95°C for 9 minutes, a cycle was set to include 95°C for 10 seconds and 68°C for 1 minute, with 40 repetitions. Using a 0.04 μl aliquot of the reaction solution as a template, PCR was carried out except that the adapter primer AP1 and the primer shown by SEQ ID NO:34 were replaced by the adapter primer AP2 and the primer shown by SEQ ID NO:35, respectively. The reaction solution was composed of 0.2 μM of the primer shown by SEQ ID NO: 35, 0.2 μM of AP2, 0.2 mM dNTPs, 2.5 mM $MgCl_2$, 0.2 μl of AmpliTaq Gold DNA polymerase (Perkin-Elmer Co.) and 1/10 volume of 10-fold concentrated AmpliTaq Gold buffer, which were mixed together to make the whole volume of the reaction solution 20 μl. Using Thermal Cycler (Perkin-Elmer Co.) for amplification, after maintaining at 95°C for 9 minutes, a cycle was set to include 95°C for 10 seconds and 66°C for 1 minute, with 40 repetitions, followed by maintaining at 72°C for 10 minutes. The PCR solution was subjected to electrophoresis using 3.5% Nusieve GTG Agarose (Takara Shuzo Co.) and DNA was extracted from the bands around 300 bp, which were detected by ethidium bromide staining, using GeneClean Spin kit (Bio 101 Co.). The DNA was subcloned to plasmid pcr 2.1 using TOPO TA cloning kit (Invitrogen Co.) and then introduced into *Escherichia coli* TOPO10. From the resulting transformant, the plasmid DNA was purified using QIA prep8 mini prep kit (Qiagen Co.). The reaction for base sequencing was carried out using DyeDeoxy Terminator Cycle Sequence Kit (Perkin-Elmer Co.). Decoding by an automated fluorescent sequencer gave the sequence shown by SEQ ID NO:36.

EXAMPLE 36 Acquisition of the full-length sequence of the gene encoding bovine SENR ligand precursor protein

In order to acquire a sequence containing the full length of cDNA encoding bovine SENR ligand precursor protein, which is deduced from the sequence information at the 5' and 3' end obtained by the RACE technique as described in EXAMPLES 34 and 35, PCR was carried out using the primers shown by SEQ ID NO:37 and SEQ ID NO:38, in which an aliquot of the double-stranded cDNA solution corresponding to 4 ng of mRNA was used as a template. The reaction solution was composed of 0.5 μM each of the two primers, 0.2 mM dNTPs, 2.5 mM $MgCl_2$, 0.2 μl of AmpliTaq Gold DNA polymerase (Perkin-Elmer Co.) and 1/10 volume of 10-fold concentrated AmpliTaq Gold buffer, which were mixed together to make the whole volume of the reaction solution 20 μl. Using Thermal Cycler (Perkin-Elmer Co.) for amplification, after maintaining at 95°C for 9 minutes, a cycle was set to include 95°C for 10 seconds, 62°C for 20 seconds and 72°C for 1 minute, with 40 repetitions, followed by maintaining at 72°C for 10 minutes. The PCR solution was subjected to electrophoresis using 3.5% Nusieve GTG Agarose (Takara Shuzo Co.) and DNA was extracted from the bands around 490 bp, which were detected by ethidium bromide staining, using GeneClean Spin kit (Bio 101 Co.). The DNA was subcloned to plasmid pcr 2.1 using TOPO TA cloning kit (Invitrogen Co.) and then introduced into *Escherichia coli* TOPO10. From the resulting transformant, the plasmid DNA was purified using QIA prep8 mini prep kit (Qiagen Co.). The reaction for base sequencing was carried out using DyeDeoxy Terminator Cycle Sequence Kit (Perkin-Elmer Co.). Decoding by an automated fluorescent sequencer gave the sequence shown by SEQ ID NO:30. In this sequence, the full length of bovine SENR ligand precursor was contained. Thus, *Escherichia coli* TOP10 was transformed by this plasmid to obtain *Escherichia coli* TOP10/pCR-buro. The amino acid sequence of the bovine SENR ligand precursor protein translated from SEQ ID NO:30 are shown in SEQ ID NO:29. Also, the amino acid sequence of bovine SENR ligand deduced from the amino acid sequence of bovine SENR ligand precursor protein is shown by SEQ ID NO:21 and the base sequence encoding the same is shown by SEQ ID NO:28, respectively. The total base sequence and amino acid sequence of bovine SENR ligand precursor protein are shown in FIG. 14.

EXAMPLE 37 Preparation of the antibody to SENR ligand polypeptide

Using as an antigen "Haze" or long-jawed mudsucker, urotensin II (SEQ ID NO:39) which coincides in the C-terminal structure (Cys-Phe-Trp-Lys-Tyr-Cys-Val) with swine, bovine and human SENR ligand polypeptides, an antibody capable of recognizing the C terminus of SENR ligand polypeptides was prepared. "Haze" urotensin II peptide, 1 mg, and 4 mg of BTG (bovine thyroglobulin) were bound to one another, using 30 mg of ECDI (1-ethyl-3-(3-dimethylaminopropyl) -carbodiimide, Dojin Kagaku Co.) to produce the "Haze" urotensin II-carrier protein complex. The "Haze" urotensin II-carrier protein complex was dialyzed against 0.15 M NaCl aqueous solution, which was then mixed with dialysate and Freund's complete adjuvant. Using the mixture as an antigen, Balb/c mice (female, 6 to 8 weeks old) were primary immunized with "Haze" urotensin II in 20 μg/mouse. Three weeks after the first immunization, the complex was mixed with Freund's incomplete adjuvant, which was used as an antigen for second immunization. The animal was immunized with a mixture of the complex and Freund's incomplete adjuvant every 2 weeks until its antibody titer increased.

The antibody titer was assayed by enzyme immunoassay utilizing biotinylated "Haze" urotensin II peptide. The biotinylated "Haze" urotensin II peptide ([N-biotinyl-$Ala^1$]-urotensin II) was obtained by subjecting the reaction product of NHS-biotin (N-hydroxysuccinimidobiotin) and "Haze" urotensin II peptide to preparative HPLC. The structure of the thus obtained N-terminal labeled biotinylated "Haze" urotensin II peptide was identified by the fact that no N terminus could be detected by mass analysis and N-terminal sequencing. Enzyme immunoassay was performed as follows. A 96-well immunoplate coated with anti-mouse IgG sheep IgG fraction solution was blocked with Block Ace (Dai-Nippon Pharmaceutical Co.) followed by reacting serially diluted immune mouse sera and the biotinylated "Haze" urotensin II peptide in each well at 4°C for 16 hours. After washing the well, peroxidase-labeled streptoavidin was reacted in the well at room temperature for 4 hours. Finally, the well was washed and a peroxidase substrate was added to the well, whereby a color generated on the substrate was measured with a 96-well multi-photometer. The sera added to the wells in which the substrate generated a color was determined to be sera with an increased antibody titer. Since the antibody detected here binds to the N-terminal labeled biotinylated "Haze" urotensin II, the antibody is considered to recognize the C-terminal structure of the peptide. The antibody contained in these sera is capable of recognizing swine, bovine and human SENR ligand polypeptides. This was confirmed by the results that in the enzyme immunoassay described above, binding between the biotinylated "Haze" urotensin II peptide and the antibody was inhibited by adding these peptides to the well and thus, a color formation was inhibited.

(Sequence Listing Free Text)

SEQ ID NO: 7

Other information on the sequence: The two Cys residues at the 6th and 11th form intramolecular disulfide bond.

SEQ ID NO: 8

Other information on the sequence: The two Cys residues at the 6th and 11th form intramolecular disulfide bond.

SEQ ID NO: 21

Other information on the sequence: The two Cys residues at the 6th and 11th form intramolecular disulfide bond.

SEQ ID NO: 22

Other information on the sequence: The two Cys residues at the 5th and 10th form intramolecular disulfide bond.

SEQ ID NO: 39

Other information on the sequence: The two Cys residues at the 5th and 11th form intramolecular disulfide bond.

Industrial Applicability

The DNA encoding the polypeptide of the present invention or the polypeptide of the present invention can be used: (1) for the survey of physiological activities that the polypeptide of the present invention possesses, (2) for preparing synthetic oligonucleotide probes or primers for PCR, (3) for acquiring DNAs encoding ligands to SENR or precursor proteins, (4) for the development of the receptor-binding assay system using the expression system of recombinant receptor protein and screening of a candidate drug, (5) for acquiring antibodies and antisera, (6) for the development of diagnostic agents using DNAs, RNAs, antibodies or antisera, (7) for the development of drugs such as agents for regulating central nervous functions, circulatory functions, heart functions, renal functions, urinary functions, sensory functions, etc., (8) for gene therapy, and the like.

**Claims**

1. A polypeptide comprising the same or substantially the same amino acid sequence as the amino acid sequence represented by SEQ ID NO: 7, its amide or ester, or a salt thereof.

2. A polypeptide or its amide or ester, or a salt thereof according to claim 1, wherein substantially the same amino acid sequence is represented by SEQ ID NO: 8 or SEQ ID NO: 21.

3. A precursor peptide of the polypeptide according to claim 1, or a salt thereof.

4. A precursor protein or a salt thereof according to claim 3, comprising the same or substantially the same amino acid sequence represented by SEQ ID NO: 18 or SEQ ID NO: 19.

5. A DNA containing a DNA having a base sequence encoding the polypeptide according to claim 1.

6. A DNA according to claim 5, which has the base sequence represented by SEQ ID NO: 27 or SEQ ID NO: 28.

7. A DNA containing a DNA having a base sequence encoding the precursor protein according to claim 3.

8. A DNA according to claim 7, which contains the base sequence represented by SEQ ID NO: 15, SEQ ID NO: 16 or SEQ ID NO: 17.

9. A recombinant vector containing the DNA according to claim 5 or 7.

10. A transformant transformed with the recombinant vector according to claim 9.

11. A method for manufacturing the polypeptide, its amide or ester, or a salt thereof according to claim 1 or the precursor protein or its salt according to claim 3, which comprises culturing the transformant according to claim 10, producing and accumulating the polypeptide according to claim 1 or the precursor protein according to claim 3 and collecting the same.

12. An antibody to the polypeptide, its amide or ester, or a salt thereof according to claim 1 or to the precursor protein or its salt according to claim 3.

13. A pharmaceutical composition comprising the polypeptide, its amide or ester, or a salt thereof according to claim 1 or the precursor protein or its salt according to claim 3.

14. A pharmaceutical composition comprising the DNA according to claim 5 or 7.

15. A pharmaceutical composition according to claim 13 or 14, which is an agent for regulating the central functions, an agent for regulating the circulatory functions, an agent for regulating the cardiac functions, an agent for regulating the renal functions, an agent for regulating the urinary functions or an agent for regulating the sensory functions.

16. A method for screening a compound or its salt that alters the binding property between an SENR and the polypeptide, its amide or ester, or a salt thereof according to claim 1, or the precursor protein or its salt according to claim 3, which comprises using the polypeptide, its amide or ester, or a salt thereof according to claim 1 or the precursor protein or its salt according to claim 3.

17. A kit for screening a compound or its salt that alters the binding property between an SENR and the polypeptide, its amide or ester, or a salt thereof according to claim 1, or the precursor protein or its salt according to claim 3, comprising the polypeptide, its amide or ester, or a salt thereof according to claim 1 or the precursor protein or its salt according to claim 3.

18. A method for screening a compound or its salt that alters the binding property between an SENR and a polypeptide containing the amino acid sequence shown by SEQ ID NO:22, or its amide or ester, or a salt thereof, which comprises using a polypeptide containing the amino acid sequence shown by SEQ ID NO:22, or its amide or ester, or a salt thereof.

**19.** A kit for screening a compound or its salt that alters the binding property between an SENR and a polypeptide containing the amino acid sequence shown by SEQ ID NO:22, its amide or ester, or a salt thereof, comprising a polypeptide containing the amino acid sequence shown by SEQ ID NO:22, or its amide or ester, or a salt thereof.

**20.** A compound or a salt thereof that alters the binding property between 1) an SENR and the polypeptide, its amide or ester, or a salt thereof according to claim 1 or the precursor protein or its salt according to claim 3, or 2) an SENR and a polypeptide containing the amino acid sequence shown by SEQ ID NO: 22, its amide or ester, , which is obtainable by using the screening method according to claim 16 or 18 or by using the screening kit according to claim 17 or 19.

**21.** A composition for the prevention and treatment of hypertension, comprising the compound or its salt according to claim 20.

**22.** A method for quantifying the polypeptide, its amide or ester, or a salt thereof according to claim 1 or the protein or its salt according to claim 3, which comprises using the antibody according to claim 12.

**23.** A diagnostic composition for disease associated with the functions of the polypeptide, its amide or ester, or a salt thereof according to claim 1 or the precursor protein or its salt according to claim 3, comprising the antibody according to claim 12.

# Fig.1

```
5'- GTCGACATGG CTCTGAGCCT GGAGTCTACA ACAAGCTTTC ATATGCTCAC    50
    CGTGTCCGGA AGCACTGTGA CTGAGCTGCC TGGTGACTCC AACGTGTCCC   100
    TCAACAGTTC CTGGTCCGGC CCAACAGATC CCAGCTCCCT GAAAGACCTT   150
    GTGGCCACGG GTGTCATCGG GGCAGTGCTC TCAGCCATGG GTGTGGTGGG   200
    CATGGTGGGA AATGTATACA CTTTGGTGGT CATGTGCCGG TTTCTGCGTG   250
    CCTCGGCCTC CATGTACGTC TATGTGGTCA ACCTAGCGCT GGCTGATCTG   300
    CTGTACCTGC TGAGCATTCC CTTCATCATA GCCACCTACG TCACTAAGGA   350
    CTGGCACTTT GGAGATGTGG GCTGCAGAGT CCTCTTTAGC CTGGACTTCC   400
    TGACAATGCA CGCCAGCATC TTCACCCTGA CCATAATGAG CAGCGAACGC   450
    TATGCAGCCG TACTGAGGCC TCTGGACACA GTCCAGCGCT CCAAGGGTTA   500
    CCGTAAGCTG CTGGTGCTGG GCACCTGGTT GCTGGCACTG CTGCTGACCC   550
    TACCCATGAT GCTTGCCATC CAGCTGGTCC GCAGGGGCTC TAAGAGCCTC   600
    TGCCTGCCAG CCTGGGGCCC TCGTGCCCAC CGTACTTACC TAACGTTGCT   650
    CTTTGGGACC AGCATTGTGG GGCCTGGCTT GGTCATTGGG CTGCTCTATG   700
    TCCGTCTGGC CAGGGCCTAC TGGCTATCTC AGCAAGCTTC TTTCAAGCAG   750
    ACACGGCGGC TGCCCAACCC CAGGGTGCTC TACCTCATCC TTGGTATCGT   800
    CCTTCTCTTC TGGGCCTGCT TTCTACCCTT CTGGCTGTGG CAGCTGCTGG   850
    CCCAGTACCA CGAGGCCATG CCACTGACTC CCGAGACTGC ACGCATTGTC   900
    AACTACCTGA CCACCTGCCT CACTTATGGC AACAGTTGCA TCAATCCCTT   950
    CCTCTACACT CTGCTCACCA AGAACTATCG AGAGTACCTA CGTGGCCGCC  1000
    AGCGGTCACT GGGTAGTAGT TGCCACAGCC CAGGGAGTCC TGGCAGCTTC  1050
    CTGCCCAGCC GAGTCCACCT CCAGCAGGAC TCGGGCCGCT CGCTGTCCTC  1100
    CAGCAGCCAA CAGGCCACAG AGACCCTCAT GCTGTCTCCA GTCCCCCGTA  1150
    ACGGGGCCCT TCTCTGAGAG TGCACTGTGC AATACTAGT-3'           1189
```

Fig.2

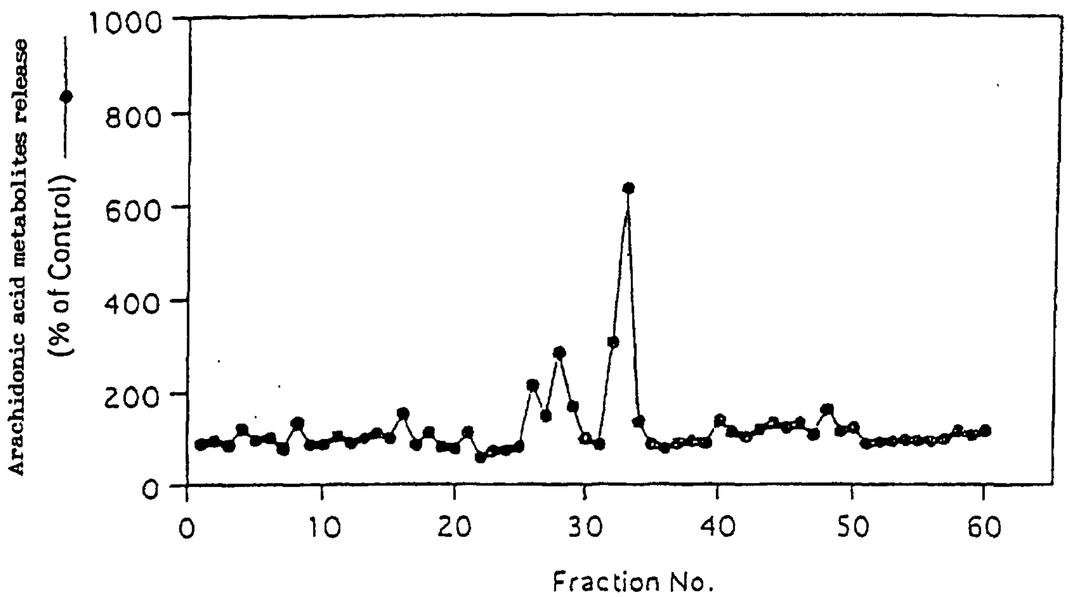
Arachidonic acid metabolites release
(% of Control)
1000
800
600
400
200
0
0
10
20
30
40
50
60
Fraction No.

Fig.3

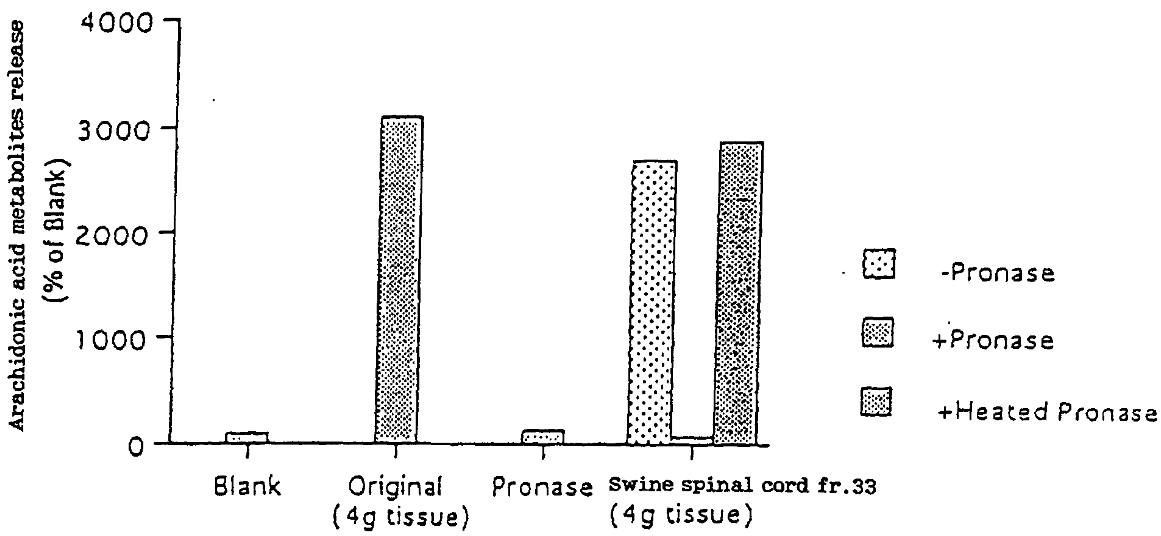
Arachidonic acid metabolites release
(% of Blank)
4000
3000
2000
1000
0
Blank
Original
(4g tissue)
Pronase
Swine spinal cord fr.33
(4g tissue)
-Pronase
+Pronase
+Heated Pronase

Fig.4

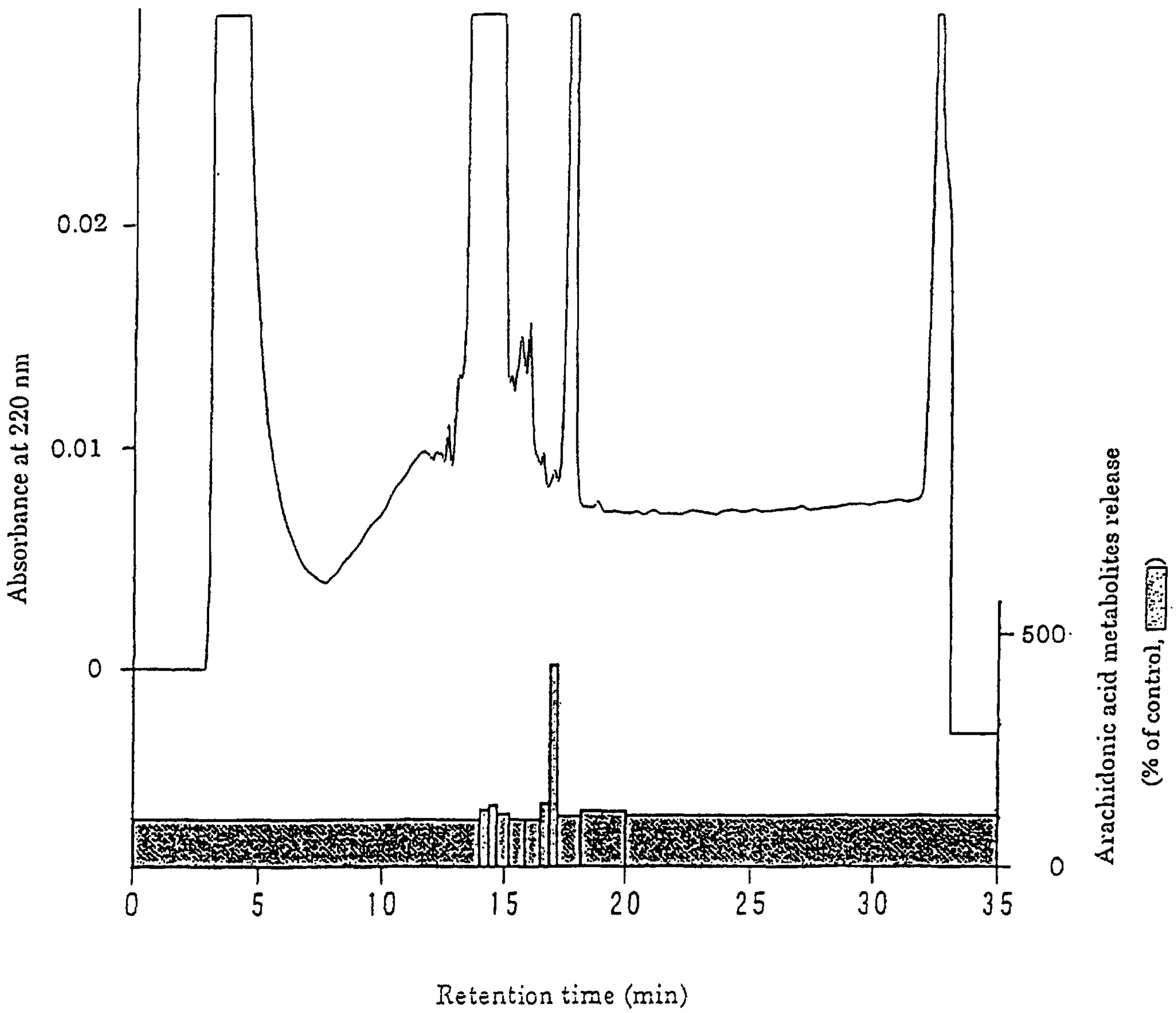
0.02
0.01
0
Absorbance at 220 nm
500
0
Arachidonic acid metabolites release
(% of control, )
0
5
10
15
20
25
30
35
Retention time (min)

Fig.5

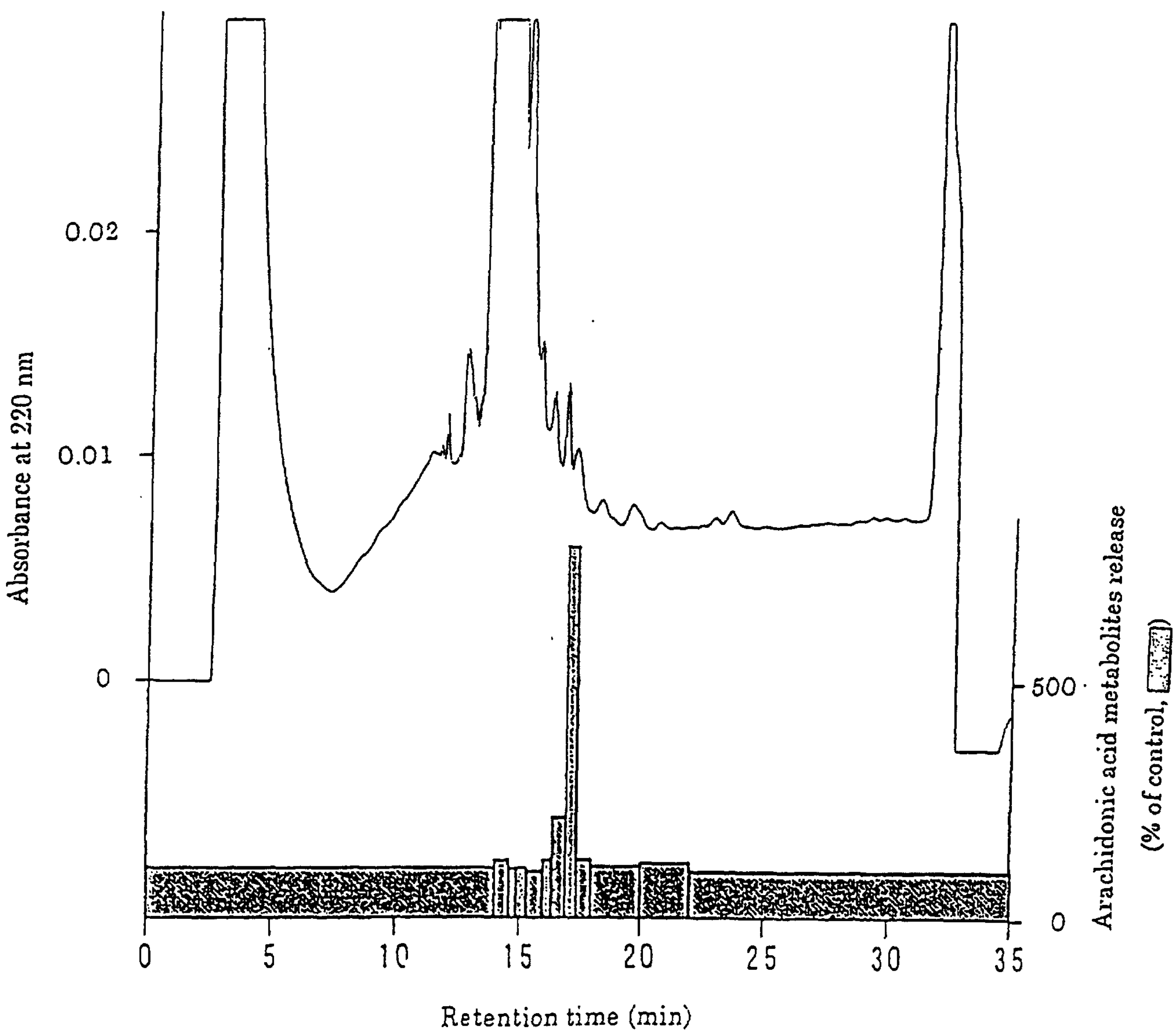
0.02
0.01
0
Absorbance at 220 nm
500
0
Arachidonic acid metabolites release
(% of control, )
0
5
10
15
20
25
30
35
Retention time (min)

Fig.6

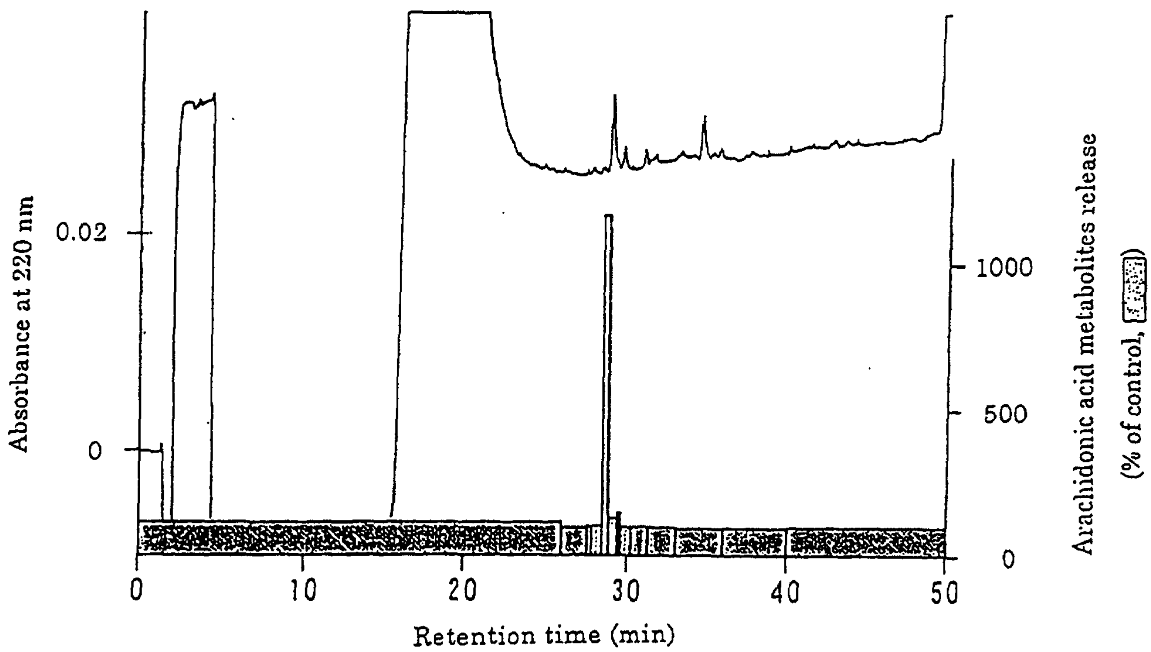
Absorbance at 220 nm
0.02
0
1000
500
0
Arachidonic acid metabolites release
(% of control, )
0
10
20
30
40
50
Retention time (min)

Fig.7

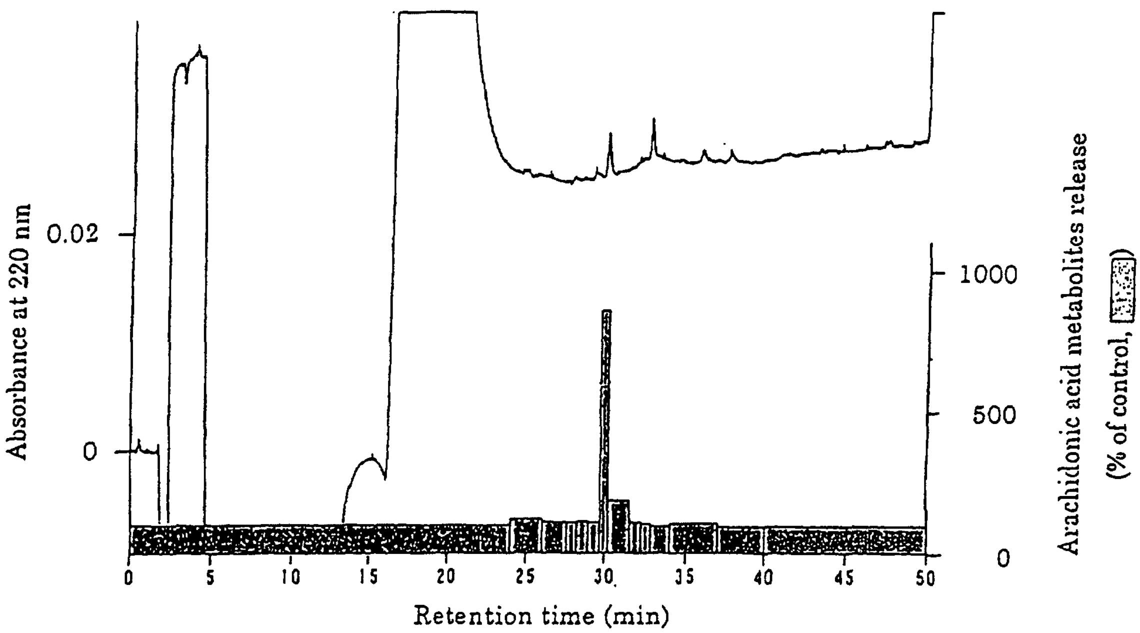
Absorbance at 220 nm
0.02
0
1000
500
0
Arachidonic acid metabolites release
(% of control, )
0
5
10
15
20
25
30
35
40
45
50
Retention time (min)

# Fig.8

1

AGT TGA GGC TTC GGA CCA ACA GAA GCC AGG AAG GAA GTG TCC TGC CTC CTG CCA GTC ATG
Met

10 20

TCC AAG CTG GTC CCC TGC TTG CTC CTC CTA GGA TGC TTA GGT CTC CTC TTC GCT CTT CCC
Ser Lys Leu Val Pro Cys Leu Leu Leu Leu Gly Cys Leu Gly Leu Leu Phe Ala Leu Pro

30 40

GTC CCT GAC TCC AGG AAA GAG CCC CTG CCC TTC TCA GCA CCT GAA GAT GTC AGA TCA GCT
Val Pro Asp Ser Arg Lys Glu Pro Leu Pro Phe Ser Ala Pro Glu Asp Val Arg Ser Ala

50 60

TGG GAT GAG CTG GAA AGA GCC TCC CTT CTT CAG ATG CTG CCA GAG ACG CCA GGT GCA GAG
Trp Asp Glu Leu Glu Arg Ala Ser Leu Leu Gln Met Leu Pro Glu Thr Pro Gly Ala Glu

70 80

GCA GGA GAG GAT CTC AGG GAA GCA GAT GCC GGA ATG GAC ATT TTT TAC CCA AGA GGA GAA
Ala Gly Glu Asp Leu Arg Glu Ala Asp Ala Gly Met Asp Ile Phe Tyr Pro Arg Gly Glu

90 100

ATG AGA AAG GCT TTC TCT GGA CAA GAT CCT AAC ATT TTT CTG AGT CAC CTT TTG GCC AGA
Met Arg Lys Ala Phe Ser Gly Gln Asp Pro Asn Ile Phe Leu Ser His Leu Leu Ala Arg

110 120

ATC AAG AAA CCA TAC AAG AAA CGT GGG CCC CCC TCT GAA TGC TTC TGG AAA TAC TGT GTC
Ile Lys Lys Pro Tyr Lys Lys Arg Gly Pro Pro Ser Glu Cys Phe Trp Lys Tyr Cys Val

TGA AGT CAC CTC AAC AAC AAC CAT CTT AGA AAA TGT AAA AAA AGT GCT TGA CTT GAC AGC

AGT GCA GAT GAA AAA CCA GGC AAA CCC TAC TCT GTT CAC TAT TAT CTG GAA AAT AAA CCC

TTT GTG TTT GGC AAG TTA AAA AAA AAA AAA AAA AAA AAA AAA AAA AAA AAA AAA AAA AAA

AAA AAA AAA AAA AAA AAA AAA AAA AAA AAA AAA AAA AAA AAA AAA AAA A

Fig.9

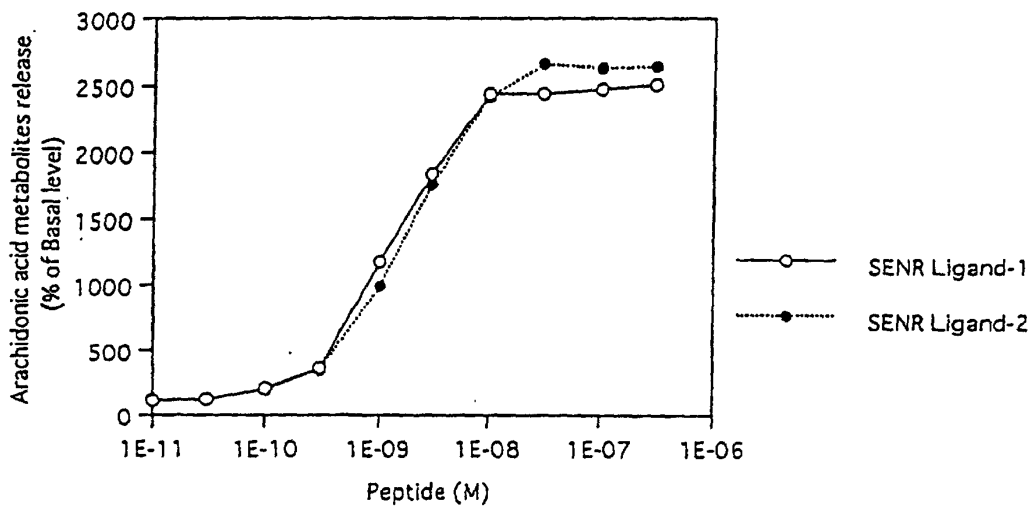
3000
2500
2000
1500
1000
500
0
Arachidonic acid metabolites release
(% of Basal level)
1E-11
1E-10
1E-09
1E-08
1E-07
1E-06
Peptide (M)
SENR Ligand-1
SENR Ligand-2

Fig.10

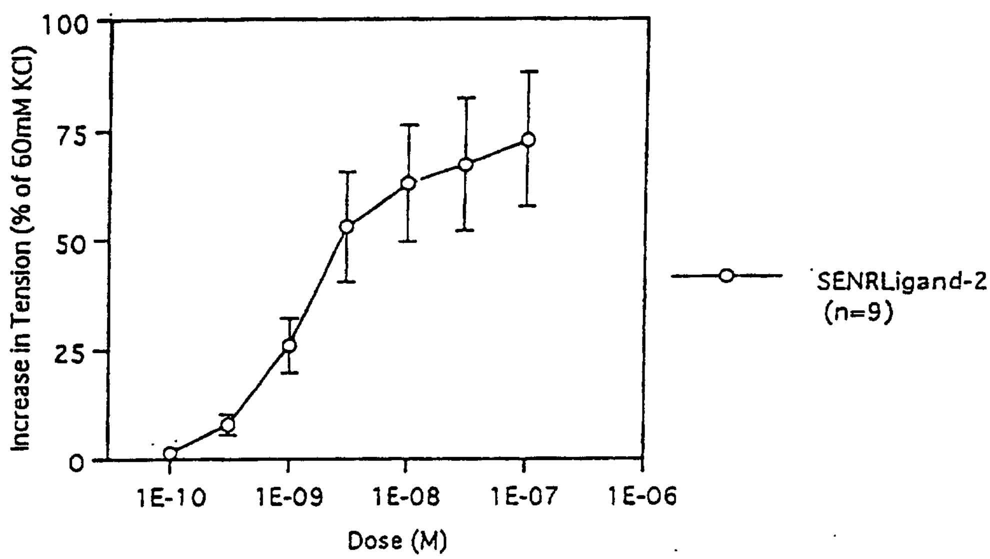
100
75
50
25
0
Increase in Tension (% of 60mM KCl)
1E-10
1E-09
1E-08
1E-07
1E-06
Dose (M)
SENRLigand-2
(n=9)

Fig.11

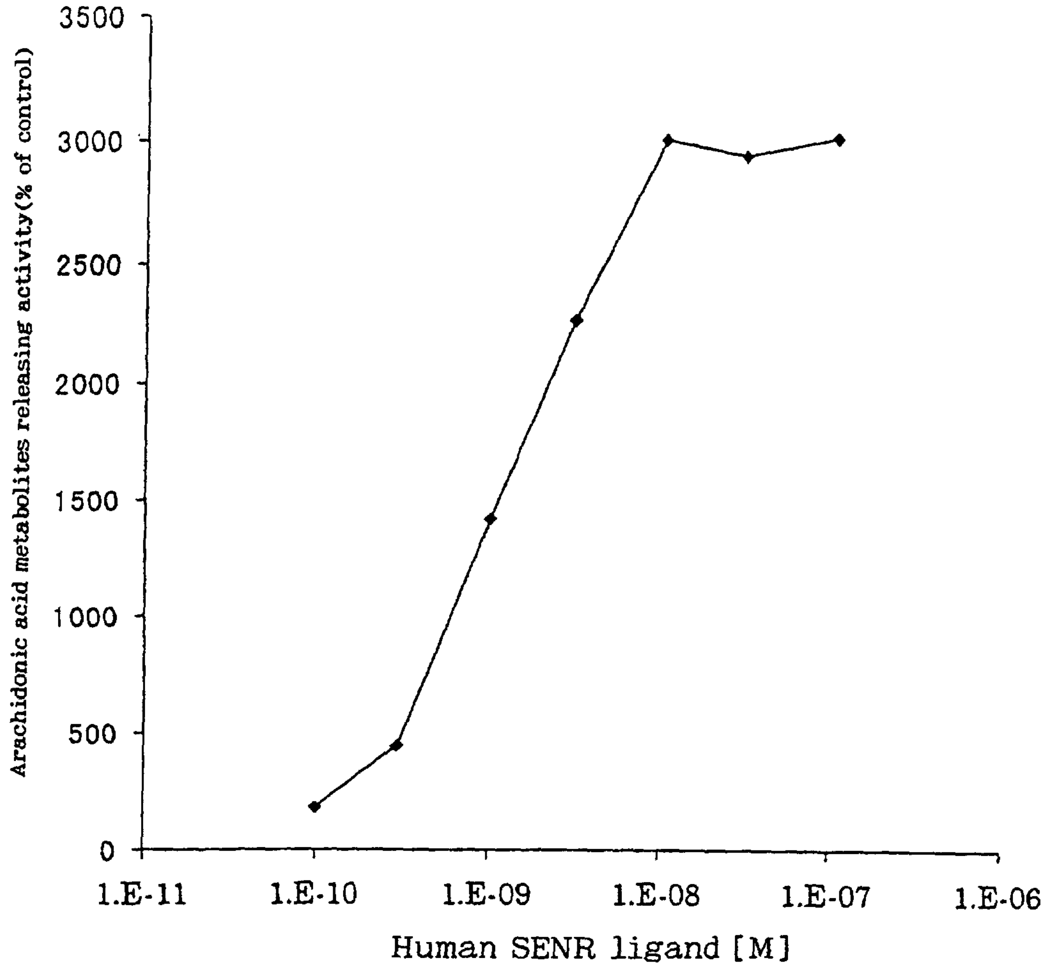
3500
3000
2500
2000
1500
1000
500
0
Arachidonic acid metabolites releasing activity(% of control)
1.E-11
1.E-10
1.E-09
1.E-08
1.E-07
1.E-06
Human SENR ligand [M]

Fig.12

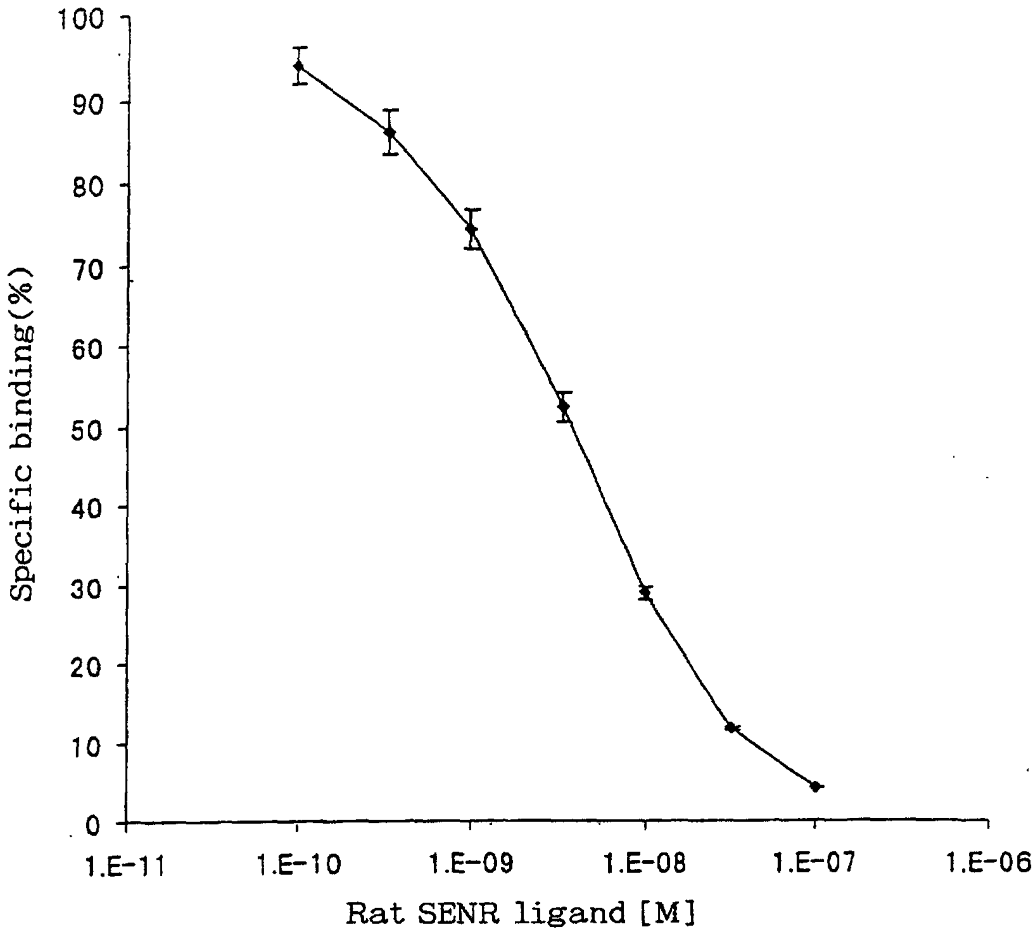
100
90
80
70
60
50
40
30
20
10
0
Specific binding(%)
1.E-11
1.E-10
1.E-09
1.E-08
1.E-07
1.E-06
Rat SENR ligand [M]

Fig.13

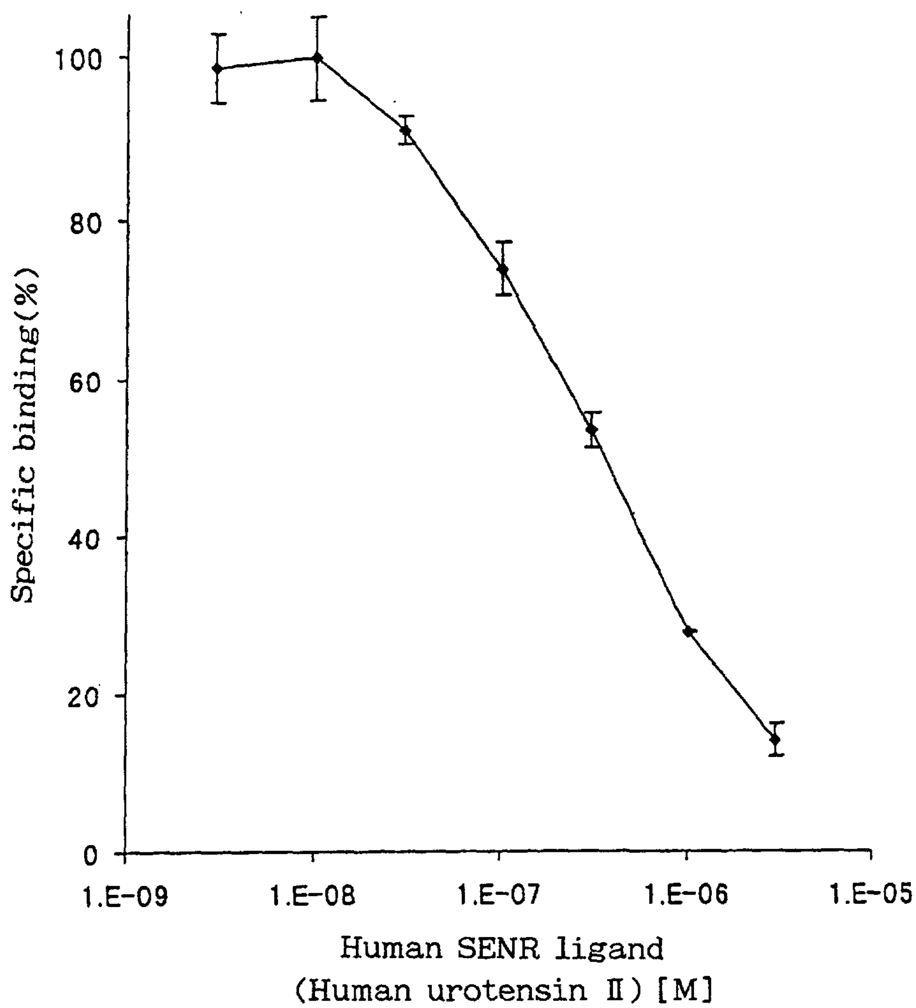
100
80
60
40
20
0
Specific binding(%)
1.E-09
1.E-08
1.E-07
1.E-06
1.E-05
Human SENR ligand
(Human urotensin II) [M]

# Fig.14

```
                                          10                                          20
ATG TAT AAG CTG GTC TCC TGC TGT TTG CTT TTC ATA GGA TCC TTA AAT CCG CTC CTG TCT
Met Tyr Lys Leu Val Ser Cys Cys Leu Leu Phe Ile Gly Ser Leu Asn Pro Leu Leu Ser

                                          30                                          40
CTT CCT GTC CTT GAC TCC AGG CAA GAG TCC CTG CAG CTC TTA GCA CCT GAA GAT GTC AGA
Leu Pro Val Leu Asp Ser Arg Gln Glu Ser Leu Gln Leu Leu Ala Pro Glu Asp Val Arg

                                          50                                          60
TCA ACT CTG GAT GAG CTG GAA AGA GCG TCT CTT CTG CAG ATG CTG CCA GAG ATG TCA GGC
Ser Thr Leu Asp Glu Leu Glu Arg Ala Ser Leu Leu Gln Met Leu Pro Glu Met Ser Gly

                                          70                                          80
GCA GAG ACA GGA GAG GGT CTT AGG AAC ACA GAT CCC ATT ACC AAC ATT TTT TAC CCA AGA
Ala Glu Thr Gly Glu Gly Leu Arg Asn Thr Asp Pro Ile Thr Asn Ile Phe Tyr Pro Arg

                                          90                                         100
GGA AAC ATG AGA AAG GCC TTC TCT GGG CAA GAT CCT AAG CTT TTC CTG AGT GAC CTT TTG
Gly Asn Met Arg Lys Ala Phe Ser Gly Gln Asp Pro Lys Leu Phe Leu Ser Asp Leu Leu

                                         110                                         120
TCC AGA ATT AGG AAA CAA TCT AAG AAA CGT|GGA CCT TCC TCT GAA TGC TTC TGG AAA TAC
Ser Arg Ile Arg Lys Gln Ser Lys Lys Arg|Gly Pro Ser Ser Glu Cys Phe Trp Lys Tyr

    122
TGT GTC|TGA AGC AAA ATG ACC CTC TAC TAG TTA CCT CCA AGA CGA CCA TCT GAG AAA ATG
Cys Val|***

TAA AAT AAA GA
```

## INTERNATIONAL SEARCH REPORT

International application No.
PCT/JP99/06649

A. CLASSIFICATION OF SUBJECT MATTER
Int.Cl[7] C07K 14/46, C12N 15/12, C12N 1/21, C12N 5/10, C12P 21/02, C07K 16/18, C12P 21/08, A61P 25/00, A61P 9/00, A61P 13/00, A61P 27/00, G01N 33/53

According to International Patent Classification (IPC) or to both national classification and IPC

B. FIELDS SEARCHED

Minimum documentation searched (classification system followed by classification symbols)
Int.Cl[7] C07K 14/00~16/46, C12N 15/00~90, C12N 1/00~5/28, C12P 21/00~08, A61P 1/00~27/00

Documentation searched other than minimum documentation to the extent that such documents are included in the fields searched

Electronic data base consulted during the international search (name of data base and, where practicable, search terms used)
SwissProt/PIR/GeneSeq, CA(STN), REGISTRY(STN), Genbank/EMBL/DDBJ/GeneSeq, WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| PX<br>PY | Nature, Vol.401, No.6750, (Sep.1999), p.282-286, Robert S. Ames, et al., "Human urotensis-II is a potent vasoconstrictor and agonist for the orphan receptor GPR14" | 1- 4<br>5-23 |
| PX<br>PY | Proc.Natl.Acad.Sci.USA, Vol.95, No.26, (Dec.1998), p.15803-15808, Yolaine Coulouarn et al., "Cloning of the cDNA encoding the urotensin II precursor in frog and human reveals intense expression of the urotensin II gene in motoneurons of the spinal cord" | 1- 4<br>5-23 |
| PX<br>PY | Biochem.Biophys.Res.Commun., (Nov.1999), VOl.265, No.1, p.123-129, Mori M. et al., "Urotensin II is the endogenous ligand of a G-protein-coupled orphan receptor, SENR (GPR14)" | 1 -11<br>12-23 |
| PX<br>PY | WO, 99/35266, A2 (SMITHKLINE BEECHAM CORP. et al.), 15 July, 1999 (15.07.99) (Family: none) | 5-10<br>1-23 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 March, 2000 (14.03.00) | 28 March, 2000 (28.03.00) |
| Name and mailing address of the ISA/<br>Japanese Patent Office | Authorized officer |
| Facsimile No. | Telephone No. |

Form PCT/ISA/210 (second sheet) (July 1992)